# EUROPEAN PATENT APPLICATION

(11) **EP 4 265 715 A1**
(43) Date of publication of application: **25.10.2023**
(21) Application number: 22739365.9
(22) Date of filing: 11.01.2022
(51) Int. Cl.: C12N 5/0735, C12N 5/074, C12N 5/0775, C12N 5/0789, C12N 5/0797, C12N 5/10, C12N 7/01, C12N 15/113, C12N 15/12, C12N 15/13, C12N 15/34, C12N 15/40, C12N 15/51, C12N 15/62, C12N 15/63, A01K 67/027

(54) **NUCLEIC ACID MOLECULE CONTAINING INCORPORATED GENE ENCODING FUSED PROTEIN OF LIGAND AND PROTEIN HAVING PHYSIOLOGICAL ACTIVITY**

(30) Priority: 12.01.2021 JP 2021002567
(71) Applicant: JCR Pharmaceuticals Co., Ltd., Ashiya-shi, Hyogo 659-0021 (JP)
(72) Inventor: KINOSHITA, Masafumi, Kobe-shi, Hyogo 651-2241 (JP); IIZUKA, Shunsuke, Kobe-shi, Hyogo 651-2241 (JP); IMAKIIRE, Atsushi, Kobe-shi, Hyogo 651-2241 (JP); TAKAGI, Haruna, Kobe-shi, Hyogo 651-2241 (JP); SONODA, Hiroyuki, Kobe-shi, Hyogo 651-2241 (JP)
(74) Representative: Boult Wade Tennant LLP
(86) International application number: PCT/JP2022/000466
(87) International publication number: WO 2022/153957

(57) **Abstract**

The invention provides a nucleic acid molecule comprising a gene encoding a fusion protein of a ligand and a protein having physiological activity, to be used for expressing the fusion protein in cells, tissue or the body. The invention further provides a nucleic acid molecule comprising the fusion protein-encoding gene, which is in the form of a plasmid, in a form encapsulated in a recombinant virus virion, or a form encapsulated in liposomes or lipid nanoparticles. The invention further relates to the use of a medicine which is a nucleic acid molecule comprising the fusion protein-encoding gene.

## Description

### Technical Field

The present invention relates to a nucleic acid molecule comprising a gene encoding a fusion protein of a ligand and a protein having physiological activity, to be used to cause the fusion protein to function in cells, tissue or a body, and for example, it relates to a T vector incorporating a nucleic acid molecule encoding the fusion protein in an AAV viral vector system, or a vector plasmid incorporating a nucleic acid molecule encoding the fusion protein in a lentivirus vector system.

### Background Art

Most recombinant proteins used in medicine are administered to patients by parenteral means such as subcutaneous injection, intramuscular injection or intravenous injection. For chronic disease patients, it is necessary for administration of the medicine to be repeated over long periods, creating a heavy burden on patients when the administration is by parenteral means.

Recombinant proteins include fusion proteins of antibodies and proteins having physiological activity. Fusion proteins of antibodies and lysosomal enzymes are examples of such fusion proteins (PTLs 1 to 5 and NPL 1).

Lysosomal disease is a genetic disease caused by decrease or loss of lysosomal enzyme activity due to an abnormality in a gene encoding for a lysosomal enzyme normally present in lysosomes. Lysosomal disease patients are administered recombinant lysosomal enzymes by intravenous injection, as enzyme replacement therapy with the goal of replenishing reduced or absent lysosomal enzymes. Because lysosomal disease patients suffer from genetic disease, such enzyme replacement therapy must be received for life.

Some lysosomal diseases can lead to brain damage. Treatment for brain damage requires supplementation of lysosomal enzymes into the brain, but since almost all lysosomal enzymes administered by intravenous injection fail to cross the blood brain barrier (BBB), the lysosomal enzymes cannot be supplied into the brain.

A method of supplying lysosomal enzymes into the brain has been reported, the method relying on intravenous injection of a fusion protein having a lysosomal enzyme bonded to an antibody that recognizes as antigen a molecule that is present on vascular endothelial cell surfaces (NPL 2). The fusion protein can bind to the surfaces of vascular endothelial cells via the antibody portion, passing through the BBB and reaching the brain. The fusion protein thus allows the lysosomal enzyme to be supplied to the brain. Even when the lysosomal enzyme is in the form of such a fusion protein, however, patients with the genetic lysosomal disease must still receive enzyme replacement therapy with the fusion protein for their entire life.

### Citation List

### Patent Literature

[PTL 1] WO2016/208695
[PTL 2] WO2018/124121
[PTL 3] US20070082380
[PTL 4] US20090053219
[PTL 5] US20110110935

### Non-Patent Literature

[NPL 1] Sonoda H. et al., Mol Ther. 26. 1366-74 (2018)
[NPL 2] Okuyama T. et al., Mol Ther. 26. 27. 456-64 (2019)

### Summary of Invention

### Technical Problem

It is an object of the present invention to provide a viral vector incorporating a gene encoding a fusion protein of a ligand and a protein having physiological activity, to be used for expressing the fusion protein in the body.

### Solution to Problem

During the course of research toward achieving the aforementioned object, the present inventors incorporated a gene encoding a protein comprising an antibody bonded to human iduronate-2-sulfatase, a type of human lysosomal enzyme, into a T vector in an AAV viral vector system, and found that the fusion protein of the antibody and human lysosomal enzyme is expressed in the body or cultured cells of mammals infected with rAAV virion prepared using the T vector, and the present invention was completed based on this finding. Specifically, the invention includes the following.
1. A nucleic acid molecule comprising any one of the following nucleotide sequences (1) to (6):
   (1) a nucleotide sequence comprising a first inverted terminal repeat (ITR) or a functional equivalent thereof, a nucleotide sequence encoding a fusion protein of a ligand and a protein having physiological activity, and a nucleotide sequence comprising a second inverted terminal repeat (ITR) or a functional equivalent thereof, in this order toward the downstream end;
   (2) a nucleotide sequence comprising a first inverted terminal repeat (ITR) or a functional equivalent thereof, a nucleotide sequence comprising a regulatory element of gene expression, a nucleotide sequence encoding a fusion protein of a ligand and a protein having physiological activity, and a nucleotide sequence comprising a second inverted terminal repeat (ITR) or a functional equivalent thereof, in this order toward the downstream end;
   (3) a nucleotide sequence comprising a first long terminal repeat (LTR) or a functional equivalent thereof, a nucleotide sequence encoding a fusion protein of a ligand and a protein having physiological activity, and a nucleotide sequence comprising a second long terminal repeat (LTR) or a functional equivalent thereof, in this order toward the downstream end;
   (4) a nucleotide sequence comprising a first long terminal repeat (LTR) or a functional equivalent thereof, a nucleotide sequence comprising a regulatory element of gene expression, a nucleotide sequence encoding a fusion protein of a ligand and a protein having physiological activity, and a nucleotide sequence comprising a second long terminal repeat (LTR) or a functional equivalent thereof, in this order toward the downstream end;
   (5) a nucleotide sequence comprising a leader or a functional equivalent thereof, a nucleotide sequence encoding a fusion protein of a ligand and a protein having physiological activity, and a nucleotide sequence comprising a trailer or a functional equivalent thereof, in this order toward the downstream end; or
   (6) a nucleotide sequence comprising a leader or a functional equivalent thereof, a nucleotide sequence comprising a regulatory element of gene expression, a nucleotide sequence encoding a fusion protein of a ligand and a protein having physiological activity, and a nucleotide sequence comprising a trailer or a functional equivalent thereof, in this order toward the downstream end.
2. The nucleic acid molecule according to 1 above, wherein the ligand is an antibody.
3. The nucleic acid molecule according to 2 above, selected from the following (1) to (4);
   (1) the nucleotide sequence encoding the fusion protein of the antibody and the protein having physiological activity comprises a nucleotide sequence encoding a conjugate in which the protein having physiological activity is bonded to the C-terminus of the heavy chain of the antibody, and a nucleotide sequence encoding the light chain of the antibody;
   (2) the nucleotide sequence encoding the fusion protein of the antibody and the protein having physiological activity comprises a nucleotide sequence encoding a conjugate in which the protein having physiological activity is bonded to the N-terminus of the heavy chain of the antibody, and a nucleotide sequence encoding the light chain of the antibody;
   (3) the nucleotide sequence encoding the fusion protein of the antibody and the protein having physiological activity comprises a nucleotide sequence encoding a conjugate in which the protein having physiological activity is bonded to the C-terminus of the light chain of the antibody, and a nucleotide sequence encoding the heavy chain of the antibody; or
   (4) the nucleotide sequence encoding the fusion protein of the antibody and the protein having physiological activity comprises a nucleotide sequence encoding a conjugate in which the protein having physiological activity is bonded to the N-terminus of the light chain of the antibody, and a nucleotide sequence encoding the heavy chain of the antibody.
4. The nucleic acid molecule according to 2 above, selected from the following (1) to (4);
   (1) the nucleotide sequence encoding the fusion protein of the antibody and the protein having physiological activity comprises a nucleotide sequence encoding a conjugate in which the protein having physiological activity is bonded to the C-terminus of the heavy chain of the antibody via a linker, and a nucleotide sequence encoding the light chain of the antibody;
   (2) the nucleotide sequence encoding the fusion protein of the antibody and the protein having physiological activity comprises a nucleotide sequence encoding a conjugate in which the protein having physiological activity is bonded to the N-terminus of the heavy chain of the antibody via a linker, and a nucleotide sequence encoding the light chain of the antibody;
   (3) the nucleotide sequence encoding the fusion protein of the antibody and the protein having physiological activity comprises a nucleotide sequence encoding a conjugate in which the protein having physiological activity is bonded to the C-terminus of the light chain of the antibody via a linker, and a nucleotide sequence encoding the heavy chain of the antibody; or
   (4) the nucleotide sequence encoding the fusion protein of the antibody and the protein having physiological activity comprises a nucleotide sequence encoding a conjugate in which the protein having physiological activity is bonded to the N-terminus of the light chain of the antibody via a linker, and a nucleotide sequence encoding the heavy chain of the antibody.
5. The nucleic acid molecule according to 4 above, wherein the linker is a peptide consisting of 1 to 50 amino acid residues.
6. The nucleic acid molecule according to 5 above, wherein the linker is a peptide comprising an amino acid sequence selected from the group consisting of a single glycine, a single serine, the amino acid sequence Gly-Ser, the amino acid sequence Gly-Gly-Ser, the amino acid sequence set forth as SEQ ID NO: 1, the amino acid sequence set forth as SEQ ID NO: 2, the amino acid sequence set forth as SEQ ID NO: 3, and an amino acid sequence consisting of 2 to 10 of any of said amino acid sequences that are consecutively linked.
7. The nucleic acid molecule according to 2 above, selected from the following (1) to (4);
   (1) the nucleotide sequence encoding the fusion protein of the antibody and the protein having physiological activity comprises a nucleotide sequence encoding a conjugate in which the heavy chain of the antibody is bonded to the C-terminus of the light chain of the antibody via a second linker and the protein having physiological activity is bonded to the C-terminus of the heavy chain of the antibody directly or via a linker;
   (2) the nucleotide sequence encoding the fusion protein of the antibody and the protein having physiological activity comprises a nucleotide sequence encoding a conjugate in which the light chain of the antibody is bonded to the C-terminus of the heavy chain of the antibody via a second linker and the protein having physiological activity is bonded to the C-terminus of the light chain of the antibody directly or via a linker;
   (3) the nucleotide sequence encoding the fusion protein of the antibody and the protein having physiological activity comprises a nucleotide sequence encoding a conjugate in which the heavy chain of the antibody is bonded to the C-terminus of the protein having physiological activity directly or via a linker and the light chain of the antibody is bonded to the C-terminus of the heavy chain of the antibody via a second linker; or
   (4) the nucleotide sequence encoding the fusion protein of the antibody and the protein having physiological activity comprises a nucleotide sequence encoding a conjugate in which the light chain of the antibody is bonded to the C-terminus of the protein having physiological activity directly or via a linker and the heavy chain of the antibody is bonded to the C-terminus of the light chain of the antibody via a second linker.
8. The nucleic acid molecule according to 7 above, wherein the linker is a peptide consisting of 1 to 50 amino acid residues.
9. The nucleic acid molecule according to 8 above, wherein the linker is a peptide comprising an amino acid sequence selected from the group consisting of a single glycine, a single serine, the amino acid sequence Gly-Ser, the amino acid sequence Gly-Gly-Ser, the amino acid sequence set forth as SEQ ID NO: 1, the amino acid sequence set forth as SEQ ID NO: 2, the amino acid sequence set forth as SEQ ID NO: 3, and an amino acid sequence consisting of 2 to 10 of any of said amino acid sequences that are consecutively linked.
10. The nucleic acid molecule according to 9 above, wherein the second linker consists of 8 to 50 amino acid residues.
11. The nucleic acid molecule according to 8 above, wherein the second linker is selected from the group consisting of the amino acid sequence Gly-Ser, the amino acid sequence Gly-Gly-Ser, the amino acid sequence Gly-Gly-Gly, the amino acid sequence set forth as SEQ ID NO: 1, the amino acid sequence set forth as SEQ ID NO: 2, the amino acid sequence set forth as SEQ ID NO: 3, an amino acid sequence consisting of three consecutively linked sequences that are each the sequence set forth as SEQ ID NO: 1, and an amino acid sequence consisting of 2 to 10 of any of said amino acid sequences that are consecutively linked.
12. The nucleic acid molecule according to 3 above, selected from the following (1) to (4);
   (1) the nucleotide sequence encoding the fusion protein of the antibody and the protein having physiological activity comprises a nucleotide sequence encoding a conjugate in which the protein having physiological activity is bonded to the C-terminus of the heavy chain of the antibody, a nucleotide sequence encoding an internal ribosome entry site, and a nucleotide sequence encoding the light chain of the antibody, in this order toward the downstream end;
   (2) the nucleotide sequence encoding the fusion protein of the antibody and the protein having physiological activity comprises a nucleotide sequence encoding a conjugate in which the protein having physiological activity is bonded to the N-terminus of the heavy chain of the antibody, a nucleotide sequence encoding an internal ribosome entry site, and a nucleotide sequence encoding the light chain of the antibody, in this order toward the downstream end;
   (3) the nucleotide sequence encoding the fusion protein of the antibody and the protein having physiological activity comprises a nucleotide sequence encoding a conjugate in which the protein having physiological activity is bonded to the C-terminus of the light chain of the antibody, a nucleotide sequence encoding an internal ribosome entry site, and a nucleotide sequence encoding the heavy chain of the antibody, in this order toward the downstream end; or
   (4) the nucleotide sequence encoding the fusion protein of the antibody and the protein having physiological activity comprises a nucleotide sequence encoding a conjugate in which the protein having physiological activity is bonded to the N-terminus of the light chain of the antibody, a nucleotide sequence encoding an internal ribosome entry site, and a nucleotide sequence encoding the heavy chain of the antibody, in this order toward the downstream end.
13. The nucleic acid molecule according to any one of 4 to 6 above, selected from the following (1) to (4);
   (1) the nucleotide sequence encoding the fusion protein of the antibody and the protein having physiological activity comprises a nucleotide sequence encoding a conjugate in which the protein having physiological activity is bonded to the C-terminus of the heavy chain of the antibody via a linker, a nucleotide sequence encoding an internal ribosome entry site, and a nucleotide sequence encoding the light chain of the antibody, in this order toward the downstream end;
   (2) the nucleotide sequence encoding the fusion protein of the antibody and the protein having physiological activity comprises a nucleotide sequence encoding a conjugate in which the protein having physiological activity is bonded to the N-terminus of the heavy chain of the antibody via a linker, a nucleotide sequence encoding an internal ribosome entry site, and a nucleotide sequence encoding the light chain of the antibody, in this order toward the downstream end;
   (3) the nucleotide sequence encoding the fusion protein of the antibody and the protein having physiological activity comprises a nucleotide sequence encoding a conjugate in which the protein having physiological activity is bonded to the C-terminus of the light chain of the antibody via a linker, a nucleotide sequence encoding an internal ribosome entry site, and a nucleotide sequence encoding the heavy chain of the antibody, in this order toward the downstream end; or
   (4) the nucleotide sequence encoding the fusion protein of the antibody and the protein having physiological activity comprises a nucleotide sequence encoding a conjugate in which the protein having physiological activity is bonded to the N-terminus of the light chain of the antibody via a linker, a nucleotide sequence encoding an internal ribosome entry site, and a nucleotide sequence encoding the heavy chain of the antibody, in this order toward the downstream end.
14. The nucleic acid molecule according to 12 or 13 above, wherein the internal ribosome entry site is derived from the 5' untranslated region of a virus or gene selected from the group consisting of a virus belonging to the family Picornaviridae, foot-and-mouth disease virus, hepatitis A virus, hepatitis C virus, coronavirus, bovine enterovirus, Cyler's murine encephalomyelitis virus, Coxsackievirus group B, the human immunoglobulin heavy chain binding protein gene, the Drosophila Antennapedia gene and the Drosophila Ultrabithorax gene.
15. The nucleic acid molecule according to 12 or 13 above, wherein the internal ribosome entry site is derived from the 5' untranslated region of a virus belonging to the family Picornaviridae.
16. The nucleic acid molecule according to any one of 2 to 14 above, wherein the antibody is an antigen-binding fragment.
17. The nucleic acid molecule according to any one of 2 to 14 above, wherein the antibody is Fab.
18. The nucleic acid molecule according to 2 above, wherein the antibody is a single-domain antibody.
19. The nucleic acid molecule according to any one of 2 to 18 above, wherein the antibody has specific affinity for a protein present on the surfaces of vascular endothelial cells.
20. The nucleic acid molecule according to 19 above, wherein the vascular endothelial cells are cerebrovascular endothelial cells.
21. The nucleic acid molecule according to 19 or 20 above, wherein the protein present on the surfaces of the vascular endothelial cells is selected from the group consisting of transferrin receptor, insulin receptor, leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor.
22. The nucleic acid molecule according to any one of 1 to 21 above, wherein the regulatory element of gene expression is selected from the group consisting of cytomegalovirus-derived promoter, SV40 early promoter, human elongation factor-1α (EF-1α) promoter, human ubiquitin C promoter, retroviral Rous sarcoma virus-LTR promoter, dihydrofolate reductase promoter, β-actin promoter, phosphoglycerate kinase (PGK) promoter, mouse albumin promoter, human albumin promoter, human α-1 antitrypsin promoter and mouse α-fetoprotein enhancer/mouse albumin promoter.
23. The nucleic acid molecule according to 1 above, wherein the ligand has specific affinity for a protein present on the surfaces of vascular endothelial cells.
24. The nucleic acid molecule according to 23 above, wherein the vascular endothelial cells are cerebrovascular endothelial cells.
25. The nucleic acid molecule according to 23 or 24 above, wherein the protein present on the surfaces of the vascular endothelial cells is selected from the group consisting of transferrin receptor, insulin receptor, leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor.
26. The nucleic acid molecule according to 23 or 24 above, wherein the ligand is selected from the group consisting of transferrin, insulin, leptin, insulin-like growth factor I, insulin-like growth factor II, lipoprotein, and low density lipoprotein.
27. The nucleic acid molecule according to any one of 1 to 26 above, wherein the protein having physiological activity is selected from the group consisting of growth hormone, lysosomal enzymes, somatomedin, insulin, glucagon, cytokines, lymphokines, blood coagulation factors, antibodies, fusion proteins of antibodies and other proteins, granulocyte-macrophage colony-stimulating factor (GM-CSF), granulocyte-colony stimulating factor (G-CSF), macrophage colony-stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, DNasel, thyroid-stimulating hormone (TSH), nerve growth factor (NGF), ciliary neurotrophic factor (CNTF), glial cell-line neurotrophic factor (GDNF), neurotrophin 3, neurotrophin 4/5, neurotrophin 6, neuregulin 1, activin, basic fibroblast growth factor (bFGF), fibroblast growth factor 2 (FGF2), epidermal growth factor (EGF), vascular endothelial growth factor (VEGF), interferon-α, interferon-β, interferon-γ, interleukin-6, PD-1, PD-1 ligand, tumor necrosis factor-α receptor (TNF-α receptor), enzymes having beta amyloid degrading activity, etanercept, pegvisomant, metreleptin, abatacept, asfotase, GLP-1 receptor agonist, and antibody medicines.
28. The nucleic acid molecule according to any one of 1 to 26 above, wherein the protein having physiological activity is selected from the group consisting of α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, palmitoyl protein thioesterase-1, tripeptidyl peptidase-1, hyaluronidase-1, CLN1 and CLN2.
29. The nucleic acid molecule according to any one of 1 to 28 above, wherein the first inverted terminal repeat and the second inverted terminal repeat are each one derived from an adeno-associated virus, one derived from an adenovirus, or a variant thereof.
30. The nucleic acid molecule according to any one of 1 to 28 above, wherein the first inverted terminal repeat comprises the nucleotide sequence set forth as SEQ ID NO: 4, and the second inverted terminal repeat comprises the nucleotide sequence set forth as SEQ ID NO: 5.
31. The nucleic acid molecule according to any one of 1 to 28 above, wherein the first inverted terminal repeat is selected from the following (1) to (3) and the second inverted terminal repeat is selected from the following (4) to (6):
   (1) a nucleotide sequence having identity not lower than 80% to the nucleotide sequence set forth as SEQ ID NO: 4,
   (2) a nucleotide sequence having identity not lower than 90% to the nucleotide sequence set forth as SEQ ID NO: 4,
   (3) the nucleotide sequence set forth as SEQ ID NO: 4, but modified by a substitution, deletion or addition of 1 to 20 nucleotides, and
   (4) a nucleotide sequence having identity not lower than 80% to the nucleotide sequence set forth as SEQ ID NO: 5,
   (5) a nucleotide sequence having identity not lower than 90% to the nucleotide sequence set forth as SEQ ID NO: 5,
   (6) the nucleotide sequence set forth as SEQ ID NO: 5, but modified by a substitution, deletion or addition of 1 to 20 nucleotides.
32. The nucleic acid molecule according to any one of 1 to 28 above, wherein the functional equivalent of the first inverted terminal repeat comprises the nucleotide sequence set forth as SEQ ID NO: 6, and the functional equivalent of the second inverted terminal repeat comprises the nucleotide sequence set forth as SEQ ID NO: 7.
33. The nucleic acid molecule according to any one of 1 to 28 above, wherein the functional equivalent of the first inverted terminal repeat is selected from the following (1) to (3) and the functional equivalent of the second inverted terminal repeat is selected from the following (4) to (6):
   (1) a nucleotide sequence having identity not lower than 80% to the nucleotide sequence set forth as SEQ ID NO: 6,
   (2) a nucleotide sequence having identity not lower than 90% to the nucleotide sequence set forth as SEQ ID NO: 6,
   (3) the nucleotide sequence set forth as SEQ ID NO: 6, but modified by a substitution, deletion or addition of 1 to 20 nucleotides, and
   (4) a nucleotide sequence having identity not lower than 80% to the nucleotide sequence set forth as SEQ ID NO: 7,
   (5) a nucleotide sequence having identity not lower than 90% to the nucleotide sequence set forth as SEQ ID NO: 7,
   (6) the nucleotide sequence set forth as SEQ ID NO: 7, but modified by a substitution, deletion or addition of 1 to 20 nucleotides.
34. The nucleic acid molecule according to any one of 1 to 28 above, wherein the first long terminal repeat and the second long terminal repeat are each one derived from a lentivirus or retrovirus, or a variant thereof.
35. The nucleic acid molecule according to any one of 1 to 28 above, wherein the first long terminal repeat comprises the nucleotide sequence set forth as SEQ ID NO: 43, and the second long terminal repeat comprises the nucleotide sequence set forth as SEQ ID NO: 44.
36. The nucleic acid molecule according to any one of 1 to 28 above, wherein the first long terminal repeat is selected from the following (1) to (3) and the second long terminal repeat is selected from the following (4) to (6):
   (1) a nucleotide sequence having identity not lower than 80% to the nucleotide sequence set forth as SEQ ID NO: 43,
   (2) a nucleotide sequence having identity not lower than 90% to the nucleotide sequence set forth as SEQ ID NO: 43,
   (3) the nucleotide sequence set forth as SEQ ID NO: 43, but modified by 1 to 20 nucleotide substitutions, deletions or additions, and
   (4) a nucleotide sequence having identity not lower than 80% to the nucleotide sequence set forth as SEQ ID NO: 44,
   (5) a nucleotide sequence having identity not lower than 90% to the nucleotide sequence set forth as SEQ ID NO: 44,
   (6) the nucleotide sequence set forth as SEQ ID NO: 44, but modified by 1 to 20 nucleotide substitutions, deletions or additions.
37. The nucleic acid molecule according to any one of 1 to 28 above, wherein the leader and the trailer are each one derived from Sendai virus, or a variant thereof.
38. The nucleic acid molecule according to any one of 1 to 28 above, wherein the leader comprises the nucleotide sequence set forth as SEQ ID NO: 45, and the trailer comprises the nucleotide sequence set forth as SEQ ID NO: 46.
39. The nucleic acid molecule according to any one of 1 to 28 above, wherein the leader is selected from the following (1) to (3) and the trailer is selected from the following (4) to (6):
   (1) a nucleotide sequence having identity not lower than 80% to the nucleotide sequence set forth as SEQ ID NO: 45,
   (2) a nucleotide sequence having identity not lower than 90% to the nucleotide sequence set forth as SEQ ID NO: 45,
   (3) the nucleotide sequence set forth as SEQ ID NO: 45, but modified by a substitution, deletion or addition of 1 to 20 nucleotides, and
   (4) a nucleotide sequence having identity not lower than 80% to the nucleotide sequence set forth as SEQ ID NO: 46,
   (5) a nucleotide sequence having identity not lower than 90% to the nucleotide sequence set forth as SEQ ID NO: 47,
   (6) the nucleotide sequence set forth as SEQ ID NO: 47, but modified by a substitution, deletion or addition of 1 to 20 nucleotides.
40. A cell, tissue or animal, into which the nucleic acid molecule according to any one of 1 to 39 above has been introduced.
41. A stem cell, into which the nucleic acid molecule according to any one of 1 to 39 above has been introduced.
42. The stem cell according to 41 above, which is selected from the group consisting of mesenchymal stem cells, dental pulp stem cells, hematopoietic stem cells, embryonic stem cells, endothelial stem cells, mammary stem cells, intestinal stem cells, liver stem cells, pancreatic stem cells, neural stem cells and iPS cells.
43. A plasmid comprising the nucleic acid molecule according to any one of 1 to 39 above.
44. A virion comprising the nucleic acid molecule according to any one of 1 to 39 above.

### Advantageous Effects of Invention

According to the invention it is possible to provide a nucleic acid molecule comprising a gene encoding a fusion protein of a ligand and a protein having physiological activity, to be used for expressing the fusion protein in cells, tissue or a body.

### Brief Description of Drawings

Fig. 1 is a schematic diagram showing the structure of AAV8-mMAP-Fab-hI2S vector (plasmid).
Fig. 2 is a schematic diagram showing the structure of pR2(mod)C8 vector.

### Description of Embodiments

For the purpose of the invention, "nucleic acid molecule" refers primarily to DNA having deoxyribonucleotide polymerized by phosphodiester bonds, or RNA having ribonucleotide polymerized by phosphodiester bonds.

When the nucleic acid molecule is DNA, the DNA may be a single strand (single-stranded) or a double strand, including the complementary strand. When the DNA is a single strand, the DNA may be the plus strand or the minus strand. Each deoxyribonucleotide composing the DNA may be in the naturally-occurring form or a modification of the naturally-occurring form, so long as the protein-encoding gene in the DNA can be translated into mRNA in cells of a mammal (especially a human). According to one embodiment of the invention, each deoxyribonucleotide composing the DNA may be in the naturally-occurring form or a modification of the naturally-occurring form so long as the protein-encoding gene in the DNA can be translated into mRNA, and also all or part of the DNA can be replicated, in cells of a mammal (especially a human).

When the nucleic acid molecule is RNA, the RNA may be a single strand (single-stranded) or a double strand, including the complementary strand. When the RNA is single-stranded, the RNA may be the plus strand or the minus strand. According to one embodiment of the invention, each deoxyribonucleotide composing the RNA may be in the naturally-occurring form or a modified form, so long as the protein-encoding gene in the RNA can be reverse transcribed into DNA in cells of a mammal (especially a human). According to another embodiment of the invention, each deoxyribonucleotide composing the RNA may be in the naturally-occurring form or a modified form, so long as the protein-encoding gene in the RNA can be translated into protein in cells of a mammal (especially a human). Modification of ribonucleotides is carried out to inhibit degradation of RNA by RNase and increase the stability of the RNA in cells, for example.

According to another embodiment, "inverted terminal repeat (ITR)" refers to a nucleotide sequence present at the ends of a viral genome and including repeats of the same sequence. The ITR is preferably one derived from an adeno-associated virus or one derived from adenovirus. For example, adeno-associated virus ITR is the region which has a chain length of about 145 nucleotides and functions as a replication origin. According to one embodiment of the invention, the nucleic acid molecule includes two inverted terminal repeats (ITR), which are a first inverted terminal repeat (ITR) and a second inverted terminal repeat (ITR), respectively. When a gene encoding a fusion protein is situated between the two ITRs, the ITR located at the 5'-end is referred to as the first inverted terminal repeat (ITR) and the ITR located at the 3'-end is referred to as the second inverted terminal repeat (ITR). According to the invention, an inverted terminal repeat (ITR) may be derived from any virus so long as it functions as a replication origin and has at least one of the original functions of an ITR, such as the function of inserting a gene into host cells, with adeno-associated virus ITR being a preferred example. Moreover, the ITRs are not limited to wild type ITRs and may be a variant with a modification such as a substitution, deletion or addition in the nucleotide sequence of a wild type ITR, so long as they can still substitute for the wild type ITR.

When an ITR is of adeno-associated virus, it is not limited regarding the serotype of AAV and may be serotype 1, 2, 3, 4, 5, 6, 7, 8, 9, 10 or 11. For an inverted terminal repeat (ITR) of AAV of serotype 2, for example, the first ITR includes the nucleotide sequence set forth as SEQ ID NO: 4 and the second ITR includes the nucleotide sequence set forth as SEQ ID NO: 5.

The ITRs are not limited to wild type ITRs, and may have a modification such as a substitution, deletion or addition in the nucleotide sequence of a wild type ITR. When one or more nucleotides of a wild type ITR nucleotide sequence are substituted with different nucleotides, the number of substituting nucleotides is preferably 1 to 20, more preferably 1 to 10 and even more preferably 1 to 3. When the nucleotide sequence of a wild type ITR has deletions, the number of deleted nucleotides is preferably 1 to 20, more preferably 1 to 10 and even more preferably 1 to 3. Mutations that are combinations of substitutions and deletions of nucleotides may also be added. When one or more nucleotides are added to a wild type ITR, preferably 1 to 20, more preferably 1 to 10 and even more preferably 1 to 3 nucleotides are added within or at the 5'-end or 3'-end of the ITR nucleotide sequence. Mutations that are combinations of additions, substitutions and deletions of nucleotides may also be added. The nucleotide sequence of a mutated ITR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence of the wild type ITR.

A functional equivalent of an ITR is an ITR that can be functionally substituted for the ITR. The functional equivalent of the ITR may be a variant of the wild type ITR. An ITR artificially constructed based on the ITR also qualifies as a functional equivalent of the ITR so long as it can substitute for the ITR.

A functional equivalent of an AAV ITR is one that can be functionally substituted for the AAV ITR. The functional equivalent of the AAV ITR may be a variant of the wild type AAV ITR. An ITR artificially constructed based on the AAV ITR also qualifies as a functional equivalent of the AAV ITR so long as it can substitute for the AAV ITR.

One functional equivalent of the first artificially constructed AAV inverted terminal repeat (first AAV-ITR) is one having the nucleotide sequence set forth as SEQ ID NO: 6 (first AAV-ITR functional equivalent). The first AAV-ITR functional equivalent includes the nucleotide sequence set forth as SEQ ID NO: 6 having a substitution, deletion or mutation, so long as it can be used as a functional substitute for the first AAV ITR. When one or more nucleotides of the nucleotide sequence set forth as SEQ ID NO: 6 are substituted with different nucleotides, the number of substituting nucleotides is preferably 1 to 20, more preferably 1 to 20, yet more preferably 1 to 5 and even more preferably 1 to 3. When one or more nucleotides of the nucleotide sequence set forth as SEQ ID NO: 6 are deleted, the number of deleted nucleotides is preferably 1 to 20, more preferably 1 to 10, yet more preferably 1 to 5 and even more preferably 1 to 3. ITRs with mutations that are combinations of substitutions and deletions of nucleotides also qualify as the first AAV-ITR functional equivalent. When one or more nucleotides are added to the nucleotide sequence set forth as SEQ ID NO: 6, preferably 1 to 20, more preferably 1 to 10, yet more preferably 1 to 5 and even more preferably 1 to 3 nucleotides are added within or at the 5'-end or 3'-end of the nucleotide sequence. ITRs with mutations that are combinations of additions, substitutions and deletions of nucleotides also qualify as the first AAV-ITR functional equivalent. The nucleotide sequence of a mutated ITR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 6.

One functional equivalent of the artificially constructed second inverted terminal repeat (second AAV-ITR) is one having the nucleotide sequence set forth as SEQ ID NO: 7 (second AAV-ITR functional equivalent). The second AAV-ITR functional equivalent includes the nucleotide sequence set forth as SEQ ID NO: 7 having a substitution, deletion or mutation, so long as it can be used as a functional substitute for the second AAV ITR. When one or more nucleotides of the nucleotide sequence set forth as SEQ ID NO: 7 are substituted with different nucleotides, the number of substituting nucleotides is preferably 1 to 20, more preferably 1 to 10, yet more preferably 1 to 5 and even more preferably 1 to 3. When one or more nucleotides of the nucleotide sequence set forth as SEQ ID NO: 7 are deleted, the number of deleted nucleotides is preferably 1 to 20, more preferably 1 to 10, yet more preferably 1 to 5 and even more preferably 1 to 3. ITRs with mutations that are combinations of substitutions and deletions of nucleotides also qualify as the second AAV-ITR functional equivalent. When one or more nucleotides are added to the nucleotide sequence set forth as SEQ ID NO: 7, preferably 1 to 20, more preferably 1 to 10, yet more preferably 1 to 5 and even more preferably 1 to 3 nucleotides are added within or at the 5'-end or 3'-end of the nucleotide sequence. ITRs with mutations that are combinations of additions, substitutions and deletions of nucleotides also qualify as the second AAV-ITR functional equivalent. The nucleotide sequence of a mutated ITR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 7.

According to one embodiment, "long terminal repeat (LTR)" refers to a nucleotide sequence present at the ends of a eukaryotic retrotransposon, or a retrovirus genome or lentivirus genome, with several hundred to several thousand repeats of the same sequence. According to another embodiment, the nucleic acid molecule includes two long terminal repeats (LTR), which are a first long terminal repeat (LTR) and second long terminal repeat (LTR), respectively. When a gene encoding a fusion protein is situated between the two LTRs, the LTR located at the 5'-end is referred to as the first long terminal repeat (LTR) and the LTR located at the 3'-end is referred to as the second long terminal repeat (LTR). According to the invention, a long terminal repeat (LTR) may be derived from any virus so long as it functions as a replication origin and has at least one of the original functions of an LTR, such as the function of inserting a gene into host cells, with retrovirus genome and lentivirus being preferred examples. Moreover, the LTRs are not limited to wild type LTRs, and may be a variant with a modification such as a substitution, deletion or addition in the nucleotide sequence of a wild type LTR, so long as they can still substitute for the wild type LTR.

When one or more nucleotides of a wild type LTR nucleotide sequence are substituted with different nucleotides, the number of substituting nucleotides is preferably 1 to 20, more preferably 1 to 10 and even more preferably 1 to 3. When the nucleotide sequence of a wild type LTR has deletions, the number of deleted nucleotides is preferably 1 to 20, more preferably 1 to 10 and even more preferably 1 to 3. Mutations that are combinations of substitutions and deletions of nucleotides may also be added. When one or more nucleotides are added to a wild type LTR, preferably 1 to 20, more preferably 1 to 10 and even more preferably 1 to 3 nucleotides are added within or at the 5'-end or 3'-end of the LTR nucleotide sequence. Mutations that are combinations of additions, substitutions and deletions of nucleotides may also be added. The nucleotide sequence of a mutated LTR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence of the wild type LTR.

A functional equivalent of an LTR is an LTR that can be used as a functional substitute for the LTR. The functional equivalent of the LTR may be a variant of the wild type LTR. An LTR artificially constructed based on the LTR also qualifies as a functional equivalent of the LTR so long as it can substitute for the LTR.

A functional equivalent of a lentivirus LTR may be any one that can be used as a functional substitute for the lentivirus LTR. A functional equivalent of a lentivirus LTR may be a variant of a wild type lentivirus LTR. An LTR artificially constructed based on the lentivirus LTR also qualifies as a functional equivalent of the lentivirus LTR so long as it can substitute for the lentivirus LTR.

A functional equivalent of a retrovirus LTR may be any one that can be used as a functional substitute for the retrovirus LTR. A functional equivalent of a retrovirus LTR may be a variant of a wild type retrovirus LTR. An LTR artificially constructed based on the retrovirus LTR also qualifies as a functional equivalent of the retrovirus LTR so long as it can substitute for the retrovirus LTR.

One functional equivalent of the first LTR is one having the nucleotide sequence set forth as SEQ ID NO: 43. The first LTR functional equivalent includes the nucleotide sequence set forth as SEQ ID NO: 43 having a substitution, deletion or mutation, so long as it can be functionally used as the first LTR. When one or more nucleotides of the nucleotide sequence set forth as SEQ ID NO: 43 are substituted with different nucleotides, the number of substituting nucleotides is preferably 1 to 20, more preferably 1 to 10, yet more preferably 1 to 5 and even more preferably 1 to 3. When one or more nucleotides of the nucleotide sequence set forth as SEQ ID NO: 43 are deleted, the number of deleted nucleotides is preferably 1 to 20, more preferably 1 to 10, yet more preferably 1 to 5 and even more preferably 1 to 3. LTRs with mutations that are combinations of substitutions and deletions of nucleotides also qualify as the first LTR functional equivalent. When one or more nucleotides are added to the nucleotide sequence set forth as SEQ ID NO: 43, preferably 1 to 20, more preferably 1 to 10, yet more preferably 1 to 5 and even more preferably 1 to 3 nucleotides are added within or at the 5'-end or 3'-end of the nucleotide sequence. LTRs with mutations that are combinations of additions, substitutions and deletions of nucleotides also qualify as the first LTR functional equivalent. The nucleotide sequence of a mutated LTR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 43.

One functional equivalent of the second LTR is one having the nucleotide sequence set forth as SEQ ID NO: 44. The second LTR functional equivalent includes the nucleotide sequence set forth as SEQ ID NO: 44 having a substitution, deletion or mutation, so long as it can be functionally used as the second LTR. When one or more nucleotides of the nucleotide sequence set forth as SEQ ID NO: 44 are substituted with different nucleotides, the number of substituting nucleotides is preferably 1 to 20, more preferably 1 to 10, yet more preferably 1 to 5 and even more preferably 1 to 3. When one or more nucleotides of the nucleotide sequence set forth as SEQ ID NO: 44 are deleted, the number of deleted nucleotides is preferably 1 to 20, more preferably 1 to 10, yet more preferably 1 to 5 and even more preferably 1 to 3. LTRs with mutations that are combinations of substitutions and deletions of nucleotides also qualify as the second LTR functional equivalent. When one or more nucleotides are added to the nucleotide sequence set forth as SEQ ID NO: 44, preferably 1 to 20, more preferably 1 to 10, yet more preferably 1 to 5 and even more preferably 1 to 3 nucleotides are added within or at the 5'-end or 3'-end of the nucleotide sequence. LTRs with mutations that are combinations of additions, substitutions and deletions of nucleotides also qualify as the second LTR functional equivalent. The nucleotide sequence of a mutated LTR exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 44.

According to one embodiment of the invention, "leader" and "trailer" refer to nucleotide sequences present at the ends of a viral genome, which are partially complementary to each other. The leader and trailer derived from Sendai virus may be preferably used. Both of the Sendai virus leader and trailer are the region with chain length of about 50 nucleotides. According to one embodiment, the leader is located at the 5'-end of the gene encoding the fusion protein, and the trailer is located at the 3'-end. The leader and/or trailer may also be variants each with a modification such as a substitution, deletion or addition in the nucleotide sequence of the wild type, so long as each can still substitute for its respective wild type.

A functional equivalent of a leader is one that can be used as a functional substitute for the leader. A functional equivalent of a leader may be a variant of the LTR of the wild type leader. A leader artificially constructed based on the leader also qualifies as a functional equivalent of the leader, so long as it can substitute for the leader. The same applies for the trailer.

According to a preferred embodiment of the invention, the leader is one derived from Sendai virus having the nucleotide sequence set forth as SEQ ID NO: 45, and the trailer is one derived from Sendai virus having the nucleotide sequence set forth as SEQ ID NO: 46.

A leader and trailer derived from wild type Sendai virus with modifications are also suitable for use for the invention. When one or more nucleotides of a nucleotide sequence are substituted with different nucleotides, the number of substituting nucleotides is preferably 1 to 20, more preferably 1 to 10 and even more preferably 1 to 3. When the nucleotide sequence of a wild type leader and/or trailer has deletions, the number of deleted nucleotides is preferably 1 to 20, more preferably 1 to 10 and even more preferably 1 to 3. Mutations that are combinations of substitutions and deletions of nucleotides may also be added. When one or more nucleotides are added, preferably 1 to 20, more preferably 1 to 10 and even more preferably 1 to 3 nucleotides are added within or at the 5'-end or 3'-end of the nucleotide sequence of the leader and/or trailer. Mutations that are combinations of additions, substitutions and deletions of nucleotides may also be added. The nucleotide sequence of a mutation exhibits preferably identity of not lower than 80%, more preferably identity of not lower than 85%, even more preferably identity of not lower than 90%, yet more preferably identity of not lower than 95% and most preferably identity of not lower than 98%, to the nucleotide sequence of the wild type. The manner of introducing a mutation into a Sendai virus leader and trailer may also be applied for introduction of a mutation into leaders and trailers derived from other viruses.

According to one embodiment of the invention, a nucleotide sequence that may be used as a nucleotide sequence comprising a regulatory elements for regulation of expression of the fusion protein gene include, but are not limited to, cytomegalovirus-derived promoter (optionally including the enhancer), SV40 early promoter, human elongation factor-1α (EF-1α) promoter, human ubiquitin C promoter, retroviral Rous sarcoma virus-LTR promoter, dihydrofolate reductase promoter, β-actin promoter, phosphoglycerate kinase (PGK) promoter, mouse albumin promoter, human albumin promoter and human α-1 antitrypsin promoter, so long as they are able to cause expression of the fusion protein in cells, tissue or the body of the mammal (especially a human) into which the fusion protein-encoding gene is introduced. For example, a synthetic promoter having the nucleotide sequence set forth as SEQ ID NO: 8 including mouse albumin promoter downstream from mouse α-fetoprotein enhancer (mouse α-fetoprotein enhancer/mouse albumin promoter) may be suitably used as a regulatory element of gene expression. Chicken β actin/MVM chimeric intron having the nucleotide sequence set forth as SEQ ID NO: 9 downstream from the mouse α-fetoprotein enhancer/mouse albumin promoter may also be inserted at the downstream end. Inserting such an intron can increase expression level of the protein that is regulated by the regulatory element of gene expression. In the nucleotide sequence set forth as SEQ ID NO: 8, the nucleotide sequence of nucleotides 1 to 219 constitutes the mouse α-fetoprotein enhancer, and the nucleotide sequence of nucleotides 241 to 549 constitutes the mouse albumin promoter.

The gene regulation site may be a promoter for a gene that is to be expressed in an organ-specific or cell type-specific manner. Using an organ-specific expression promoter or cell type-specific expression promoter allows the fusion protein-encoding gene incorporated into the nucleic acid molecule to be expressed specifically in the desired organ or cells. According to one embodiment of the invention, when multiple encoded cistrons are present in the nucleic acid molecule, the gene regulation site is inserted for each cistron.

According to another embodiment, an "internal ribosome entry site" is a region (structure) within an mRNA strand where the ribosome directly binds to begin translation in a cap structure-independent manner, or a region (structure) of a DNA strand that produces such a region by transcription. According to the invention, a "gene encoding an internal ribosome entry site" is a region (structure) of a DNA strand that produces the region by transcription. The internal ribosome entry site (IRES) is found in the 5' untranslated region of a virus, such as a virus belonging to the family Picornaviridae (such as poliovirus, rhinovirus or murine encephalomyocarditis virus), foot-and-mouth disease virus, hepatitis A virus, hepatitis C virus, coronavirus, bovine enterovirus, Cyler's murine encephalomyelitis virus or Coxsackievirus group B, or the 5' untranslated region of a gene such as human immunoglobulin heavy chain binding protein, Drosophila Antennapedia or Drosophila Ultrabithorax. In the case of a picornavirus, IRES is the region of approximately 450 bp in the 5' untranslated region of the mRNA. The "5' untranslated region of a virus" is the 5' untranslated region of the viral mRNA, or the region (structure) of the DNA strand that produces the region by transcription.

According to one embodiment, the internal ribosome entry site is not particularly restricted so long as it functions as an internal ribosome entry site in cells, tissue or the body of a mammal (especially a human), and any such one may be used. It is preferably an internal ribosome entry site from a viral 5' untranslated region of a virus, more preferably an internal ribosome entry site from the 5' untranslated region of a Picornaviridae virus, and even more preferably an internal ribosome entry site from the 5' untranslated region of murine encephalomyocarditis virus. One embodiment of an internal ribosome entry site from the 5' untranslated region of murine encephalomyocarditis virus is one having the nucleotide sequence set forth as SEQ ID NO: 10.

According to one embodiment, the internal ribosome entry site having the wild type nucleotide sequence may be used directly. The nucleotide sequence of the wild type internal ribosome entry site may be a mutant internal ribosome entry site having one or more mutations (such as substitutions, deletions and/or insertions), so long as it functions as an internal ribosome entry site in cells, tissue or the body of a mammal (especially a human). The internal ribosome entry site used may also be a chimeric type obtained by fusion of two or more internal ribosome entry sites.

According to one embodiment, the protein in a fusion protein of an antibody and a protein having physiological activity is not particularly restricted so long as it exhibits physiological activity in the body. Preferred proteins having physiological activity include those that are to be administered to a patient for long-term use as a medicine. Examples of medicines include growth hormone, lysosomal enzymes, somatomedin, insulin, glucagon, cytokines, lymphokines, blood coagulation factors, antibodies, fusion proteins of antibodies and other proteins, granulocyte-macrophage colony-stimulating factor (GM-CSF), granulocyte-colony stimulating factor (G-CSF), macrophage colony-stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, DNasel, thyroid-stimulating hormone (TSH), nerve growth factor (NGF), ciliary neurotrophic factor (CNTF), glial cell-line neurotrophic factor (GDNF), neurotrophin 3, neurotrophin 4/5, neurotrophin 6, neuregulin 1, activin, basic fibroblast growth factor (bFGF), fibroblast growth factor 2 (FGF2), epidermal growth factor (EGF), vascular endothelial growth factor (VEGF), interferon-α, interferon-β, interferon-γ, interleukin-6, PD-1, PD-1 ligand, tumor necrosis factor-α receptor (TNF-α receptor), enzymes having beta amyloid degrading activity, etanercept, pegvisomant, metreleptin, abatacept, asfotase and GLP-1 receptor agonist, as well as antibody medicines.

Preferred examples where the protein having physiological activity is a lysosomal enzyme include α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, palmitoyl protein thioesterase-1, tripeptidyl peptidase-1, hyaluronidase-1, CLN1 and CLN2.

According to one embodiment of the invention, the protein having physiological activity is a human protein. The protein may be a wild type, or it may be a mutated type so long as it has the original physiological activity of the protein. Herein when referring to a protein having the original physiological activity, it means that the protein has at least 20% of the physiological activity of the wild type protein. The percentage of physiological activity relative to the physiological activity of the wild type protein is more preferably at least 40%, even more preferably at least 50%, yet more preferably at least 80% and most preferably at least 90%.

When amino acids of the amino acid sequence of the protein having the physiological activity of the wild type are substituted with other amino acids, the number of substituted amino acids is preferably 1 to 20, more preferably 1 to 10 and even more preferably 1 to 3. When amino acids of the wild type protein are deleted, the number of deleted amino acids is preferably 1 to 20, more preferably 1 to 10 and even more preferably 1 to 3. Mutations that are combinations of substitutions and deletions of amino acids may also be added. When amino acids are added to the wild type protein, preferably 1 to 20, more preferably 1 to 10 and even more preferably 1 to 3 amino acids are added within or at the N-terminus or C-terminus of the amino acid sequence of the protein. Mutations that are combinations of additions, substitutions and deletions of amino acids may also be added. The amino acid sequence of a mutated protein has preferably identity of not lower than 80%, more preferably identity of not lower than 85%, even more preferably identity of not lower than 90%, yet more preferably identity of not lower than 95% and most preferably identity of not lower than 98%, to the amino acid sequence of the wild type protein.

According to the invention, the locations and types (deletion, substitution or addition) of mutations to the wild type protein can be easily confirmed by alignment of the amino acid sequences of the wild type and mutant proteins. Identity between the amino acid sequence of the wild type protein and the amino acid sequence of the mutant protein can be easily calculated using a well-known homology calculation algorithm. Examples of such algorithms include BLAST (Altschul SF. J Mol. Biol. 215. 403-10, (1990)), the similarity search method of Pearson and Lipman (Proc. Natl. Acad. Sci. USA. 85. 2444 (1988)), and the local homology algorithm of Smith and Waterman (Adv. Appl. Math. 2. 482-9(1981)).

Substitution of an amino acid in the amino acid sequence of the protein with other amino acid occur, for example, within an amino acid family including amino acids having related side chains or chemical properties. It is predicted that substitutions within such an amino acid family will not significantly alter the protein function (i.e. conservative amino acid substitution). Examples of such an amino acid family include the following:
(1) the acidic amino acids aspartic acid and glutamic acid,
(2) the basic amino acids histidine, lysine and arginine,
(3) the aromatic amino acids phenylalanine, tyrosine and tryptophan,
(4) the hydroxyl group-containing amino acids (hydroxy amino acids) serine and threonine,
(5) the hydrophobic amino acids methionine, alanine, valine, leucine and isoleucine,
(6) the neutral hydrophilic amino acids cysteine, serine, threonine, asparagine and glutamine,
(7) amino acids that affect orientation on the peptide chain, such as glycine and proline,
(8) the amide-type amino acids (polar amino acids) asparagine and glutamine,
(9) the aliphatic amino acids alanine, leucine, isoleucine and valine,
(10) amino acids with small side chains, such as alanine, glycine, serine and threonine, and
(11) amino acids with especially small side chains, such as alanine and glycine.

The antibody in the fusion protein of a ligand and a protein having physiological activity according to the invention will now be explained in detail.

According to the invention, a "ligand" is a protein that specifically binds to a target protein. An antibody is included in the definition of ligand. The protein that is the target of a ligand according to one embodiment is a protein present on the surfaces of vascular endothelial cells, and especially cerebrovascular endothelial cells. Examples of proteins present on the surfaces of vascular endothelial cells include transferrin receptor (TfR), insulin receptor (IR), leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor. OATP-F is an example of an organic anion transporter, and MCT-8 is an example of a monocarboxylic acid transporter. TfR and IR are particularly preferred targets among these proteins present on the surfaces of vascular endothelial cells, with TfR being a more preferred target.

Preferred examples of ligands other than antibodies include transferrin, insulin, leptin, insulin-like growth factor I, insulin-like growth factor II, lipoprotein and low density lipoprotein. Transferrin and insulin are especially preferred ligands, with transferrin being a more preferred ligand. These ligands may be wild type proteins, or they may be fragments or variants thereof so long as they have the function of specifically binding to the target proteins. The fusion protein of a ligand and a protein having physiological activity is able to bind to the target on the surfaces of vascular endothelial cells. When the vascular endothelial cells are cerebrovascular endothelial cells, this allows them to cross the BBB to reach central nervous system tissue.

The animal species of the antibody is not particularly restricted so long as it has the property of specifically binding to antigen, but a human antibody or humanized antibody is preferred. For example, the antibody may be an antibody of a mammal other than a human, or it may be a chimeric antibody of a human antibody and an antibody of a mammal other than a human.

A human antibody is an antibody that is entirely encoded by a human gene. However, an antibody encoded by a gene having a mutation to an original human gene for the purpose of increasing efficiency of expression of the gene is also included within the definition of "human antibody". An antibody in which two or more genes coding for human antibodies are combined, with part of one human antibody replacing part of another human antibody, is also a human antibody. A human antibody has three complementarity determining regions (CDR) on the immunoglobulin light chain, and three complementarity determining regions (CDR) on the immunoglobulin heavy chain. The three CDRs of the immunoglobulin light chain are designated as CDR1, CDR2 and CDR3, in order from the N-terminal end. The three CDRs of the immunoglobulin heavy chain are likewise designated as CDR1, CDR2 and CDR3, in order from the N-terminal end. An antibody in which the antigen specificity or affinity of a human antibody is modified by replacing a CDR of one human antibody with a CDR of another human antibody, is also a human antibody.

According to the invention, an antibody in which a mutation such as a substitution, deletion or addition has been added to the amino acid sequence of the original antibody by modification of the gene of the original human antibody, is also referred to as a "human antibody". When amino acids of the amino acid sequence of an original antibody are substituted with other amino acids, the number of substituted amino acids is preferably 1 to 20, more preferably 1 to 10, even more preferably 1 to 5 and yet more preferably 1 to 3. When amino acids of the amino acid sequence of an original antibody are deleted, the number of deleted amino acids is preferably 1 to 20, more preferably 1 to 10, even more preferably 1 to 5 and yet more preferably 1 to 3. An antibody with a mutation that is a combination of a substitution and a deletion of amino acids is also a human antibody. When amino acids are added, preferably 1 to 20, more preferably 1 to 10, even more preferably 1 to 5 and yet more preferably 1 to 3 amino acids are added within or at the N-terminal end or C-terminal end of the amino acid sequence of the original antibody. An antibody with a mutation that is a combination of an addition, substitution and deletion of amino acids is also a human antibody. The amino acid sequence of a mutated antibody has preferably identity of not lower than 80%, more preferably identity of not lower than 90%, even more preferably identity of not lower than 95% and yet more preferably identity of not lower than 98%, to the amino acid sequence of the original antibody. That is, a "human-derived gene" for the purpose of the invention includes the original human-derived gene, but also genes obtained by adding modifications to the original human-derived gene.

According to the invention, the term "humanized antibody" refers to an antibody wherein the amino acid sequence of part of the variable region (for example, all or part of the CDR) is derived from a mammal other than a human, and the rest of the region is human-derived. For example, the humanized antibody may be an antibody constructed by replacing the three complementarity determining regions (CDR) on the immunoglobulin light chain and the three complementarity determining regions (CDR) on the immunoglobulin heavy chain of a human antibody with CDRs of another mammal. The species of the other mammal from which the CDRs transplanted into the appropriate sites of the human antibody are derived is not particularly restricted so long as it is a mammal other than a human, but it is preferably a mouse, rat, rabbit, horse or non-human primate, and preferably a mouse or rat, such as a mouse.

According to the invention, the following explanation applies when the antibody is a human antibody or humanized antibody. The light chains of a human antibody or humanized antibody are the λ chain and κ chain. The light chains used to form the antibody may be either λ chains or κ chains. The heavy chains of a human antibody or humanized antibody are the γ chain, µ chain, α chain, β chain and ε chain, corresponding to IgG, IgM, IgA, IgD and IgE, respectively. The heavy chains composing the antibody may be γ chains, µ chains, α chains, σ chains or ε chains, and are preferably γ chains. The γ chains as heavy chains of an antibody include the γ1 chain, γ2 chain, γ3 chain and γ4 chain, corresponding to IgG1, IgG2, IgG3 and IgG4, respectively. When the heavy chains composing the antibody are γ chains, the γ chains may be γ1 chains, γ2 chains, γ3 chains or γ4 chains and are preferably γ1 chains or γ4 chains. When the antibody is a humanized antibody or human antibody and is IgG, the light chains of the antibody may be either λ chains or κ chains, and the heavy chains of the antibody may be γ1 chains, γ2 chains, γ3 chains or γ4 chains, but γ1 chains and γ4 chains are preferred. As an example of a preferred mode of the antibody, the light chains are λ chains and the heavy chains are γ1 chains.

According to the invention, the term "chimeric antibody" refers to an antibody wherein two or more different antibody fragments derived from two or more different species are linked.

A chimeric antibody of a human antibody and an antibody of another mammal is an antibody wherein part of a human antibody has been replaced by part of an antibody of a mammal other than a human. The antibody comprises an Fc region, a Fab region and a hinge region, as explained below. Specific examples of such chimeric antibodies include chimeric antibodies wherein the Fc region is derived from a human antibody while the Fab region is derived from an antibody of another mammal. Conversely, it may be a chimeric antibody wherein the Fc region is derived from another mammal and the Fab region is derived from a human antibody. The hinge region may be derived from either the human antibody or the antibody of the other mammal. The same applies for a humanized anti-TfR antibody.

The antibody may also be considered to comprise a variable region and a constant region. Other specific examples of chimeric antibodies include antibodies wherein the heavy chain constant region (C_{H}) and the light chain constant region (C_{L}) are derived from a human antibody and the heavy chain variable region (V_{H}) and the light chain variable region (V_{L}) are derived from an antibody of another mammal, or conversely, wherein the heavy chain constant region (C_{H}) and the light chain constant region (C_{L}) are derived from an antibody of another mammal and the heavy chain variable region (V_{H}) and light chain variable region (V_{L}) are derived from a human antibody. The species of the other mammal is not particularly restricted so long as it is a mammal other than a human, but it is preferably a mouse, rat, rabbit, horse or non-human primate, and more preferably a mouse. The same applies for a humanized anti-TfR antibody.

A chimeric antibody of a human antibody and a mouse antibody is typically referred to as a "human/mouse chimeric antibody". A human/mouse chimeric antibody may be a chimeric antibody wherein the Fc region is derived from a human antibody and the Fab region is derived from a mouse antibody, or conversely, a chimeric antibody wherein the Fc region is derived from a mouse antibody and the Fab region is derived from a human antibody. The hinge region may be derived from either the human antibody or the mouse antibody. Other specific examples of human/mouse chimeric antibodies include an antibody wherein the heavy chain constant region (C_{H}) and the light chain constant region (C_{L}) are derived from a human antibody and the heavy chain variable region (V_{H}) and the light chain variable region (V_{L}) are derived from a mouse antibody, or conversely, wherein the heavy chain constant region (C_{H}) and the light chain constant region (C_{L}) are derived from a mouse antibody and the heavy chain variable region (V_{H}) and light chain variable region (V_{L}) are derived from a human antibody. The same applies for a humanized anti-TfR antibody.

An antibody originally has a basic construction comprising two immunoglobulin light chains and two immunoglobulin heavy chains, for a total of 4 polypeptide chains. As used herein, however, in addition to that having a basic construction, the term "antibody" includes the following basic structures:
(1) an antibody comprising one immunoglobulin light chain and one immunoglobulin heavy chain, for a total of two polypeptide chains, and
   more specifically described hereafter:
   (2) a single-stranded antibody having a linker bonded to the C-terminal end of the immunoglobulin light chain and further the immunoglobulin heavy chain consecutively bonded to the C-terminal end,
   (3) a single-stranded antibody having a linker bonded to the C-terminal end of the immunoglobulin heavy chain and further the immunoglobulin light chain consecutively bonded to the C-terminal end,
   (4) a single-stranded antibody (scFv) having a linker bonded to the C-terminal end of the variable region of the immunoglobulin heavy chain and the variable region of the immunoglobulin light chain bonded to the C-terminal end, and
   (5) a single-stranded antibody (scFv) having a linker bonded to the C-terminal end of the variable region of the immunoglobulin light chain and further the variable region of the immunoglobulin heavy chain bonded to the C-terminal end. Furthermore:
   (6) an antibody consisting of the Fab region and lacking the Fc region from the original basic structure antibody, and an antibody consisting of the Fab region and all or part of the hinge region (including Fab, F(ab') and F(ab')₂), as well as
   (7) a single-domain antibody is also included within the definition of "antibody" according to the invention. Also included in the scope of "antibody" according to the invention is a single-stranded antibody scFv, obtained by bonding the light chain variable region and the heavy chain variable region via a linker.

The term "linker" as used herein means multiple amino acids bonded by peptide bonds into a peptide chain. The linker consisting of such a peptide chain may also be referred to as a "peptide linker". The "linker" may also be referred to as "linker sequence", depending on context. By bonding the N-terminus of the linker to the C-terminus of another protein by a peptide bond, and bonding the N-terminus of yet another protein to the C-terminus of the linker, it is possible to form a conjugate of the two proteins via the linker.

An antibody having the basic structure composed of two light chains and two heavy chains for a total of 4 polypeptide chains has three complementarity determining regions (CDR) in the light chain variable region (V_{L}) and three complementarity determining regions (CDR) in the heavy chain variable region (V_{H}). The three CDRs of the light chain are designated as CDR1, CDR2 and CDR3, in order from the N-terminal end. The three CDRs of the heavy chain are likewise designated as CDR1, CDR2 and CDR3, in order from the N-terminal end. However, an antibody wherein all or portions of the CDRs are incomplete or lacking is still an antibody so long as it has the property of specifically binding to a specific antigen. The regions other than the CDRs of the light chain and heavy chain variable regions (V_{L} and V_{H}) are referred to as framework regions (FR). The FRs include FR1, FR2, FR3 and FR4, in order from the N-terminal end. The CDRs and FRs are usually present in the order: FR1, CDR1, FR2, CDR2, FR3, CDR3, FR4, from the N-terminal end.

According to one embodiment of the invention, Fab is a molecule wherein one light chain including a variable region and the C_{L} region (light chain constant region) and one heavy chain including a variable region and the C_{H}1 region (heavy chain constant region 1) are bonded by disulfide bonds at their respective cysteine residues. In Fab, the heavy chain may further include part of the hinge region in addition to the variable region and C_{H}1 region (heavy chain constant region 1), in which case the hinge region must lack the cysteine residues that are normally present and responsible for binding the heavy chains of the antibody together. In Fab, the light chain and heavy chain are bonded by disulfide bonds formed between the cysteine residues present in the light chain constant region (C_{L} region) and the cysteine residues present in the heavy chain constant region (C_{H}1 region) or hinge region. The heavy chain forming the Fab is referred to as the Fab heavy chain. Since Fab lacks cysteine residues that are in the hinge region and responsible for binding the heavy chains of the antibody together, it consists of one light chain and one heavy chain. The light chain forming the Fab includes a variable region and the C_{L} region. The heavy chain forming Fab may be one consisting of the variable region and the C_{H}1 region, or it may include part of the hinge region in addition to the variable region and the C_{H}1 region. In this case, however, the hinge region is selected so that it contains no cysteine residues that might cause binding between the heavy chains, so that disulfide bonds do not form between the two heavy chains in the hinge region. In F(ab'), the heavy chain includes, in addition to the variable region and the C_{H}1 region, also all or part of a hinge region that includes cysteine residues that cause binding between the heavy chains. F(ab')₂ is a molecule wherein two F(ab') fragments are bonded by disulfide bonds at cysteine residues present in the hinge region. The heavy chain forming F(ab') or F(ab')₂ is referred to as the Fab' heavy chain. A polymer such as a dimer or trimer that consists of multiple antibodies bonded either directly or via a linker is also considered to be an antibody. Without limitation to these, however, the concept of "antibody" according to the invention also encompasses any molecule that includes part of an antibody molecule and has the property of specifically binding to an antigen. In other words, the term "light chain" as used herein includes any chain that derives from a light chain and has part or the entirety of the amino acid sequence of its variable region. Likewise, the term "heavy chain" includes any chain that derives from a heavy chain and has part or the entirety of the amino acid sequence of its variable region. Therefore, a heavy chain that lacks the Fc region, for example, is still a heavy chain so long as it has all or part of the amino acid sequence of the variable region.

The Fc or Fc region referred to here is a region of the antibody molecule that includes a fragment consisting of the C_{H}2 region (the heavy chain constant region 2) and the C_{H}3 region (the heavy chain constant region 3).

An antibody according to one embodiment of the invention also includes:
(8) scFab, scF(ab') and scF(ab')₂, which are single-stranded antibodies obtained by bonding the respective light chains and heavy chains composing Fab, F(ab') or F(ab')₂ described in (6) above, via a linker sequence. For scFab, scF(ab') and scF(ab')₂, each may be one having a linker sequence bonded at the C-terminal end of the light chain, and a heavy chain further bonded to its C-terminal end, or one having a linker bonded to the C-terminal end of the heavy chain and a light chain further bonded to its C-terminal end. Also included in the scope of "antibody" according to the invention is a single-stranded antibody scFv, obtained by bonding the light chain variable region and the heavy chain variable region via a linker. For scFv, it may be one having a linker sequence bonded to the C-terminal end of a light chain variable region and a heavy chain variable region further bonded to its C-terminal end, or it may be one having a linker sequence bonded to the C-terminal end of a heavy chain variable region and a light chain variable region further bonded to its C-terminal end.

The term "antibody" as used herein also includes, in addition to full length antibodies as the antibodies described by (1) to (8) above, also any form of antigen-binding fragment (antibody fragment) lacking part of the full length antibodies, as a wider concept that includes (1) to (8). Antigen-binding fragments include heavy chain antibodies, light chain antibodies, VHH and VNAR, as well as the same lacking portions thereof.

The term "antigen-binding fragment" is a fragment of an antibody that retains at least part of the specific binding activity with antigen. Examples of binding fragments include Fab, Fab', F(ab')₂, a variable region (Fv), a single-stranded antibodies (scFv) having , heavy chain variable region (V_{H}) and light chain variable region (V_{L}) linked with an appropriate linker, a diabody that is a polypeptide dimer including a heavy chain variable region (V_{H}) and a light chain variable region (V_{L}), a minibody that is a dimer with part of a constant region (C_{H}3) bonded to a scFv heavy chain (H chain), and any one of other low molecular weight antibodies. There is no limitation to these, however, so long as the fragment has the ability to bind with antigen.

According to the invention, "single-stranded antibody" refers to a protein that has a linker bonded at the C-terminal end of an amino acid sequence including all or part of the variable region of an immunoglobulin light chain, and further has an amino acid sequence including all or part of the variable region of an immunoglobulin heavy chain bonded to its C-terminal end, and that is able to bind specifically to a specific antigen. Moreover, proteins that have a linker bonded at the C-terminal end of an amino acid sequence including all or part of the variable region of an immunoglobulin heavy chain, and further have an amino acid sequence including all or part of the variable region of an immunoglobulin light chain bonded to its C-terminal end, and that are able to bind specifically to a specific antigen, are also "single-stranded antibodies" according to the invention. For example, the antibodies described by (2) and (3) above are included in the definition of "single-stranded antibody". In a single-stranded antibody having an immunoglobulin light chain bonded to the C-terminal end of an immunoglobulin heavy chain via a linker, the immunoglobulin heavy chain usually has the Fc region deleted. The variable region of the immunoglobulin light chain has three complementarity determining regions (CDR) that contribute to the antigen specificity of the antibody. The variable region of the immunoglobulin heavy chain likewise has three CDRs. The CDRs are the main regions that determine the antigen specificity of the antibody. A single-stranded antibody therefore preferably includes all of the three CDRs of the immunoglobulin heavy chain and all of the three CDRs of the immunoglobulin light chain. However, the single-stranded antibody may lack one or more CDRs so long as the antigen-specific affinity of the antibody is maintained.

In a single-stranded antibody, the linker situated between the light chain and heavy chain of an immunoglobulin is a peptide chain composed of amino acid residues in a number of preferably 2 to 50, more preferably 8 to 50, even more preferably 10 to 30 and yet preferably 12 to 18 or 15 to 25, such as either 15 or 25. Such a linker is not limited to such an amino acid sequence, so long as an anti-hTfR antibody having both chains linked by it still retains affinity with hTfR, but it is preferably composed of glycine alone or of glycine and serine, having the amino acid sequence Gly-Ser, the amino acid sequence Gly-Gly-Ser, the amino acid sequence Gly-Gly-Gly, the sequence set forth as SEQ ID NO: 1, SEQ ID NO: 2, SEQ ID NO: 3 or SEQ ID NO: 13, or a sequence with these amino acid sequences repeated 2 to 10 times or 2 to 5 times. For example, when the variable region of an immunoglobulin light chain is bonded via a linker to the C-terminal end of the amino acid sequence consisting of the entire region of the variable region of the immunoglobulin heavy chain, to obtain ScFV, it is preferred to use a linker having the sequence of SEQ ID NO: 13.

According to one embodiment of the invention, the antibody is an antibody derived from an animal belonging to the family Camelidae (which includes alpacas). Some antibodies of Camelidae animals have two heavy chains linked by disulfide bonds. Such antibodies having two heavy chains are referred to as heavy chain antibodies. VHH is an antibody consisting of one heavy chain that includes the heavy chain variable region forming a heavy chain antibody, or an antibody consisting of one heavy chain lacking the constant region (CH) forming a heavy chain antibody. VHH is also a type of antibody according to this embodiment of the invention. A Camelidae animal-derived antibody (such as VHH) having a mutation added to the amino acid sequence to reduce antigenicity when the antibody is administered to a human, is also an antibody according to one embodiment of the invention. When one or more mutations are to be added to amino acids in a Camelidae animal antibody, the same types of mutations may be added as for other antibodies described herein. An antibody consisting of two light chains linked by disulfide bonds is also an antibody according to this embodiment of the invention. Such antibodies having two light chains are referred to as light chain antibodies.

The antibody according to one embodiment of the invention is a shark-derived antibody. Shark antibodies have two heavy chains linked by disulfide bonds. Such antibodies having two heavy chains are referred to as heavy chain antibodies. VNAR is an antibody consisting of one heavy chain that includes the heavy chain variable region forming a heavy chain antibody, or an antibody consisting of one heavy chain lacking the constant region (CH) forming a heavy chain antibody. VNAR is also a type of antibody according to this embodiment of the invention. A shark-derived antibody (such as VNAR) having a mutation added to the amino acid sequence to reduce antigenicity when the antibody is administered to a human, is also an antibody according to one embodiment of the invention. When mutations are to be added to amino acids in a shark antibody, the same types of mutations may be added as for other antibodies described herein. Humanized shark antibodies are also a type of antibody according to this embodiment.

According to one embodiment, a single-domain antibody is an antibody having the property of specifically binding to antigen at a single variable region. Single-domain antibodies include antibodies wherein the variable region consists entirely of a heavy chain variable region (heavy chain single-domain antibody) and antibodies wherein the variable region consists entirely of a light chain variable region (light chain single-domain antibody). VHH and VNAR are types of single-domain antibodies.

According to one embodiment, the antibody in a fusion protein of an antibody and a protein having physiological activity is not particularly restricted so long as it exhibits affinity for a specific antigen in the body. For example, the antibody may be one having specific affinity for a protein present on the surfaces of vascular endothelial cells. Examples of proteins present on the surfaces of vascular endothelial cells include transferrin receptor (TfR), insulin receptor (IR), leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor. OATP-F is an example of an organic anion transporter, and MCT-8 is an example of a monocarboxylic acid transporter. TfR and IR are particularly preferred targets among these proteins present on the surfaces of vascular endothelial cells, with TfR being a more preferred target. When the antibody is one that binds with a protein present on the surfaces of vascular endothelial cells, the fusion protein is able to exhibit its physiological activity after having been taken up into vascular endothelial cells and reaching tissues, and therefore the fusion protein can be used as a medicine that must exhibit its drug effect in various tissues. For example, when the antibody has specific affinity for TfR or IR, the fusion protein may be used as a medicine that must exhibit its drug effect in muscles.

According to one embodiment, the antibody is one having specific affinity for a protein present on the surfaces of cerebrovascular endothelial cells. Examples of proteins present on the surfaces of cerebrovascular endothelial cells include transferrin receptor (TfR), insulin receptor (IR), leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor. OATP-F is an example of an organic anion transporter, and MCT-8 is an example of a monocarboxylic acid transporter. TfR and IR are particularly preferred targets among these proteins present on the surfaces of vascular endothelial cells, with TfR being a more preferred target. When the antibody is one that binds with a protein present on the surfaces of cerebrovascular endothelial cells, the fusion protein is able to exhibit its physiological activity after having crossed the BBB and reached brain tissue, and therefore the fusion protein can be used as a medicine that must exhibit its drug effect in the brain.

According to the invention, "human transferrin receptor" or "hTfR" refers to a membrane protein having the amino acid sequence set forth as SEQ ID NO: 11. According to one embodiment, the anti-hTfR antibody of the invention is one that specifically binds to the portion of the amino acid sequence set forth as SEQ ID NO: 11 from the cysteine residue at position 89 from the N-terminal end to phenylalanine at the C-terminal end (the extracellular domain of hTfR), but this is not limitative.

The light chains of the anti-hTfR antibody (Fab) according to one embodiment are the amino acid sequence set forth as SEQ ID NO: 31 or 32 as the amino acid sequence of the light chain CDR1 of the anti-hTfR antibody, the amino acid sequence set forth as SEQ ID NO: 33 or 34 as the amino acid sequence of the light chain CDR2 of the hTfR antibody, and the amino acid sequence set forth as SEQ ID NO: 35 as the amino acid sequence of the light chain CDR3 of the hTfR antibody. The heavy chains of the anti-hTfR antibody (Fab) according to one embodiment are the amino acid sequence set forth as SEQ ID NO: 36 or 37 as the amino acid sequence of the heavy chain CDR1 of the anti-hTfR antibody, the amino acid sequence set forth as SEQ ID NO: 38 or 39 as the amino acid sequence of the heavy chain CDR2 of the hTfR antibody, and the amino acid sequence set forth as SEQ ID NO: 40 or 41 as the amino acid sequence of the heavy chain CDR3 of the hTfR antibody.

The anti-hTfR antibody, which is Fab according to one embodiment, includes a light chain having the amino acid sequence set forth as SEQ ID NO: 15 and a heavy chain having the amino acid sequence set forth as SEQ ID NO: 16.

The form of bonding of the antibody and the protein having physiological activity in the fusion protein of the antibody and the protein having physiological activity may be any of the following (a) to (h):
(a) the antibody is a single-stranded antibody having the light chain of the antibody bonded to the C-terminal end of the heavy chain of the antibody, and the protein having physiological activity is bonded to the N-terminal end of the single-stranded antibody either directly or via a linker,
(b) the antibody is a single-stranded antibody having the light chain of the antibody bonded to the C-terminal end of the heavy chain of the antibody, and the protein having physiological activity is bonded to the C-terminal end of the single-stranded antibody either directly or via a linker,
(c) the antibody is a single-stranded antibody having the heavy chain of the antibody bonded to the C-terminal end of the light chain of the antibody, and the protein having physiological activity is bonded to the N-terminal end of the single-stranded antibody either directly or via a linker,
(d) the antibody is a single-stranded antibody having the heavy chain of the antibody bonded to the C-terminal end of the light chain of the antibody, and the protein having physiological activity is bonded to the C-terminal end of the single-stranded antibody either directly or via a linker,
(e) the antibody is an antibody including at least one light chain and at least one heavy chain, and the protein having physiological activity is bonded to the C-terminal end of the light chain of the antibody either directly or via a linker,
(f) the antibody is an antibody including at least one light chain and at least one heavy chain, and the protein having physiological activity is bonded to the N-terminus of the light chain of the antibody side either directly or via a linker,
(g) the antibody is an antibody including at least one light chain and at least one heavy chain, and the protein having physiological activity is bonded to the C-terminal end of the heavy chain of the antibody either directly or via a linker, and
(h) the antibody is an antibody including at least one light chain and at least one heavy chain, and the protein having physiological activity is bonded to the N-terminus of the heavy chain of the antibody side either directly or via a linker.

In (a) to (h) above, when a linker is situated within the fusion protein of the antibody and the protein having physiological activity, its sequence is composed of preferably 1 to 50, more preferably 1 to 17, even more preferably 1 to 10 and yet more preferably 1 to 5 amino acids, but the number of amino acids composing the linker may be appropriately adjusted to 1, 2, 3, 1 to 17, 1 to 10, 10 to 40, 20 to 34, 23 to 31 or 25 to 29, for example, depending on the human lysosomal enzyme to be bonded to the antibody. The amino acid sequence of such a linker is not particularly restricted so long as the linked antibody retains affinity with antigen and the protein linked by the linker can exhibit its physiological activity under physiological conditions. The amino acid sequence may consist of glycine and serine, such as a single amino acid which is either glycine or serine, or the amino acid sequence Gly-Ser, or the amino acid sequence Gly-Gly-Ser, or any of the sequences set forth as SEQ ID NO: 1, SEQ ID NO: 2, SEQ ID NO: 3 or SEQ ID NO: 13, or a sequence consisting of 2 to 50 amino acids with 2 to 10 or 2 to 5 contiguous amino acids from aforementioned amino acid sequences, or a sequence consisting of 2 to 17, 2 to 10, 10 to 40, 20 to 34, 23 to 31 or 25 to 29 amino acids. For example, suitable linkers include one having the amino acid sequence Gly-Ser, one having the amino acid sequence set forth as SEQ ID NO: 13, and one consisting of 17 amino acids with Gly-Ser added to the C-terminus of the amino acid sequence set forth as SEQ ID NO: 13.

For convenience, a linker connecting a light chain or heavy chain of an antibody with a protein having physiological activity will be referred to herein simply as "linker" or "first linker", and a linker connecting a light chain with a heavy chain in a single stranded antibody will be referred to as "second linker".

The nucleic acid molecule according to one embodiment of the invention includes a gene encoding a fusion protein of an antibody and a protein having physiological activity, between a first inverted terminal repeat (ITR) and a second inverted terminal repeat (ITR). The following (1) to (4) are examples of such nucleic acid molecules:
(1) the fusion protein includes an antibody light chain and a conjugate of a protein having physiological activity bonded to the C-terminal end or N-terminal end of an antibody heavy chain either directly or via a linker, and the nucleic acid molecule includes a nucleotide sequence encoding an internal ribosome entry site downstream of the gene encoding the light chain, and a nucleotide sequence encoding the conjugate even further downstream thereof;
(2) the fusion protein includes an antibody light chain and a conjugate of a protein having physiological activity bonded to the C-terminal end or N-terminal end of an antibody heavy chain either directly or via a linker, and the nucleic acid molecule includes a nucleotide sequence encoding an internal ribosome entry site downstream of the gene encoding the conjugate, and a sequence encoding the light chain even further downstream thereof;
(3) the fusion protein includes an antibody heavy chain and a conjugate of a protein having physiological activity bonded to the C-terminal end or N-terminal end of an antibody light chain either directly or via a linker, and the nucleic acid molecule includes a nucleotide sequence encoding an internal ribosome entry site downstream of the gene encoding the heavy chain and a nucleotide sequence encoding the conjugate even further downstream thereof; and
(4) the fusion protein includes an antibody heavy chain and a conjugate of a protein having physiological activity bonded to the C-terminal end or N-terminal end of an antibody light chain either directly or via a linker, and the nucleic acid molecule includes a nucleotide sequence encoding an internal ribosome entry site downstream of the gene encoding the conjugate, and a nucleotide sequence encoding the heavy chain even further downstream thereof.

In (1) to (4) above, the nucleic acid molecule may be one that includes the nucleotide sequence of a regulatory element of gene expression between the first inverted terminal repeat (ITR) and the fusion protein-encoding gene. Also in (1) to (4) above, the nucleotide sequence encoding the internal ribosome entry site may be replaced by a nucleotide sequence that includes the regulatory element of gene expression. When the nucleic acid molecule has two regulatory elements of gene expression, they will be referred to as the first regulatory element of gene expression and first regulatory element of gene expression, in order from the first inverted terminal repeat (ITR) end. In (1) to (4) above, the nucleotide sequence encoding the internal ribosome entry site may be replaced by a nucleotide sequence encoding a 2A auto-cleaving peptide. The 2A peptide derived from porcine teschovirus is a suitable example of a 2A auto-cleaving peptide.

The nucleic acid molecule according to one embodiment of the invention, when the antibody is a single-stranded antibody, includes a gene encoding a fusion protein of a single-stranded antibody and a protein having physiological activity, between a first inverted terminal repeat (ITR) and a second inverted terminal repeat (ITR). The following (1) to (4) are examples of such nucleic acid molecules:
(1) the nucleic acid molecule includes a gene encoding a fusion protein having a light chain bonded to the C-terminal end of the heavy chain of an antibody via a second linker, and a protein having physiological activity further bonded to the C-terminal end of the light chain either directly or via a linker;
(2) the nucleic acid molecule includes a gene encoding a fusion protein having a heavy chain bonded to the C-terminal end of the light chain of an antibody via a second linker, and a protein having physiological activity further bonded to the C-terminal end of the light chain either directly or via a linker;
(3) the nucleic acid molecule includes a gene encoding a fusion protein having an antibody heavy chain bonded to the C-terminus end of a protein having physiological activity either directly or via a linker, and a light chain further bonded to the C-terminal end of the heavy chain;
(4) the nucleic acid molecule includes a gene encoding a fusion protein having an antibody light chain bonded to the C-terminus end of a protein having physiological activity either directly or via a linker, and a heavy chain further bonded to the C-terminal end of the heavy chain.

In (1) to (4) above, the nucleic acid molecule may be one that includes the nucleotide sequence of a regulatory element of gene expression between the first inverted terminal repeat (ITR) and the fusion protein-encoding gene.

One embodiment of a nucleic acid molecule including a gene encoding a fusion protein of an antibody and a protein having physiological activity, between a first inverted terminal repeat (ITR) and a second inverted terminal repeat (ITR), wherein the fusion protein includes an antibody light chain and a conjugate of the protein having physiological activity bonded to the C-terminal end of the heavy chain of the antibody, and the nucleic acid molecule includes a nucleotide sequence encoding an internal ribosome entry site downstream of the gene encoding the light chain and the gene encoding the conjugate further downstream thereof, is a nucleic acid molecule wherein the conjugate is one having human iduronate-2-sulfatase (hI2S) bonded downstream of the Fab region of the heavy chain of anti-hTfR antibody via a linker, and the antibody light chain is the light chain of anti-hTfR antibody. A specific example is one wherein the Fab region of the heavy chain of anti-hTfR antibody includes the amino acid sequence set forth as SEQ ID NO: 12, the linker includes the amino acid sequence set forth as SEQ ID NO: 13, hI2S includes the amino acid sequence set forth as SEQ ID NO: 14, and the light chain of the anti-hTfR antibody includes the amino acid sequence set forth as SEQ ID NO: 15. The conjugate in this case includes the entirety of the amino acid sequence set forth as SEQ ID NO: 42.

One embodiment of a nucleic acid molecule including a gene encoding a fusion protein of an antibody and a protein having physiological activity, between a first inverted terminal repeat (ITR) and a second inverted terminal repeat (ITR), wherein the fusion protein includes an antibody light chain and a conjugate of the protein having physiological activity bonded to the C-terminal end of the heavy chain of the antibody, and the nucleic acid molecule includes a nucleotide sequence encoding porcine teschovirus 2A peptide downstream of the gene encoding the light chain and the gene encoding the conjugate further downstream thereof, is a nucleic acid molecule wherein the conjugate is one having β-galactosidase (hGLB 1) bonded downstream from the Fab region of the heavy chain of anti-hTfR antibody via a linker, and the antibody light chain is the light chain of anti-hTfR antibody. A specific example is one wherein the Fab region of the heavy chain of anti-hTfR antibody includes the amino acid sequence set forth as SEQ ID NO: 12, the linker includes the amino acid sequence set forth as SEQ ID NO: 13, hGLB1 includes the amino acid sequence set forth as SEQ ID NO: 50, and the light chain of the anti-hTfR antibody includes the amino acid sequence set forth as SEQ ID NO: 15. The conjugate in this case includes the entirety of the amino acid sequence set forth as SEQ ID NO: 48.

The nucleic acid molecule according to one embodiment of the invention includes a gene encoding a fusion protein of an antibody and a protein having physiological activity, between a first long terminal repeat (LTR) and a second long terminal repeat (LTR). The following (1) to (4) are examples of such nucleic acid molecules:
(1) the fusion protein includes an antibody light chain and a conjugate of a protein having physiological activity bonded to the C-terminal end or N-terminal end of an antibody heavy chain, and the nucleic acid molecule includes a nucleotide sequence encoding an internal ribosome entry site downstream of the gene encoding the light chain, and a nucleotide sequence encoding the conjugate even further downstream thereof;
(2) the fusion protein includes an antibody light chain and a conjugate of a protein having physiological activity bonded to the C-terminal end or N-terminal end of an antibody heavy chain, and the nucleic acid molecule includes a nucleotide sequence encoding an internal ribosome entry site downstream of the gene encoding the conjugate, and a sequence encoding the light chain even further downstream thereof;
(3) the fusion protein includes an antibody heavy chain and a conjugate of a protein having physiological activity bonded to the C-terminal end or N-terminal end of an antibody light chain, and the nucleic acid molecule includes a nucleotide sequence encoding an internal ribosome entry site downstream of the gene encoding the heavy chain and a nucleotide sequence encoding the conjugate even further downstream thereof; and
(4) the fusion protein includes an antibody heavy chain and a conjugate of a protein having physiological activity bonded to the C-terminal end or N-terminal end of an antibody light chain, and the nucleic acid molecule includes a nucleotide sequence encoding an internal ribosome entry site downstream of the gene encoding the conjugate, and a nucleotide sequence encoding the heavy chain even further downstream thereof.

In (1) to (4) above, the nucleic acid molecule may be one that includes the nucleotide sequence of a regulatory element of gene expression between the first inverted terminal repeat (LTR) and the fusion protein-encoding gene. Also in (1) to (4) above, the nucleotide sequence encoding the internal ribosome entry site may be replaced by a nucleotide sequence that includes the regulatory element of gene expression. When the nucleic acid molecule has two regulatory elements of gene expression, they will be referred to as the first regulatory element of gene expression and first regulatory element of gene expression, in order from the first inverted terminal repeat (LTR) end. In (1) to (4) above, the nucleotide sequence encoding the internal ribosome entry site may be replaced by a nucleotide sequence encoding a 2A auto-cleaving peptide. The 2A peptide derived from porcine teschovirus is a suitable example of a 2A auto-cleaving peptide.

The nucleic acid molecule according to one embodiment of the invention, when the antibody is a single-stranded antibody, includes a gene encoding a fusion protein of a single-stranded antibody and a protein having physiological activity, between a first long terminal repeat (LTR) and a second long terminal repeat (LTR). The following (1) to (4) are examples of such nucleic acid molecules:
(1) the nucleic acid molecule includes a gene encoding a fusion protein having a light chain bonded to the C-terminal end of the heavy chain of an antibody via a second linker, and a protein having physiological activity further bonded to the C-terminal end of the light chain either directly or via a linker;
(2) the nucleic acid molecule includes a gene encoding a fusion protein having a heavy chain bonded to the C-terminal end of the light chain of an antibody via a second linker, and a protein having physiological activity further bonded to the C-terminal end of the light chain either directly or via a linker;
(3) the nucleic acid molecule includes a gene encoding a fusion protein having an antibody heavy chain bonded to the C-terminus end of a protein having physiological activity either directly or via a linker, and a light chain further bonded to the C-terminal end of the heavy chain;
(4) the nucleic acid molecule includes a gene encoding a fusion protein having an antibody light chain bonded to the C-terminus end of a protein having physiological activity either directly or via a linker, and a heavy chain further bonded to the C-terminal end of the heavy chain.

In (1) to (4) above, the nucleic acid molecule may be one that includes the nucleotide sequence of a regulatory element of gene expression between the first long terminal repeat (LTR) and the fusion protein-encoding gene.

For the nucleic acid molecule including a nucleotide sequence encoding an internal ribosome entry site, expression of one peptide chain forming the fusion protein is regulated from the regulatory element of gene expression, and expression of the other peptide chain is regulated from the nucleotide sequence encoding the internal ribosome entry site. The two peptide chains pair in cells to form a fusion protein of the antibody and the protein having physiological activity.

The nucleic acid molecule according to one embodiment may be used in an AAV vector system. When wild type AAV infects human host cells by itself, the viral genome is taken up in a site-specific manner into chromosome 19, via the inverted terminal repeats (ITR) present at both ends of the viral genome. Virtually none of the genes of the viral genome taken up into the host cells genome are expressed, but when the cells are also infected with a helper virus, AAV is cut out of the host genome and replication of the infectious virus begins. When the helper virus is an adenovirus, the helper-functioning genes are E1A, E1B, E2A, VA1 and E4. When the host cells are HEK293 cells, i.e. human fetal kidney tissue-derived cells, that have been transformed by E1A and E1B of the adenovirus, the E1A and E1B genes are originally expressed in the host cells.

The wild type AAV genome includes the two genes *rep* and *cap.* The *rep* proteins produced from the *rep* gene (*rep*78*, rep*68*, rep*52 and *rep*40) are essential for capsid formation, while also mediating incorporation of the viral genome into the chromosome. The *cap* gene produces three capsid proteins (VP1, VP2 and VP3).

In the wild type AAV genome, ITR is present at both ends, with the *rep* gene and *cap* gene situated between the ITRs. The genome is enveloped by the capsid, forming the AAV virion. Recombinant AAV virion (rAAV virion) has the region including the *rep* gene and *cap* gene replaced by a gene encoding an exogenous protein, and encapsulated in a capsid.

The rAAV virion is used in therapy as a vector for delivering exogenous genes to cells.

Production of recombinant AAV virion to be used for introduction of an exogenous gene into cells, tissue or the body generally involves the use of three different plasmids: (1) a plasmid having a structure including a nucleotide sequence that includes a first inverted terminal repeat (ITR) and a nucleotide sequence that includes a second inverted terminal repeat (ITR), both derived from a virus such as AAV, and a gene encoding a protein of interest, disposed between the two ITRs (plasmid 1), (2) a plasmid comprising the AAV Rep gene having function necessary for incorporating the nucleotide sequence of the region inserted between the ITR sequences (also including the ITR sequences) into the genome of the host cells, and a gene encoding the capsid protein of AAV (plasmid 2), and (3) a plasmid comprising the E2A region, E4 region and VA1 RNA region of an adenovirus (plasmid 3). This is not limitative, however, and any two of plasmids 1 to 3 may be linked into a single plasmid, with the remaining plasmid thus constituting two different plasmids. Alternatively, plasmids 1 to 3 may be linked into a single plasmid. The protein of interest for the purpose of the invention is a fusion protein of a ligand and a protein having physiological activity.

Production of a recombinant AAV virion is generally carried out by first introducing the three different plasmids by common transfection into host cells such as HEK293 cells having the adenovirus E1a gene and E1b gene incorporated into the genome. As a result, the region comprising the nucleotide sequence including the first inverted terminal repeat (ITR) and the nucleotide sequence including the second inverted terminal repeat (ITR), as well as the gene encoding the protein of interest situated between the two ITRs, is replicated in the host cells, and the single strand DNA product is packaged in the AAV capsid protein, forming a recombinant AAV virion. The recombinant AAV virion is infective and can thus be used to introduce the exogenous gene into cells, tissue or the body. The exogenous gene according to one embodiment of the invention is the gene encoding the fusion protein of a ligand and a protein having physiological activity. The fusion protein is expressed from the gene in the cells into which it has been introduced.

The Rep protein of the adeno-associated virus (AAV) is encoded by the AAV *rep* gene. The Rep protein has the function necessary for incorporating the AAV genome, for example, into the genome of the host cells via the ITRs present in the genome. Several subtypes of Rep protein exist, but the two subtypes Rep68 and Rep78 are essential for incorporation of the AAV genome into the genome of host cells. Rep68 and Rep78 are translation products of two different mRNAs transcribed by alternative splicing from the same gene. According to the invention, when referring to the Rep protein of AAV, itincludes at least the two types of proteins i.e., Rep68 and Rep78.

According to one embodiment, the nucleotide sequence encoding Rep protein of adeno-associated virus (the nucleotide sequence of the Rep region), refers to a nucleotide sequence encoding at least Rep68 and Rep78, or such a nucleotide sequence with mutations. The Rep protein is preferably AAV serotype 2, but this is not limitative, and it may be serotype 1, 3, 4, 5, 6, 7, 8, 9, 10 or 11 instead. A preferred example of a nucleotide sequence encoding Rep protein of wild type AAV serotype 2 is one having the nucleotide sequence set forth as SEQ ID NO: 24.

Rep68 may also be a variant with the amino acid sequence of wild type Rep68 of AAV serotype 1, 2, 3, 4, 5, 6, 7, 8, 9, 10 or 11 modified by a substitution, deletion or addition, so long as it exhibits the same function. Rep68 variants with such mutations are also included as "Rep68", according to the invention.

When amino acids of the amino acid sequence of wild type Rep68 are substituted with other amino acids, the number of substituted amino acids is preferably 1 to 10, more preferably 1 to 5 and even more preferably 1 to 3. When amino acids in the amino acid sequence of wild type Rep68 are deleted, the number of deleted amino acids is preferably 1 to 10, more preferably 1 to 5 and even more preferably 1 to 3. Rep68 with a mutation that is a combination of a substitution and a deletion of amino acids is also considered to be Rep68. When amino acids are added, preferably 1 to 10, more preferably 1 to 5 and even more preferably 1 to 3 amino acids are added within or at the N-terminus or C-terminus of the amino acid sequence of the wild type Rep68. Rep68 with a mutation that is a combination of an addition, substitution and deletion of amino acids is also considered to be Rep68. The amino acid sequence of mutated Rep68 has preferably identity of not lower than 80%, more preferably identity of not lower than 85%, even more preferably identity of not lower than 90%, yet more preferably identity of not lower than 95% and most preferably identity of not lower than 98%, to the amino acid sequence of the wild type Rep68.

Rep78 may also be a variant with the amino acid sequence of wild type Rep78 of AAV serotype 1, 2, 3, 4, 5, 6, 7, 8, 9, 10 or 11 modified by a substitution, deletion or addition, so long as it exhibits the same function. Rep78 variants with such mutations are also included as "Rep78", according to the invention.

When amino acids of the amino acid sequence of wild type Rep78 are substituted with other amino acids, the number of substituted amino acids is preferably 1 to 10, more preferably 1 to 5 and even more preferably 1 to 3. When amino acids in the amino acid sequence of wild type Rep78 are deleted, the number of deleted amino acids is preferably 1 to 10, more preferably 1 to 5 and even more preferably 1 to 3. Rep78 with a mutation that is a combination of a substitution and a deletion of amino acids is also considered to be Rep78. When amino acids are added, preferably 1 to 10, more preferably 1 to 5 and even more preferably 1 to 3 amino acids are added within or at the N-terminus or C-terminus of the amino acid sequence of the wild type Rep78. Rep78 with a mutation that is a combination of an addition, substitution and deletion of amino acids is also considered to be Rep78. The amino acid sequence of mutated Rep78 has preferably identity of not lower than 80%, more preferably identity of not lower than 85%, even more preferably identity of not lower than 90%, yet more preferably identity of not lower than 95% and most preferably identity of not lower than 98%, to the amino acid sequence of the wild type Rep78.

A "functional equivalent" of AAV Rep protein is, for Rep68, one that can be used as a functional substitute for Rep68, and for Rep78 it is one that can be used as a functional substitute for Rep78. A functional equivalent of Rep protein may be a variant of the wild type Rep protein.

The nucleotide sequence set forth as SEQ ID NO: 24 may be modified with a substitution, deletion or addition, so long as it still encodes functional Rep68 and Rep78. When one or more nucleotides of the nucleotide sequence set forth as SEQ ID NO: 24 are substituted with different nucleotides, the number of substituting nucleotides is preferably 1 to 20, more preferably 1 to 10 and even more preferably 1 to 3. When one or more nucleotides of the nucleotide sequence set forth as SEQ ID NO: 24 are deleted, the number of deleted nucleotides is preferably 1 to 20, more preferably 1 to 10 and even more preferably 1 to 3. Mutations that are combinations of substitutions and deletions of these nucleotides may also be used as nucleotide sequences encoding Rep protein. When one or more nucleotides are added, preferably 1 to 20, more preferably 1 to 10 and even more preferably 1 to 3 nucleotides are added within or at the 5'-end or 3'-end of the nucleotide sequence set forth as SEQ ID NO: 24. Mutations that are combinations of additions, substitutions and deletions of these nucleotides may also be used as nucleic acid molecules encoding Rep protein. A mutated nucleotide sequence has preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 24. When such mutations are added, however, the start codon of the gene encoding the Rep proteins, Rep68 and Rep78, is preferably ACG.

According to one embodiment, the nucleotide sequence encoding Cap protein of adeno-associated virus (the nucleotide sequence of the Cap region), refers to a nucleotide sequence encoding at least VP1, as a protein composing the capsid of AAV, or a nucleotide sequence including such a nucleotide sequence with mutations. VP1 is preferably AAV serotype 8, but this is not limitative, and it may be serotype 1, 2, 3, 4, 5, 6, 7, 8, 9, 10 or 11 instead. A preferred example of a nucleotide sequence for the Cap region of serotype 8 is the one including the nucleotide sequence set forth as SEQ ID NO: 22.

VP1 may also be the amino acid sequence of wild type VP1 of AAV serotype 1, 2, 3, 4, 5, 6, 7, 8, 9, 10 or 11 modified by a substitution, deletion or addition, so long as it exhibits the same function. According to one embodiment, VP1 with a mutation is also considered as "VP1".

When one or more nucleotides of the nucleotide sequence set forth as SEQ ID NO: 22 are substituted with different nucleotides, the number of substituting nucleotides is preferably 1 to 20, more preferably 1 to 10 and even more preferably 1 to 3. When one or more nucleotides of the nucleotide sequence set forth as SEQ ID NO: 22 are deleted, the number of deleted nucleotides is preferably 1 to 20, more preferably 1 to 10 and even more preferably 1 to 3. Mutations that are combinations of substitutions and deletions of these nucleotides may also be used as nucleotide sequences encoding Cap protein. When one or more nucleotides are added, preferably 1 to 20, more preferably 1 to 10 and even more preferably 1 to 3 nucleotides are added within or at the 5'-end or 3'-end of the nucleotide sequence set forth as SEQ ID NO: 22. Mutations that are combinations of additions, substitutions and deletions of these nucleotides may also be used as nucleic acid molecules encoding Cap protein. The mutated nucleotide sequence has preferably identity of not lower than 80%, more preferably identity of not lower than 85%, even more preferably identity of not lower than 90%, yet more preferably identity of not lower than 95% and most preferably identity of not lower than 98%, to the nucleotide sequence set forth as SEQ ID NO: 22.

By using an AAV vector, it is possible to obtain a recombinant AAV virion packaging a nucleic acid molecule that includes an exogenous gene between the first ITR and second ITR. The recombinant AAV virion is infective and can thus be used to introduce the exogenous gene into cells, tissue or the body. The exogenous gene according to the invention is the gene encoding the fusion protein of a ligand and a protein having physiological activity. The fusion protein is expressed from the gene in the cells into which the gene has been introduced. Recombinant virions packaging a nucleic acid molecule including an exogenous gene between the first ITR and second ITR, that can be used to introduce genes into cells, include not only AAV vector systems but also adenovirus vector systems. By using an adenovirus vector system, it is possible to obtain a recombinant adenovirus virion packaging a nucleic acid molecule that includes an exogenous gene between the first ITR and second ITR. The recombinant adenovirus virion is infective and can thus be used to introduce the exogenous gene into cells, tissue or the body.

Lentivirus is a virus belonging to the family Retroviridae, subfamily Orthoretrovirinae, genus Lentivirus, and it has a single-stranded (+)-strand RNA genome (ssRNA). The lentivirus genome includes as essential genes, *gag* (region encoding structural proteins including the capsid protein), *pol* (region encoding an enzyme group including reverse transcriptase) and *env* (region encoding the envelope proteins necessary for binding to host cells), which lie between two LTRs (5' LTR and 3' LTR). In addition to these, the lentivirus genome includes as auxiliary genes, *rev* (region encoding a protein that binds with RRE (*rev* responsive element) in viral RNA and functions to transport viral RNA from the nucleus to the cytoplasm), and *tat* (region encoding a protein that binds with TAR in the 5' LTR and functions to increase LTR promoter activity), as well as *vif, vpr, vpu* and *nef.*

Lentivirus is family of enveloped viruses that infect cells by way of fusion of the envelope with cell membranes. Lentiviruses are RNA viruses, containing reverse transcriptase in the virion. After infection with a lentivirus, the single-stranded plus strand DNA is replicated from the (+)-strand RNA genome by reverse transcriptase, and double-stranded DNA is further synthesized. A protein that is a constituent of the virion, is expressed from the double-stranded DNA and package the (+)-strand RNA genome, leading to virion proliferation. According to one embodiment of the invention, the lentivirus vector may be, but is not limited to, any of those developed based on the genome of HIV-1, a type of lentivirus.

First generation lentivirus vectors comprise three types of plasmids: a packaging plasmid, a Env plasmid and a transfection plasmid. A packaging plasmid has the genes *gag* and *pol* under the control of CMV promoter. An Env plasmid has the *env* gene under the control of CMV promoter. A transfection plasmid has 5' LTR, RRE, a gene encoding a protein of interest under the control of CMV promoter, and 3' LTR. By introducing these into host cells by a common transfection method, a recombinant virion is obtained having a nucleic acid molecule including an exogenous gene between the first ITR and second ITR, packaged in a capsid protein. The packaging plasmid for the first generation lentivirus vector includes the viral auxiliary genes *rev, tat, vif, vpr, vpu* and *nef.* The protein of interest for the purpose of the invention is a fusion protein of a ligand and a protein having physiological activity. Examples of suitable promoters other than CMV promoter, as promoters for regulation of the gene encoding the protein of interest, include SV40 early promoter, human elongation factor-1α (EF-1α) promoter, human ubiquitin C promoter, retroviral Rous sarcoma virus-LTR promoter, dihydrofolate reductase promoter, β-actin promoter, phosphoglycerate kinase (PGK) promoter, mouse albumin promoter, human albumin promoter and human α-1 antitrypsin promoter. For example, a synthetic promoter having the nucleotide sequence set forth as SEQ ID NO: 8 including mouse albumin promoter downstream from mouse α-fetoprotein enhancer (mouse α-fetoprotein enhancer/mouse albumin promoter) may be suitably used.

Second generation lentivirus vectors, similar to the first generation, also comprise three types of plasmids: a packaging plasmid, a Env plasmid (envelope plasmids) and a transfection plasmid. However, the unnecessary auxiliary genes *vif, vpr, vpu* and *nef* are deleted from the packaging plasmid.

Third generation lentivirus vectors comprise four types of plasmids: a packaging plasmid, a Env plasmid (envelope plasmids) a Rev plasmid and a transfection plasmid. In the third generation, the second generation packaging plasmid *rev* is independently classified as Rev plasmid. In this case, *tat* is deleted from the packaging plasmid. In addition, TAR in the 5' LTR of the transfection plasmid is replaced by CMV promoter.

The recombinant virion is infective and can thus be used to introduce the exogenous gene into cells, tissue or the body. The exogenous gene according to the invention is the gene encoding the fusion protein of a ligand and a protein having physiological activity. The fusion protein is expressed from the gene in the cells into which the gene has been introduced.

Retroviruses have a single-stranded (+)-strand RNA genome (ssRNA). The viral genome encodes *gag* (encoding structural proteins including the capsid protein), *pol* (encoding an enzymes including reverse transcriptase) *env* (encoding the envelope proteins necessary for binding to host cells), and a packaging signal (Ψ), which are situated between two long terminal repeats (LTRs: 5' LTR and 3' LTR). When a retrovirus infects host cells, the (+)-strand RNA and reverse transcriptase migrate into the cells and are reverse transcribed into double-stranded DNA.

Retrovirus vectors are developed primarily based on murine leukemia virus, and the viral genome is split to cause loss of auto-replicating ability while maintaining infectivity, in order to eliminate pathogenicity and increase safety. First-generation retrovirus vectors consist of a packaging plasmid (the viral genome minus the packaging signal) and a transfection plasmid (the packaging signal, a portion of *gag,* and the exogenous gene sandwiched at both ends by 5' LTR and 3' LTR). Homologous recombination at the *gag* sequence that is present in both the packaging plasmid and the transfection plasmid produces an auto-replicative retrovirus, or RC (replication competent) virus. In a second-generation retrovirus vector, the 3'-end LTR of the first-generation packaging plasmid is replaced with a poly-A addition signal. Development of an RC virus therefore requires simultaneous homologous recombination at 2 sites, the *gag* sequence and upstream from the poly-A addition signal, and since this has very low probability of developing, safety is increased as a result. The third generation consists of three plasmids, with the second-generation packaging plasmid further divided into a plasmid encoding *gag*/*pol* and a plasmid encoding *env.* Development of an RC virus therefore requires the occurrence of simultaneous homologous recombination at 3 sites, and since this has extremely low probability of developing, safety is further increased as a result.

Generally, for production of a recombinant retrovirus virion, first the packaging plasmid and transfection plasmid are introduced into host cells by a common transfection method. This results in replication in the host cells of the 5' LTR and 3' LTR and the region containing the gene encoding for the protein of interest disposed between the two LTRs, with the produced single-stranded (+)-strand RNA being packaged in the retrovirus capsid protein, thus forming a recombinant retrovirus virion. The recombinant retrovirus virion is infective and can thus be used to introduce the exogenous gene into cells, tissue or the body. The exogenous gene according to the invention is the gene encoding the fusion protein of a ligand and a protein having physiological activity. The fusion protein is expressed from the gene in the cells into which the gene has been introduced.

According to one embodiment, the nucleic acid molecule may be in a form encapsulated in liposomes or lipid nanoparticles (LNP). Liposomes are spherical vesicles with a lipid bilayer, being composed mainly of phospholipids, and particularly phosphatidylcholine. This is not limitative, however, and the liposomes may have those to which other lipids such as egg yolk phosphatidylethanolamine have beenadded as well, so long as a lipid bilayer is formed. Since cell membranes are composed mainly of phospholipid bilayer membranes, liposomes have the advantage of superior biocompatibility. Lipid nanoparticles are particles composed mainly of lipids and having diameters of 10 nm to 1000 nm and typically less than about 200 nm, with the ability to encapsulate hydrophobic (lipophilic) molecules, and their major constituent components are biocompatible lipids such as triglycerides, diglycerides, monoglycerides, fatty acids and steroids. When liposomes or lipid nanoparticles encapsulating a gene are administered to the body, they directly fuse with cell membranes or are taken up into cells by endocytosis, subsequently migrating to the nucleus and delivering the gene to the cells. A gene transfer method using liposomes or lipid nanoparticles is superior to gene transfer using viral vectors in that there is no limit to the size of the gene to be transferred, and the degree of safety is higher. Liposomes and lipid nanoparticles or the like encapsulating the nucleic acid molecule of the invention can be used to deliver a gene for a fusion protein of a ligand and a protein having physiological activity, into cells, tissue or the body. The fusion protein is expressed from the gene in the cells into which the gene has been introduced. As used herein, "liposomes and lipid nanoparticles or the like" refers not only to liposomes and lipid nanoparticles but also to any nano or microparticles suitable for use, such as polymer nanoparticles, micelles, emulsions, nanoemulsions, microspheres, nanospheres, microcapsules, nanocapsules, dendrimers, nanogels, metal nanoparticles and other drug delivery systems (DDS).

According to the invention, the following (1) to (9) are examples of the behavior of a nucleic acid molecule introduced into cells, tissue or the body in the form of a plasmid, in a form encapsulated in a recombinant virus virion, or a form encapsulated in liposomes or lipid nanoparticles. The behavior of a nucleic acid molecule is not limited to these, however.
(1) According to one embodiment, the nucleic acid molecule is single-stranded (+)-strand RNA, and once introduced into cells, the gene encoding the fusion protein of a ligand and a protein having physiological activity, which is contained in the nucleic acid molecule, is translated and the fusion protein is expressed.
(2) According to one embodiment, the nucleic acid molecule is single-stranded (+)-strand RNA, and once introduced into cells, the nucleic acid molecule is reverse transcribed to single-stranded (+)-strand DNA, after which the DNA is transcribed and translated to express the fusion protein.
(3) According to one embodiment, the nucleic acid molecule is single-stranded (+)-strand RNA or (-)-strand RNA, and once introduced into cells, the nucleic acid molecule is reverse transcribed and then converted to double-stranded DNA, after which the DNA is transcribed and translated to express the fusion protein.
(4) According to one embodiment, the nucleic acid molecule is single-stranded (+)-strand or (-)-strand RNA, and once introduced into cells, the nucleic acid molecule is reverse transcribed and then converted to double-stranded DNA, after which the DNA is randomly recombined or homologously recombined with the host cell genome and incorporated into the genome, and the incorporated DNA is transcribed and translated to express the fusion protein.
(5) According to one embodiment, the nucleic acid molecule is single-stranded (+)-strand DNA, and once introduced into cells, the nucleic acid molecule is transcribed and translated to express the fusion protein.
(6) According to one embodiment, the nucleic acid molecule is single-stranded (-)-strand DNA, and once introduced into cells, double-stranded DNA is synthesized from the nucleic acid molecule, and then the double-stranded DNA is transcribed and translated to express the fusion protein.
(7) According to one embodiment, the nucleic acid molecule is single-stranded (+)-strand DNA or (-)-strand DNA, and once introduced into cells, double-stranded DNA is synthesized from the nucleic acid molecule, after which the DNA is randomly recombined or homologously recombined with the host cell genome and incorporated into the genome, and the incorporated DNA is transcribed and translated to express the fusion protein.
(8) According to one embodiment, the nucleic acid molecule is double-stranded DNA, and once introduced into cells, the nucleic acid molecule is transcribed and translated to express the fusion protein.
(9) According to one embodiment, the nucleic acid molecule is double-stranded DNA, the DNA is randomly recombined or homologously recombined with the host cell genome and incorporated into the genome, and the incorporated DNA is transcribed and translated to express the fusion protein.

The nucleic acid molecule may be introduced into cells, tissue or the body in the form of a plasmid, in the form encapsulated in a recombinant virus virion, or in the form encapsulated in liposomes or lipid nanoparticles.

When the destination of the nucleic acid molecule is the body, the nucleic acid molecule is administered by parenteral means such as subcutaneous injection, intramuscular injection or intravenous injection, in the form of a plasmid, in a form encapsulated in a recombinant virus virion, or a form encapsulated in liposomes or lipid nanoparticles.

The nucleic acid molecule in the form of a plasmid, in the form encapsulated in a recombinant virus virion or in the form encapsulated in liposomes or lipid nanoparticles may be used as any of a wide variety of medicines. The following explanation pertains to usage of the nucleic acid molecule in a case where the fusion protein encoded by the nucleic acid molecule specifically recognizes a molecule (surface antigen) on the surfaces of cerebrovascular endothelial cells, and the protein having physiological activity is a human lysosomal enzyme.

When the human lysosomal enzyme is α-L-iduronidase it may be used as a central nervous system disorder therapeutic agent for Hurler syndrome or Hurler-Scheie syndrome, when it is iduronate-2-sulfatase it may be used as a central nervous system disorder therapeutic agent for Hunter's syndrome, when it is glucocerebrosidase it may be used as a central nervous system disorder therapeutic agent for Gaucher disease, when it is β-galactosidase it may be used as a central nervous system disorder therapeutic agent for GM1-gangliosidosis type 1 to 3, when it is a GM2 activated protein it may be used as a central nervous system disorder therapeutic agent for GM2-gangliosidosis AB variant, when it is β-hexosaminidase A it may be used as a central nervous system disorder therapeutic agent for Sandhoffs disease and Tay-Sachs disease, when it is β-hexosaminidase B it may be used as a central nervous system disorder therapeutic agent for Sandhoffs disease, when it is N-acetylglucosamine-1-phosphotransferase it may be used as a central nervous system disorder therapeutic agent for I-cell disease, when it is α-mannosidase it may be used as a central nervous system disorder therapeutic agent for alpha-mannosidosis, when it is β-mannosidase it may be used as a central nervous system disorder therapeutic agent for beta-mannosidosis, when it is galactosylceramidase it may be used as a central nervous system disorder therapeutic agent for Krabbe disease, when it is saposin C it may be used as a central nervous system disorder therapeutic agent for Gaucher-like storage disease, when it is arylsulfatase A it may be used as a central nervous system disorder therapeutic agent for metachromatic white matter degeneration (metachromatic leukodystrophy), when it is α-L-fucosidase it may be used as a central nervous system disorder therapeutic agent for fucosidosis, when it is aspartylglucosaminidase it may be used as a central nervous system disorder therapeutic agent for aspartylglucosaminuria, when it is α-N-acetylgalactosaminidase it may be used as a central nervous system disorder therapeutic agent for Schindler's disease and Kawasaki disease, when it is acid sphingomyelinase it may be used as a central nervous system disorder therapeutic agent for Niemann-Pick disease, when it is α-galactosidase A it may be used as a central nervous system disorder therapeutic agent for Fabry disease, when it is β-glucuronidase it may be used as a central nervous system disorder therapeutic agent for Sly syndrome, when it is heparan N-sulfatase, α-N-acetylglucosaminidase, acetyl CoA α-glucosaminide N-acetyltransferase or N-acetylglucosamine-6-sulfatase it may be used as a central nervous system disorder therapeutic agent for Sanfilippo syndrome, when it is acid ceramidase it may be used as a central nervous system disorder therapeutic agent for Farber's disease, when it is amylo-1,6-glucosidase it may be used as a central nervous system disorder therapeutic agent for Cori disease (Forbes-Cori disease), when it is sialidase it may be used as a central nervous system disorder therapeutic agent for sialidase deficiency, when it is aspartylglucosaminidase it may be used as a central nervous system disorder therapeutic agent for aspartylglucosaminuria, when it is palmitoyl protein thioesterase-1 (PPT-1) it may be used as a central nervous system disorder therapeutic agent for neuronal ceroid lipofuscinosis or Santavuori-Haltia disease, when it is tripeptidyl peptidase-1 (TPP-1) it may be used as a central nervous system disorder therapeutic agent for neuronal ceroid lipofuscinosis or Jansky-Bielschowsky disease, when it is hyaluronidase-1 it may be used as a central nervous system disorder therapeutic agent for hyaluronidase deficiency, and when it is CLN1 or CLN2 it may be used as a central nervous system disorder therapeutic agent for Batten's disease.

When the destination of the nucleic acid molecule is cells, the cell type is not particularly restricted, but mesenchymal stem cells, dental pulp stem cells, hematopoietic stem cells, embryonic stem cells, endothelial stem cells, mammary stem cells, intestinal stem cells, liver stem cells, pancreatic stem cells, neural stem cells and iPS cells are included.

The cells into which the nucleic acid molecule has been transferred express the fusion protein and can therefore be grafted into a patient to be treated. For example, cells where the transferred nucleic acid molecule has human lysosomal enzyme as the protein with physiological activity can be used for the purposes described above.

### Examples

### [Example 1] Construction ofpAAV-mMAP-Fab-hI2S vector

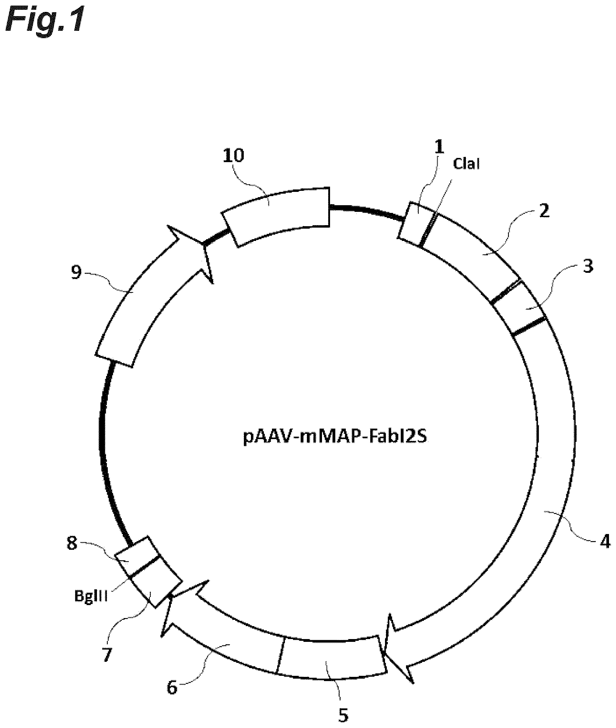

A DNA fragment was synthesized containing the nucleotide sequence set forth as SEQ ID NO: 18, which includes the ClaI site, mouse α-fetoprotein enhancer/mouse albumin promoter, chicken β actin/MVM chimeric intron, a gene encoding a conjugate of hI2S bonded to the C-terminal end of the Fab region of the anti-hTfR antibody heavy chain, an internal ribosome entry site (IRES) derived from the 5' untranslated region of murine encephalomyocarditis virus, a gene encoding the anti-hTfR antibody light chain, the poly-A sequence of bovine growth hormone, and the BglII site, in order from the 5'-end. The DNA fragment was digested with ClaI and BglII. A pAAV-CMV vector (Takara Bio, Inc.) was digested with ClaI and BglII and the aforementioned restriction enzyme-treated DNA fragment was inserted into it. The obtained plasmid was designated as pAAV-mMAP-Fab-hI2S vector (Fig. 1). The pAAV-mMAP-Fab-hI2S vector has a structure including a first AAV-ITR functional equivalent (SEQ ID NO: 6), mouse α-fetoprotein enhancer/mouse albumin promoter (SEQ ID NO: 8), chicken β actin/MVM chimeric intron (SEQ ID NO: 9), a gene encoding a conjugate with hI2S bonded to the C-terminal end of the Fab region of the anti-hTfR antibody heavy chain (SEQ ID NO: 42), an internal ribosome entry site derived from the 5' untranslated region of murine encephalomyocarditis virus, a gene encoding the light chain of anti-hTfR antibody (SEQ ID NO: 10), the poly-A signal of bovine growth hormone (SEQ ID NO: 17) and a second AAV-ITR functional equivalent (SEQ ID NO: 7), in order from the upstream end. The pAAV-mMAP-Fab-hI2S vector also includes an ampicillin resistance gene and a replication origin (ColE1 ori).

### [Example 2] Construction of pR2(mod)C6 vector

A DNA fragment was synthesized containing the nucleotide sequence set forth as SEQ ID NO: 19, which includes the AfeI site, RsrII site, BsrGI site and AvrII site, an ampicillin resistance gene site, a replication origin (ColE1 ori), the RsrII site, the 5' portion of the Rep region of AAV2 including the p5 promoter, and the SacII site, in order from the 5'-end. The DNA fragment was digested with AfeI and SacII. A pRC6 vector (Takara Bio, Inc.) was digested with AfeI and SacII and the aforementioned restriction enzyme-treated synthetic gene was inserted into it. The obtained plasmid was designated as pR2(mod)C6 vector.

### [Example 3] Construction of pR2(mod)C8 vector

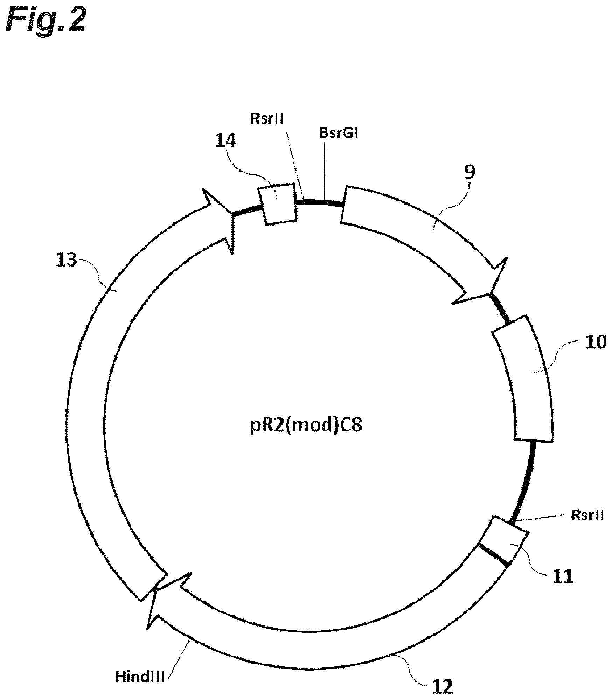

A DNA fragment was synthesized containing the nucleotide sequence set forth as SEQ ID NO: 20, which includes the HindIII site, the 3' portion of the Rep region of AAV2, the Cap region of AAV8, p5 promoter functioning as an enhancer, the RsrII site and the BsrGI site, in order from the 5'-end. The DNA fragment was digested with HindIII and BsrGI. A pR2(mod)C6 vector was digested with HindIII and BsrGI and the aforementioned restriction enzyme-treated synthetic gene was inserted into it. The obtained plasmid was designated as pR2(mod)C8 vector (Fig. 2). The pR2(mod)C8 vector includes the Rep region of AAV2 including the p5 promoter (SEQ ID NO: 21), the Cap region of AAV8 (SEQ ID NO: 22), and the p5 promoter functioning as an enhancer (SEQ ID NO: 23), in order from the upstream end. The pR2(mod)C8 vector also includes an ampicillin resistance gene and a replication origin (ColE1 ori). The Rep region of AAV2 including the p5 promoter (SEQ ID NO: 21) includes a nucleotide sequence including the Rep region of AAV2 set forth as SEQ ID NO: 24 downstream of the p5 promoter (SEQ ID NO: 23).

### [Example 4] Production of Fab-hI2S-rAAV

HEK293 cells, as a cell line derived from human embryonic kidney cells, were used as the host cells. The HEK293 cells were seeded in a 10-layer cell stack chamber (Thermo Fisher Scientific) at 6.3 × 10⁸ cells per cell stack chamber, and cultured in 10% FBS-containing MEM medium (Thermo Fisher Scientific) for 16 hours at 37°C in the presence of 5% CO₂. The DNA solution for transfection was a solution prepared containing pHelper vector (Takara Bio, Inc.), pR2(mod)C8 vector and pAAV-mMAP-Fab-hI2S vector. The solution contained the three types of plasmids at equimolar concentrations, with a total DNA concentration of 1.0 mg/mL and was adjusted to a total liquid volume of 2.2 mL. After adding polyethyleneimine to the solution to a weight ratio of DNA (µg):polyethyleneimine (µg) = 1:2, MEM medium was added to adjust the liquid volume to 1.5 L, to prepare a transfection solution. At 16 hours after the start of culturing, all of the culture supernatant was removed from the 10-layer cell stack chamber, and then the total 1.5 L of transfection solution was added, culturing for 5 days at 37°C in the presence of 5% CO₂, to produce rAAV virion. Some of the rAAV virion produced by the host cells was released into the culture supernatant, and some remained in the host cells. The obtained rAAV virion was designated as Fab-hI2S-rAAV When used to infect cells, Fab-hI2S-rAAV is expected to express a fusion protein that includes a conjugate having hI2S bonded to the C-terminal end of the heavy chain of the anti-hTfR antibody Fab region, and the anti-hTfR antibody light chain.

### [Example 5] Recovery of culture supernatant containing Fab-hI2S-rAAV

Upon completion of the culturing in Example 4, the cell culture solution was filtered with 5.0 µm of ULTRA Prime GF, 2 inch capsule, GG, with a pore size of 5 µm (Cytiva), and then subsequently filtered with a Nalgen Rapid-Flow PES filter unit (Thermo Fisher Scientific) having a pore size of 0.2 µm.

### [Example 6] Removal of free nucleic acids in Fab-hI2S-rAAV-containing culture supernatant

After adding the endonuclease Benzonase (Merck) to the culture supernatant recovered in Example 5 to a concentration of 10 U/mL, the mixture was incubated at 37°C for 1 hour. The Benzonase-treated culture supernatant was filtered using a Nalgen Rapid-Flow PES filter unit (Thermo Fisher Scientific) having a pore size of 0.2 µm, to obtain a thawed Fab-hI2S-rAAV-containing cell solution.

### [Example 7] Chromatography purification of Fab-hI2S-rAAV using affinity resin

A column (1.6 cm diameter, 20 cm height (column volume: ~20 mL)) packed with POROS AAVX resin (Thermo Fisher Scientific) was equilibrated with 20 mmol/L Tris buffer containing 4 mmol/L MgCl₂ and 150 mmol/L (pH 7.5). The thawed Fab-hI2S-rAAV-containing cell solution obtained in Example 6 was passed through the column at a flow rate of 5 mL/min for adsorption of the Fab-hI2S-rAAV onto the resin. Next, 20 mmol/L Tris buffer containing 4 mmol/L MgCl₂ and 400 mmol/L arginine (pH 7.5) was passed through the column in an amount of at least 5 times the column volume for washing, and then 20 mmol/L Tris buffer containing 4 mmol/L MgCl₂ and 150 mmol/L NaCl (pH 7.5) was passed through in an amount of at least 3 times the column volume, for further washing of the column. Subsequently, 10 mM citrate buffer containing 4 mM MgCl₂ (pH 3.5) was passed through the column in an amount of at least twice the column volume to elute the Fab-hI2S-rAAV adsorbed onto the resin, and the fraction containing Fab-hI2S-rAAV was obtained. Finally, 500 mmol/L Tris buffer containing 2 mM MgCl₂ (pH 8.5) was added to the fraction for adjustment to pH 7.0.

### [Example 8] Concentration of Fab-hI2S-rAAV by ultrafiltration membrane

The affinity column-eluted fraction obtained in Example 7 was circulated through a SUIS PD (LP) Hystream 100 kDa (Repligen) cassette equilibrated with 20 mmol/L Tris buffer containing 4 mmol/L MgCl₂ and 150 mmol/L NaCl (pH 7.5), at a flux of 7 L/min/m² or lower for concentrating it to approximately 8 mL, and recovered. The ultrafiltration membrane was washed twice using 2 mL of 20 mmol/L Tris buffer containing 4 mmol/L MgCl₂ and 150 mmol/L NaCl, (pH 7.5), and the washing solution was combined with the previously recovered solution to obtain a total of about 12 mL of concentrate.

### [Example 9] Purification of Fab-hI2S-rAAV by ultracentrifugation using iodixanol

PBS-MK buffer (20 mmol/L sodium phosphate buffer containing 136.9 mmol/L NaCl, 28.2 mmol/L KCl and 1 mmol/L MgCl₂ (pH 7.4) was added to 60% iodixanol (versatile density gradient centrifugation medium, OptiPre^{™}, Cosmo Bio Co., Ltd.), to prepare 45% iodixanol. PBS-MK buffer was also added to 60% iodixanol to prepare 25% iodixanol. In addition, 1 M NaCl/PBS-MK buffer (PBS-MK buffer containing 1 mol/L sodium chloride) was added to 60% iodixanol to prepare 15% iodixanol.

60% iodixanol with added phenol red, and then 45% iodixanol, and then 25% iodixanol with added phenol red, and further then 15% iodixanol, at 5 mL, 8 mL, 5 mL and 8 mL respectively, were layered in that order from the bottom of a 39 mL Quick-Seal^{™} Round-Top Polypropylene Tube, 25 X, 89 mm (Beckman Coulter, Inc.). After adding approximately 12 mL of the concentrate obtained in Example 8, the tube was filled with 1 M NaCl/PBS-MK buffer. The injection port of the tube was then closed with a cordless tube topper sealer kit (Beckman Coulter, Inc.), and the tube was attached to the 70Ti rotor of an OptimaXPN ultracentrifuge (Beckman Coulter, Inc.) and centrifuged for 2 hours at a rotational speed of 63,000 rpm (408,500 × g). A Fraction Recovery System (Beckman Coulter, Inc.) was used to connect a needle-equipped Tygon E-LFL tube at the bottom of the centrifuged tube, and after puncturing the top of the tube with a 18 G syringe needle to open an air hole, the solvent was fractionated in 1 mL portions from the bottom of the tube using a peristaltic pump. The 4th to 9th or 5th to 8th fractions were recovered to obtain fractions containing the purified Fab-hI2S-rAAV.

### [Example 10] Ultrafiltration membrane concentration of Fab-hI2S-rAAV and buffer exchange

The fractions containing Fab-hI2S-rAAV obtained in Example 9 were concentrated by centrifugal separation under arbitrary conditions of 3200 rpm (2100 × g) or lower for 20 minutes or less, using an Amicon Ultra 15 centrifugal filter unit, 50 kDa (Merck). The resulting concentrated Fab-hI2S-rAAV solution was diluted with a 30-fold amount of 350 mmol/L NaCl/PBS buffer (20 mmol/L sodium phosphate buffer containing 486.9 mmol/L NaCl and 2.7 mmol/L KCl (pH 7.4)). This was repeated 3 times to obtain a Fab-hI2S-rAAV solution having the solvent replaced by 350 mmol/L NaCl/PBS buffer. The Fab-hI2S-rAAV solution was supplied for the following experiment.

### [Example 11] Measurement of rAAV genome content in Fab-hI2S-rAAV solution

The amount of viral genome in the Fab-hI2S-rAAV solution obtained in Example 10 was measured by Droplet Digital PCR. The measuring method is described in detail below.

After adding 1.6 µL of DNaseI (Takara Bio, Inc.), 2 µL of 10 × DNaseI Buffer (Takara Bio, Inc.) and 14.4 µL of 0.05% F-68-containing water to 2 µL of the Fab-hI2S-rAAV solution, the mixture was incubated at 37°C for 30 minutes, digesting the DNA not encapsulated in the Fab-hI2S-rAAV.

The DNaseI-treated Fab-hI2S-rAAV solution was appropriately diluted with 0.05% F-68-containing TE buffer to prepare a sample solution for Droplet Digital PCR. A FAM-labeled 20 × primer/probe mix was prepared containing 1.0 µM of forward primer (primer SI-1, SEQ ID NO: 25), 1.0 µM of reverse primer (primer SI-2, SEQ ID NO: 26) and 0.25 µM of probe (probe SI-1, prepared by modifying with FAM as the reporter dye at the 5'-end and BHQ1 as the quencher dye at the 3'-end of SEQ ID NO: 27). A HEX-labeled 20 × primer/probe mix was prepared containing 1.0 µM of forward primer (primer SI-3, SEQ ID NO: 28), 1.0 µM of reverse primer (primer SI-4, SEQ ID NO: 29) and 0.25 µM of probe (probe SI-2, prepared by modifying with HEX as the reporter dye at the 5'-end and BHQ1 as the quencher dye at the 3'-end of SEQ ID NO: 30).

To 2 µL of the sample solution for Droplet Digital PCR was added 10 µL of ddPCR Supermix for probes (no dUTP) (BioRad), 1 µL of FAM-labeled 20 × primer/probe mix, 1 µL of HEX-labeled 20 × primer/probe mix, 2 µL of 5 M betaine solution (Sigma Corp.) and 4 µL of 0.05% F-68-containing water, to prepare a PCR reaction solution. A Droplet Generator (BioRad) was used to prepare a suspension droplet containing 20 µL of the PCR reaction solution and 70 µL of Droplet Generator Oil for Probes (BioRad). The droplet was supplied to a QX200 Droplet Digital PCR system (BioRad). The PCR conditions were: denaturation reaction (95°C, 10 minutes), 40 cycles of 3-step PCR (95°C, 30 sec → 60°C, 60 sec → 72°C, 15 sec), and PCR enzyme inactivation treatment (98°C, 10 minutes). The FAM/HEX double-positive droplet was defined as the rAAV-positive droplet, and the rAAV genome content (vg: viral genome) was determined using QuantaSoft Version 1.7 (BioRad). The DNA regions amplified in the PCR procedure are the bovine growth hormone poly-A and ITR interior regions.

### [Example 12] Creation of I2S-KO/hTfR-KI mice

I2S-KO/hTfR-KI mice are mice that are homodeficient for the iduronate-2-sulfatase (I2S) gene and heterozygous for the chimeric TfR gene. The I2S-KO/hTfR-KI mice were created basically by the following method. A DNA fragment was chemically synthesized to have a neomycin resistance gene inserted in the loxP sequence at the 3'-end of cDNA encoding chimeric TfR wherein the intracellular domain is the amino acid sequence of mouse TfR and the extracellular domain is the amino acid sequence of human TfR. The DNA fragment was incorporated by a common method into a targeting vector with a 5' arm sequence and a 3' arm sequence, and transferred into mouse ES cells by electroporation. The gene transferred mouse ES cells were selectively cultured in the presence of neomycin, and mouse ES cells were selected which had the targeting vector incorporated into the chromosomes by homologous recombination. The obtained gene recombinant mouse ES cells were implanted into ICR mice at the 8-cell stage (host embryo), and grafted into pseudopregnant mice (recipient mice) obtained by cross-breeding with deferent duct-ligated mice. The coat colors of the obtained offspring (chimeric mice) were judged, and individuals were selected having highly efficient contribution of the ES cells to body formation, i.e. with high percentage of white hair of the total hair. The chimeric mice individuals were cross-bred with ICR mice to obtain F1 mice. The white F1 mice were selected, DNA extracted from the tail tissue was analyzed, and mice having the mouse hTfR gene replaced with chimeric TfR on the chromosomes were designated as hTfR-KI mice. From these mice there were created mice homodeficient for the I2S gene and heterozygous for the chimeric TfR gene (I2S-KO/hTfR-KI mice). Creation of the I2S-KO/hTfR-KI mice was carried out by the same method as described in patent literature (WO2016/208695).

### [Example 13] Evaluation of drug effect of Fab-hI2S-rAAV using I2S-KO/hTfR-KI mice

The drug effect with administration of Fab-hI2S-rAAV to the body was evaluated using the I2S-KO/hTfR-KI mice created in Example 12. The I2S-KO/hTfR-KI mice used were male mice 9 to 14 weeks of age (at the start of administration). The Fab-hI2S-rAAV solution obtained in Example 10 was administered to male I2S-KO/hTfR-KI mice once through the caudal vein to dosages of 1.0 × 10¹⁰ vg/kg, 1.0 × 10¹¹ vg/kg, 1.0 × 10¹² vg/kg, and 1.0 × 10¹³ vg/kg (N=3 for each group). A normal control group composed of male wild type mice and a disease control group composed of male I2S-KO/hTfR-KI mice were also prepared (N=4 for each group). The normal control group and the disease control group were both administered physiological saline (Otsuka Pharmaceutical Factory, Inc.).

At 8, 15 and 22 days after administration, a portion of blood was harvested from the caudal vein into an EDTA-2K-coated blood sampling tube (Capiject, Terumo Corp.), and after allowing it to cool on ice, it was centrifuged (2000 × g, 20 minutes) and the blood plasma was collected. At 29 days after administration, cerebrospinal fluid (CSF) was harvested from the cisterna magna under anesthesia with a 3-drug mixture of Betorfal (Meiji Seika Pharma), Midazolam (Sandoz Ltd.) and Dormital (Zenoaq). The mice were then opened by thoracotomy and heart blood was sampled. The blood was harvested into an EDTA-2K-coated blood sampling tube (Capiject, Terumo Corp.), and after allowing it to cool on ice, it was centrifuged (2000 × g, 20 minutes) and the blood plasma was collected. After subsequent general perfusion with physiological saline (Otsuka Pharmaceutical Factory, Inc.), the brain, spinal cord, liver, heart, kidneys, spleen and skeletal muscle were each extracted. After measuring the wet weight of each tissue, it was immediately immersed in liquid nitrogen for rapid freezing.

### [Example 14] Quantitation of Fab-hI2S in blood plasma

Measurement of the Fab-hI2S concentration in the blood plasma supernatant was carried out by the method described in Example 15. The results are shown in Table 1.

### [Table 1]

**(Table 1) Time-related change in Fab-hI2S concentration in plasma (µg/mL plasma)**

| Administered group | | Day 8 after administration | | Day 15 after administration | | Day 22 after administration | | Day 29 after administration | |
|---|---|---|---|---|---|---|---|---|---|
| | | Mean | S.D. | Mean | S.D. | Mean | S.D. | Mean | S.D. |
| Fab-hI2S-rAAV | 1.0 10¹³ vg/kg | 0.471 | 0.148 | 1.63 | 0.80 | 1.82 | 1.10 | 3.72 | 2.71 |
| | 1.0 x 10¹² vg/kg | 0.222 | 0.081 | 0.330 | 0.097 | 0.420 | 0.146 | 0.594 | 0.209 |
| | 1.0 x 10¹¹ vg/kg | 0.0110 | 0.0118 | 0.0189 | 0.0185 | 0.0223 | 0.0242 | 0.0251 | 0.0278 |
| | 1.0 x 10¹⁰ vg/kg | 0.000250 | 0.000433 | 0.000729 | 0.000709 | 0.000897 | 0.000834 | 0.00115 | 0.00101 |

In the Fab-hI2S-rAAV-administered group, the Fab-hI2S concentration in the blood plasma increased from day 8 to day 29 after administration, at all administered doses. The Fab-hI2S concentration in the blood plasma increased in a dose-dependent manner. These data indicate that the Fab-hI2S-rAAV intravenously injected into the mice had been incorporated into the cells, that hI2S-encoding gene had been transcribed in the cells, and that translated and expressed hI2S had been released into the blood.

### [Example 15] Measurement of Fab-hI2S concentration

The Fab-hI2S in the blood plasma supernatant was measured basically by the following method. After adding Superblock Blocking Buffer in PBS (Thermo Fisher Scientific) at 150 µL into each well of a Streptavidin Gold Plate (Meso Scale Diagnostics), it was shaken for 1 hour or longer for blocking of the plate. Next, a calibration curve reference sample containing Fab-hI2S of known concentration (Fab-hI2S calibration curve reference sample) and blood plasma were each added to a mixed solution of biotinylated anti-hI2S monoclonal antibody and sulfated anti-hI2S monoclonal antibody, and the mixture was shaken for 1 hour or longer to prepare an antibody reaction solution. The anti-hI2S monoclonal antibody was obtained by culturing an anti-hI2S-producing hybridoma created by a common method using splenocytes taken from mice immunized with human iduronate-2-sulfatase having the amino acid sequence listed as SEQ ID NO: 14. One of the obtained monoclonal antibodies was modified using Biotin Labeling Kit-NH2 (Dojindo Laboratories) according to the manufacturer's protocol, to obtain biotinylated anti-hI2S monoclonal antibody. The other monoclonal antibody was modified using MSD GOLD SULFO-TAG NHS-Ester (Meso Scale Diagnostics) according to the manufacturer's protocol, to obtain sulfated anti-hI2S monoclonal antibody. The blocking solution was removed from each well and rinsed with 0.05% Tween 20-containing PBS (PBST, Sigma Aldrich), after which 25 µL of antibody reaction solution was added to each well and the plate was shaken for 1 hour or longer. After then removing the antibody reaction solution and washing with PBST, 150 µL of 4 X Read Buffer (Meso Scale Diagnostics) diluted to 1/2 with water for injection (Otsuka Pharmaceutical Factory, Inc.) was added and a Sector^{™} Imager S600 (Meso Scale Diagnostics) was used to measure the luminescence from each well. A calibration curve was generated from the measured values for the Fab-hI2S calibration curve reference sample, and the measured values for each sample were fitted to the curve to calculate the amount of Fab-hI2S per 1 mL of blood plasma (the Fab-hI2S concentration in the blood plasma). Incidentally, the Fab-hI2S in the calibration curve reference sample was obtained by expressing the fusion protein (Fab-hI2S) comprising the conjugate having human I2S bonded to the C-terminus of the Fab region of the anti-hTfR antibody heavy chain having the amino acid sequence set forth as SEQ ID NO: 42, and the anti-hTfR antibody light chain having the amino acid sequence set forth as SEQ ID NO: 15, by a common method in CHO cells.

### [Example 16] Measurement of I2S specific activity in brain tissue and spinal cord

The I2S specific activity was measured to evaluate the Fab-hI2S in brain tissue and spinal cord, based on I2S enzyme activity. The I2S specific activity was measured basically by the following method. Water for injection (Otsuka Pharmaceutical Factory, Inc.) was added to the brain and spinal cord harvested in Example 13, and after homogenization, the obtained homogenate was centrifuged and the supernatant was collected. The protein concentration of the homogenate supernatant was quantified using a BCA Protein Assay Kit (Thermo Fisher Scientific), according to the attached instructions.

Using 4-methylumbelliferone (4-MU, Sigma Aldrich, 400 to 3.13 nmol/mL) as the standard and the homogenate supernatant as the measuring sample, 50 µL each was added to each well of a FluoroNunc Plate F96 (Nunc). Next, 4-methylumbelliferyl sulfate (4-MUS, Sigma Aldrich) diluted to 0.6 mg/mL with 5 mM acetate buffer (pH 5.0) containing 0.05% BSA (Sigma Aldrich)/0.1% TritonX-100 (FujiFilm-Wako), was added as substrate at 100 µL to each well. After shaking the plate with a plate mixer, it was allowed to stand at 37°C for 4 hours for enzyme reaction. A 150 µL portion of 0.05 M glycine/NaOH buffer (pH 10.6) was added to each well to suspend the reaction, and a SpectraMax Gemini (Molecular Devices, LLC) was used for measurement at an excitation wavelength of 365 nm and a detection wavelength of 460 nm. Defining 1 unit of enzyme activity as 4-MU nmol/4 hours at 37°C, this was divided by the protein weight (mg) to calculate the specific activity. That is, the specific activity can be considered to be the enzyme activity (nmol/4 hr/mg protein) per unit weight of protein. The results of I2S specific activity measurement are shown in Table 2.

### [Table 2]

**(Table 2) Measurement results for I2S specific activity in brain tissue and spinal cord**

| Administered group | | Brain tissue (nmol/4 hr/mg protein) | | Spinal cord (nmol/4 hr/mg protein) | |
|---|---|---|---|---|---|
| | | Mean | S.D. | Mean | S.D. |
| Normal control group | | 355 | 11.0 | 227 | 21.0 |
| Disease control group | | 0.000 | 0.000 | 0.000 | 0.000 |
| Fab-hI2S-rAAV administered group | 1.0 x 10¹³ vg/kg | 28.8 | 26.4 | 44.6 | 38.2 |
| | 1.0 x 10¹² vg/kg | 1.91 | 1.70 | 4.12 | 3.60 |
| | 1.0 x 10¹¹ vg/kg | 0.366 | 0.630 | 0.671 | 0.610 |
| | 1.0 x 10¹⁰ vg/kg | 1.05 | 1.08 | 1.88 | 0.98 |

In the disease control group, no I2S enzyme activity was detected in the central nervous system tissue, but in the Fab-hI2S-rAAV administered group, I2S enzyme activity was detected in the brain tissue and spinal cord at all doses, in essentially a dose-dependent manner. These results indicate that (i) administration of Fab-hI2S-rAAV results in expression of Fab-hI2S in host animals, (ii) the anti-hTfR antibody in Fab-hI2S crosses the BBB via hTfR to reach the central nervous system tissue, and (iii) hI2S in Fab-hI2S is present as functional enzyme in the central nervous system tissue.

### [Example 17] Quantitation of heparan sulfate and dermatan sulfate in tissues

Quantitation of heparan sulfate (HS) and dermatan sulfate (DS) in blood plasma, CSF and different tissues was carried out basically by the following method. Heparan sulfate and dermatan sulfate are both substrates of I2S. In patients with Hunter's syndrome where onset is due to abnormality in the gene encoding I2S, HS and DS abnormally accumulate in tissues. Therefore, quantitation of HS and DS in tissues allows evaluation of the drug effects of medicines administered to animal models.

Solutions (a) to (l) used for the test were prepared in the following manner.

### (a) MeCN/water:

After mixing 2 mL of water for injection (Otsuka Pharmaceutical Co., Ltd.) and 18 mL of acetonitrile (FujiFilm-Wako), the mixture was used as MeCN/water.

### (b) Deuterium-labeled solvent:

A deuterium-labeled solvent was prepared by dropping 240 µL of acetyl chloride (Sigma-Aldrich) into 1.5 mL of methanol-d₄ (Sigma-Aldrich) in an ice bath.

### (c) Mobile phase A:

A mixture of 475 mL of water for injection and 25 mL of 1 M aqueous ammonium formate (FujiFilm-Wako) was prepared as mobile phase A.

### (d) Mobile phase B:

Acetonitrile and mobile phase A were mixed at 93:7 (v/v) to prepare mobile phase B.

### (e) Heparan sulfate standard solution (HS standard solution):

Heparan Sulphate (Iduron) was dissolved in water for injection to prepare a 5.0 mg/mL solution. The solution was used as the HS standard solution.

### (F) heparan sulfate internal standard solution (HS internal standard solution):

A 40 µL portion of HS standard solution was measured out into a borosilicate screw-top test tube, and the solvent was distilled off under a nitrogen stream. After adding 400 µL of deuterium-labeled solvent to the dried product and stirring, reaction was carried out at 65°C for 75 minutes for deuterium methanolysis reaction (methanolysis). After the reaction, the solvent was distilled off under a nitrogen stream. A 500 µL portion of MeCN/water was added to the dried product, and ultrasonic treatment was carried out for 30 minutes. The solution was used as a heparan sulfate internal standard solution (HS internal standard solution).

### (g) Dermatan sulfate standard solution (DS standard solution):

Chondroitin Sulfate B sodium salt from porcine intestinal mucosa (Sigma-Aldrich) was dissolved in water for injection to prepare a 5.0 mg/mL solution. The solution was used as the DS standard solution.

### (h) Dermatan sulfate internal standard solution (DS internal standard solution):

A 40 µL portion of DS standard solution was measured out into a borosilicate screw-top test tube, and the solvent was distilled off under a nitrogen stream. After adding 400 µL of deuterium-labeled solvent to the dried product and stirring, reaction was carried out at 65°C for 75 minutes for deuterium methanolysis. After the reaction, the solvent was distilled off under a nitrogen stream. A 500 µL portion of MeCN/water was added to the dried product, and ultrasonic treatment was carried out for 30 minutes. The solution was used as a dermatan sulfate internal standard solution (DS internal standard solution).

### (i) Internal standard solution

After stirring a mixture of 1 µL of HS internal standard solution and 1 µL of DS internal standard solution with 1 mL of methanol, ultrasonic treatment was carried out for 30 minutes. The solution was used as the internal standard solution.

### (j) Calibration curve samples:

980 µL of water for injection was measured out, and HS standard solution and DS standard solution were each added thereto at 10 µL, to prepare a solution comprising 50 µg/mL each of dermatan sulfate and heparan sulfate. The solution was diluted with water for injection to prepare a solution comprising 5000 ng/mL each of dermatan sulfate and heparan sulfate. The solution was serially diluted with water for injection to prepare solutions comprising dermatan sulfate and heparan sulfate each at concentrations of 25, 50, 100, 250, 500, 1000, 2500 and 5000 ng/mL. A 20 µL portion of each solution was separated off into a separate borosilicate screw-top test tube. Each solution was used as a calibration curve sample.

### (k) Solution for tissue extraction

A 1 mL portion of polyoxyethylene(10) octylphenyl ether was added to physiological saline for a total of 500 mL. The solution was used as the solution for tissue extraction.

### (l) 10% Ammonium carbonate solution

A 5 g portion of ammonium carbonate was dissolved in 50 mL of water for injection. The solution was used as a 10% ammonium carbonate solution.

20 µL of blood plasma was measured out and dispensed into a borosilicate screw-top test tube. This was used as a blood sample solution.

20 µL of harvested CSF was measured out and dispensed into a borosilicate screw-top test tube. This was used as a CSF sample solution.

The freeze-dried tissue was crushed in the solution for tissue extraction, and the supernatant was centrifuged. 20 µL of the supernatant was measured out and dispensed into a borosilicate screw-top test tube. This was used as the tissue and organ sample solution.

The solvent of each prepared sample solution and calibration curve sample was distilled off under a nitrogen stream. After adding 20 µL of 2,2-dimethoxypropane (Tokyo Kasei Kogyo Co., Ltd.) and 200 µL of methanol hydrochloride with a hydrochloric acid concentration of 3 mol/L (Sigma-Aldrich) to the dried product, the mixture was stirred and used for methanolysis with a reaction temperature of 70°C and a reaction time of 90 minutes. The reaction mixture was then cooled on ice to suspend the reaction, and 200 µL of a 10% ammonium carbonate solution and 50 µL of the internal standard solution were added. The solvent was distilled off under a nitrogen stream, and then 250 µL of water for injection was added to dissolve the dried product. This was loaded into a solid phase cartridge (OASIS HLB (1 cc, 30 mg, Waters Co.)) that had been pre-conditioned by flowing through methanol and water for injection, and after washing with water for injection, 500 µL of methanol was added and the mixture was eluted by centrifugal separation. The solvent was distilled off under a nitrogen stream, and then 2 mL of water for injection and 18 mL of acetonitrile were added and the mixture was redissolved by ultrasonic treatment. The resultant was then centrifuged and the supernatant was filled into an LC vial.

LC/MS/MS analysis was carried out using a combination of hydrophilic interaction ultra high performance liquid chromatography and tandem quadrupole mass spectrometry. The mass spectrometer (MS/MS apparatus) used was a QTRAP5500 (AB Sciex), in which was set a Nexera X2 (Shimadzu Corp.) as the HPLC apparatus. The LC column used was an Acquity UPLC^{™} BEH Amide, 1.7 µm (2.1 × 150 mm, Waters Co.). Mobile phase A and mobile phase B were used as the mobile phases. The column temperature was set to 60°C.

After equilibration of the column with mobile phase B, 5 µL of sample was injected and chromatography was carried out under the mobile phase gradient conditions shown in Table 3. The mobile phase flow rate was 0.4 mL/min.

### [Table 3]

**(Table 3) Liquid chromatography conditions**

| Time elapsed after sample injection (min) | Mobile phase A (% (v/v)) | Mobile phase B (% (v/v)) |
|---|---|---|
| 0 | 0 | 100 |
| 5.00 | 0 | 100 |
| 7.00 | 20 | 80 |
| 12.5 | 20 | 80 |
| 13.0 | 70 | 30 |
| 15.0 | 70 | 30 |
| 15.5 | 0 | 100 |
| 20.0 | 0 | 100 |

The ion source parameter for MS/MS was set as shown in Table 4, according to the instruction manual for the QTRAP5500 (AB Sciex).

### [Table 4]

**(Table 4) Parameters set for MS/MS apparatus ion source**

| Ion Source | ESI (Turbo Spray) |
|---|---|
| Polarity | Positive |
| Scan type | MRM |
| IonSpray voltage | 5500 V |
| Heater gas temperature | 425°C |
| Curtain gas (CUR) | 30.00 |
| Collision gas (CAD) | 8.00 |
| Ion Source Gas 1 (GS1) | 70.00 |
| Ion Source Gas 2 (GS2) | 70.00 |
| Entrance Potential | 10.00 |
| Duration | 15.00 min |

Table 5 shows the MS internal parameters.

### [Table 5]

**(Table 5) MS internal parameters**

| | Precursor ion (m/z) Q1 | Production (m/z) Q3 | Time (msec) | DP (volts) | Collision energy (volts) | CXP (volts) |
|---|---|---|---|---|---|---|
| HS-derived | 384.100 | 162.100 | 100.00 | 45.00 | 21.00 | 15.00 |
| HS internal standard substance | 390.200 | 162.100 | 100.00 | 45.00 | 21.00 | 15.00 |
| DS-derived | 426.100 | 236.100 | 100.00 | 45.00 | 12.00 | 17.00 |
| DS internal standard substance | 432.200 | 239.200 | 100.00 | 45.00 | 12.00 | 17.00 |

LC/MS/MS analysis for the calibration curve samples was carried out, and the peak areas on a chromatogram chart were determined for product ions derived from dermatan sulfate and heparan sulfate in each calibration curve sample (DS detected peak area and HS detected peak area). The areas of the detected peaks corresponding to product ions derived from the DS internal standard solution and HS internal standard solution (DS-IS detected peak area and HS-IS detected peak area) were also calculated.

The area of the detected peak derived from dermatan sulfate with respect to the area of the detected peak derived from the DS internal standard solution (DS detected peak area/DS-IS detected peak area) for each calibration curve sample was plotted on the ordinate, the dermatan sulfate concentration for each calibration curve sample was plotted on the abscissa, and a regression equation was obtained using quadratic discriminant analysis.

The area of the detected peak derived from heparan sulfate with respect to the area of the detected peak derived from the HS internal standard solution (HS detected peak area/HS-IS detected peak area) for each calibration curve sample was plotted on the ordinate, the heparan sulfate concentration for each calibration curve sample was plotted on the abscissa, and a regression equation was obtained using quadratic discriminant analysis.

LC/MS/MS analysis was conducted for the blood sample solution, the CSF sample solution and each tissue sample solution, and the dermatan sulfate and heparan sulfate in the sample solution was quantified by fitting to the regression equation.

The Reduction ratio (%), representing the reduction of HS and DS in each tissue, was used as the indicator of drug effect. The Reduction ratio (%) was defined as: {([KO] - [WT]) - ([Test article] - [WT])} × 100 /([KO] - [WT]). [WT] represents the mean HS or DS concentration for the normal control group, [KO] represents the mean HS or DS concentration for the disease control group, and [Test article] represents the mean HS or DS concentration for each test substance administered group.

The measurement results for HS concentration (µg/mL) and Reduction ratio (%) in the CSF, brain and spinal cord are shown in Tables 6 to 8. In the Fab-hI2S-rAAV administered group, the HS concentrations in the CSF, brain and spinal cord all had Reduction ratios (%) that were generally lower in a dose-dependent manner compared to the disease control group. This result indicates that with intravenous injection of Fab-hI2S-rAAV to mice, Fab-hI2S expressed in the body crossed the BBB and reached central nervous system tissue, exhibiting the enzyme activity of hI2S in the central nervous system tissue and decomposing HS that had accumulated in the central nervous system.

### [Table 6]

**(Table 6) Measurement results for HS concentration in CSF**

| Administered group | | Mean (µg/mL) | S.D. | Reduction ratio (%) |
|---|---|---|---|---|
| Normal control group | | 0.262 | 0.039 | - |
| Disease control group | | 8.02 | 1.52 | - |
| Fab-hI2S-rAAV administered group (vg/kg) | 1.0 x 10¹³ | 1.33 | 1.10 | 86.2 |
| | 1.0 x 10¹² | 4.46 | 1.34 | 45.9 |
| | 1.0 x 10¹¹ | 7.86 | 0.54 | 2.10 |
| | 1.0 x 10¹⁰ | 6.71 | 0.37 | 16.9 |

### [Table 7]

**(Table 7 ) Measurement results for HS concentration in brain tissue**

| Administered group | | Mean (µg/mL) | S.D. | Reduction ratio (%) |
|---|---|---|---|---|
| Normal control group | | 0.0649 | 0.0104 | - |
| Disease control group | | 5.85 | 0.10 | - |
| Fab-hI2S-rAAV administered group (vg/kg) | 1.0 x 10¹³ | 1.20 | 0.92 | 80.3 |
| | 1.0 x 10¹² | 3.88 | 1.22 | 34.1 |
| | 1.0 x 10¹¹ | 5.23 | 0.14 | 10.7 |
| | 1.0 x 10¹⁰ | 5.90 | 0.39 | -0.900 |

### [Table 8]

**(Table 8 ) Measurement results for HS concentration in spinal cord**

| Administered group | | Mean (µg/mL) | S.D. | Reduction ratio (%) |
|---|---|---|---|---|
| Normal control group | | 0.0210 | 0.0054 | - |
| Disease control group | | 2.99 | 0.35 | - |
| Fab-hI2S-rAAV administered group (vg/kg) | 1.0 x 10¹³ | 0.787 | 0.397 | 74.2 |
| | 1.0 x 10¹² | 2.19 | 0.71 | 26.9 |
| | 1.0 x 10¹¹ | 2.63 | 0.35 | 12.1 |
| | 1.0 x 10¹⁰ | 2.71 | 0.18 | 9.3 |

Tables 9 to 13 show the HS concentrations (µg/mL) and DS concentrations (µg/mL), and their Reduction ratios (%), in the blood plasma, liver, spleen, heart and kidneys. In the Fab-hI2S-rAAV administered group, the HS concentrations and DS concentrations in the organs had Reduction ratios (%) that were generally lower in a dose-dependent manner compared to the disease control group. In the liver, administration of Fab-hI2S-rAAV in a dose exceeding 1.0 × 10¹² vg/kg resulted in a lower Reduction ratio for the HS concentration and DS concentration, but dose-dependent lowering was only up to a dose of 1.0 × 10¹² vg/kg. This result indicates that with intravenous injection of Fab-hI2S-rAAV to mice, Fab-hI2S expressed in the body exhibited enzyme activity as hI2S activity not only in the central nervous system but also in different tissues, decomposing HS and DS that had accumulated in those organs.

### [Table 9]

**(Table 9) Measurement results for HS and DS concentrations in plasma**

| Administered group | | HS (µg/mL) | | | DS (µg/mL) | | |
|---|---|---|---|---|---|---|---|
| | | Mean | S.D. | Reduction ratio (%) | Mean | S.D. | Reduction ratio (%) |
| Normal control group | | 0.0741 | 0.0138 | - | 0.212 | 0.043 | - |
| Disease control group | | 22.6 | 5.8 | - | 14.0 | 4.4 | - |
| Fab-hI2S-rAAV administered group (vg/kg) | 1.0 x 10¹³ | 6.99 | 11.61 | 69.3 | 3.67 | 5.74 | 74.9 |
| | 1.0 x 10¹² | 0.744 | 0.664 | 97.0 | 0.561 | 0.290 | 97.5 |
| | 1.0 x 10¹¹ | 6.94 | 4.83 | 69.5 | 6.30 | 4.92 | 55.8 |
| | 1.0 x 10¹⁰ | 21.5 | 8.5 | 4.90 | 14.2 | 4.7 | -1.40 |

### [Table 10]

**(Table 10) Measurement results for HS and DS concentrations in liver**

| Administered group | | HS (µg/mL) | | | DS (µg/mL) | | |
|---|---|---|---|---|---|---|---|
| | | Mean | S.D. | Reduction ratio (%) | Mean | S.D. | Reduction ratio (%) |
| Normal control group | | 0.0862 | 0.0219 | - | 0.0840 | 0.0174 | - |
| Disease control group | | 30.3 | 9.4 | - | 3.16 | 0.72 | - |
| Fab-hI2S-rAAV administered group (vg/kg) | 1.0 x 10¹³ | 2.47 | 0.43 | 92.1 | 0.888 | 0.085 | 73.9 |
| | 1.0 x 10¹² | 2.11 | 0.75 | 93.3 | 0.780 | 0.202 | 77.4 |
| | 1.0 x 10¹¹ | 7.30 | 5.10 | 76.2 | 1.61 | 0.99 | 50.5 |
| | 1.0 x 10¹⁰ | 24.9 | 11.5 | 18.0 | 2.12 | 0.24 | 33.7 |

### [Table 11]

**(Table 11) Measurement results for HS and DS concentrations in spleen**

| Administered group | | HS (µg/mL) | | | DS (µg/mL) | | |
|---|---|---|---|---|---|---|---|
| | | Mean | S.D. | Reduction ratio (%) | Mean | S.D. | Reduction ratio (%) |
| Normal control group | | 0.112 | 0.019 | - | 0.0915 | 0.0144 | - |
| Disease control group | | 13.2 | 0.5 | - | 4.89 | 0.49 | - |
| Fab-hI2S-rAAV administered group (vg/kg) | 1.0 x 10¹³ | 0.283 | 0.072 | 98.7 | 0.814 | 0.133 | 94.7 |
| | 1.0 x 10¹² | 0.419 | 0.147 | 97.7 | 0.924 | 0.267 | 93.8 |
| | 1.0 x 10¹¹ | 3.56 | 1.78 | 73.8 | 2.45 | 1.02 | 82.2 |
| | 1.0 x 10¹⁰ | 11.0 | 3.0 | 17.4 | 4.93 | 1.16 | 63.3 |

### [Table 12]

**(Table 12) Measurement results for HS and DS concentrations in heart**

| Administered group | | HS (µg/mL) | | | DS (µg/mL) | | |
|---|---|---|---|---|---|---|---|
| | | Mean | S.D. | Reduction ratio (%) | Mean | S.D. | Reduction ratio (%) |
| Normal control group | | 0.0644 | 0.0061 | - | 0.0918 | 0.0348 | - |
| Disease control group | | 9.72 | 1.05 | - | 5.13 | 0.93 | - |
| Fab-hI2S-rAAV administered group (vg/kg) | 1.0 x 10¹³ | 0.603 | 0.256 | 94.4 | 0.662 | 0.234 | 88.7 |
| | 1.0 x 10¹² | 0.701 | 0.297 | 93.4 | 0.801 | 0.388 | 85.9 |
| | 1.0 x 10¹¹ | 5.57 | 2.17 | 43.0 | 3.59 | 1.53 | 30.6 |
| | 1.0 x 10¹⁰ | 8.24 | 0.32 | 15.3 | 4.25 | 0.41 | 17.5 |

### [Table 13]

**(Table 13) Measurement results for HS and DS concentrations in kidneys**

| Administered group | | HS (µg/mL) | | | DS (µg/mL) | | |
|---|---|---|---|---|---|---|---|
| | | Mean | S.D. | Reduction ratio (%) | Mean | S.D. | Reduction ratio (%) |
| Normal control group | | 0.201 | 0.015 | - | 0.0569 | 0.0042 | - |
| Disease control group | | 36.1 | 1.7 | - | 5.75 | 0.77 | - |
| Fab-hI2S-rAAV administered group (vg/kg) | 1.0 x 10¹³ | 3.06 | 2.82 | 92.0 | 0.536 | 0.103 | 91.6 |
| | 1.0 x 10¹² | 9.83 | 4.69 | 73.2 | 1.10 | 0.46 | 81.7 |
| | 1.0 x 10¹¹ | 33.0 | 8.3 | 8.80 | 5.21 | 1.61 | 9.50 |
| | 1.0 x 10¹⁰ | 36.7 | 3.4 | -1.70 | 5.05 | 0.71 | 12.3 |

The results shown above indicate that intravenous injection of Fab-hI2S-rAAV can cause Fab-hI2S to be expressed over long periods in the body, and that the expressed Fab-hI2S exhibits hI2S activity in the central nervous system and other tissues of pathological model mice, thus allowing decomposition of accumulated HS and DS, so that Fab-hI2S-rAAV is effective as a therapeutic agent for Hunter's syndrome, and particularly as a therapeutic agent for central nerve disorder associated with Hunter's syndrome.

It is considered that the same effect will be obtained even with rAAV virions incorporating lysosomal enzymes other than hI2S. Thus, rAAV virions incorporating other lysosomal enzymes are also thought to be useful as therapeutic agents for diseases caused by genetic abnormalities of other lysosomal enzymes. Moreover, rAAV virions incorporating proteins of interest such as antibody drugs and humoral factors including cytokines, instead of hI2S, are also expected to exhibit the functions of those proteins of interest in the central nervous system, and such rAAV virions are thus likewise expected to be useful as therapeutic agents for diseases associated with central nervous system disorders.

### [Example 18] Construction of pAAV-mMAP-Fab-hGLB1 vector

A DNA fragment was synthesized containing the nucleotide sequence set forth as SEQ ID NO: 47, which includes the ClaI site, mouse α-fetoprotein enhancer/mouse albumin promoter, chicken β actin/MVM chimeric intron, a gene encoding a conjugate of hGLB 1 bonded to the C-terminal end of the Fab region of the anti-hTfR antibody heavy chain, a nucleotide sequence encoding 2A peptide derived from porcine teschovirus, a gene encoding the anti-hTfR antibody light chain, the poly-A sequence of bovine growth hormone, and the BglII site, in order from the 5'-end. The DNA fragment was digested with ClaI and BglII. A pAAV-CMV vector (Takara Bio, Inc.) was digested with ClaI and BglII and the aforementioned restriction enzyme-treated DNA fragment was inserted into it. The obtained plasmid was designated as pAAV-mMAP-Fab-hGLB1 vector. The pAAV-mMAP-Fab-hGLB1 vector has a structure wherein the site indicated as reference numeral 4 of the pAAV-mMAP-Fab-hI2S vector shown in Fig. 1 is replaced by a gene encoding a conjugate with hGLB1 bonded to the C-terminal end of the Fab region of the anti-hTfR antibody heavy chain, and the site indicated as reference numeral 5 is replaced by a nucleotide sequence encoding 2A peptide derived from porcine teschovirus. The pAAV-mMAP-Fab-hGLB1 vector has a structure including a first AAV-ITR functional equivalent (SEQ ID NO: 6), mouse α-fetoprotein enhancer/mouse albumin promoter (SEQ ID NO: 8), chicken β actin/MVM chimeric intron (SEQ ID NO: 9), a gene encoding a conjugate with hGLB 1 bonded to the C-terminal end of the Fab region of the anti-hTfR antibody heavy chain (SEQ ID NO: 48), a nucleotide sequence encoding 2A peptide derived from porcine teschovirus (SEQ ID NO: 49), a gene encoding the light chain of anti-hTfR antibody (SEQ ID NO: 10), the poly-A signal of bovine growth hormone (SEQ ID NO: 17) and a second AAV-ITR functional equivalent (SEQ ID NO: 7), in order from the upstream end. The pAAV-mMAP-Fab-hGLB1 vector also includes an ampicillin resistance gene and a replication origin (ColE1 ori). Incidentally, hGLB1 is a lysosomal enzyme, and genetic abnormalities in hGLB1 cause GM1-gangliosidosis.

### [Example 19] Preparation of rAAV solution containing rAAV encoding Fab-hGLB 1 gene

A transfection DNA solution containing pHelper vector (Takara Bio, Inc.), pR2(mod)C8 vector and pAAV-mMAP-Fab-hGLB1 vector was used to produce a rAAV virion encoding the Fab-hGLB 1 gene by the method described in Example 4. The obtained rAAV virion was designated as Fab-hGLB1-rAAV. When used to infect cells, Fab-hGLB1-rAAV is expected to express a fusion protein that includes a conjugate with human β-galactosidase bonded to the C-terminal end of the heavy chain of the anti-human transferrin receptor antibody Fab region, and the anti-human hTfR antibody light chain. An rAAV solution containing rAAV encoding the Fab-hGLB1 gene (Fab-hGLB 1-rAAV solution) was obtained by the method described in Examples 5 to 10. The viral genome quantity in the Fab-hGLB1-rAAV solution was measured by the method described in Example 11.

### [Example 20] Evaluation of drug effect of Fab-hGLB 1-rAAV using GLB1-KO/hTfR-KI mice

The drug effect when administering Fab-hGLB 1-rAAV to the body was evaluated using GLB1-KO/hTfR-KI mice as GM1-gangliosidosis model mice, created by the similar method described in Example 12. GLB1-KO/hTfR-KI mice are mice that are homodeficient for the β-galactosidase (GLB1) gene and heterozygous for the chimeric TfR gene. The GLB1-KO/hTfR-KI mice used were mice 10 to 12 weeks of age (at the start of administration). The Fab-hGLB1-rAAV solution prepared in Example 19 was administered to GLB1-KO/hTfR-KI mice once through the caudal vein at dosages of 1.0 × 10¹³ vg/kg and 1.0 × 10¹⁴ vg/kg (N=3 for each group). A normal control group composed of male wild type mice and a disease control group composed of male GLB1-KO/hTfR-KI mice were also prepared (N=3 for each group). The normal control group and the disease control group were both administered physiological saline (Otsuka Pharmaceutical Factory, Inc.).

At 8, 15 and 22 days after administration, a small portion of blood was harvested from the caudal vein into an EDTA-2K-coated blood sampling tube (Capiject, Terumo Corp.), and after allowing it to cool on ice, it was centrifuged (2000 × g, 20 minutes, 4°C) and the blood plasma was collected. At 29 days after administration, cerebrospinal fluid (CSF) was harvested from the cisterna magna under anesthesia with a 3-drug mixture of Betorfal (Meiji Seika Pharma), Midazolam (Sandoz Ltd.) and Dormital (Zenoaq). The mice were then opened by thoracotomy and heart blood was sampled. The blood was harvested into an EDTA-2K-coated blood sampling tube (Capiject, Terumo Corp.), and after allowing it to cool on ice, it was centrifuged (2000 × g, 20 minutes) and the blood plasma was collected. After subsequent general perfusion with physiological saline (Otsuka Pharmaceutical Factory, Inc.), the brain, spinal cord, liver, heart, kidneys and spleen were each extracted. After measuring the wet weight of each tissue, it was immediately immersed in liquid nitrogen for rapid freezing.

### [Example 21] Quantitation of Fab-hGLB1 in blood plasma

Measurement of the Fab-hGLB 1 concentration in the blood plasma supernatant was carried out by the method described in Example 22. The results are shown in Table 14. The Fab-hGLB1 concentration was calculated as the amount of Fab-hGLB 1 per 1 mL of blood plasma (µg/mL blood plasma).

In the Fab-hGLB1-rAAV administered group, the Fab-hGLB1 concentration in the blood plasma increased from day 8 to day 29 after administration, at all administered doses. The Fab-hGLB1 concentration in the blood plasma increased in a dose-dependent manner. These data indicate that the Fab-hGLB1-rAAV intravenously injected into the mice had been incorporated into the cells, that hGLB1-encoding gene had been transcribed in the cells, and that translated and expressed hGLB1 had been released into the blood.

### [Table 14]

**(Table 14) Time-related change in Fab-hGLB 1 concentration in plasma (µg/mL plasma)**

| Administered group | | Day 8 after administration | | Day 15 after administration | | Day 22 after administration | | Day 29 after administration | |
|---|---|---|---|---|---|---|---|---|---|
| | | Mean | S.D. | Mean | S.D. | Mean | S.D. | Mean | S.D. |
| Fab-hGLB 1-rAAV (vg/kg) | 1.0 x 10¹³ | 0.239 | 0.073 | 0.381 | 0.165 | 0.576 | 0.248 | 0.663 | 0.384 |
| | 1.0 x 10¹⁴ | 0.228 | 0.062 | 1.34 | 0.32 | 1.01 | 0.38 | 2.63 | 0.10 |

### [Example 22] Measurement of Fab-hGLB1 concentration in blood plasma

The Fab-hGLB1 concentration in the blood plasma was measured basically by the following method. After adding Superblock Blocking Buffer in PBS (Thermo Fisher Scientific) at 150 µL into each well of a Streptavidin Gold Plate (Meso Scale Diagnostics), it was shaken for 1 hour or longer for blocking of the plate. Next, a calibration curve reference sample containing Fab-hGLB1 of known concentration (Fab-hGLB1 calibration curve reference sample) and blood plasma were each added to a mixed solution of biotinylated anti-hGLB 1 monoclonal antibody and sulfated anti-hGLB 1 monoclonal antibody, and the mixture was shaken for 1 hour or longer to prepare an antibody reaction solution. The anti-hGLB 1 monoclonal antibody was obtained by culturing an anti-hGLB1-producing hybridoma created by a common method using splenocytes taken from mice immunized with human β-galactosidase having the amino acid sequence set forth as SEQ ID NO: 50. One of the obtained monoclonal antibodies was modified using Biotin Labeling Kit-NH2 (Dojindo Laboratories) according to the manufacturer's protocol, to obtain biotinylated anti-hGLB 1 monoclonal antibody. The other monoclonal antibody was modified using MSD GOLD SULFO-TAG NHS-Ester (Meso Scale Diagnostics) according to the manufacturer's protocol, to obtain sulfated anti-hGLB1 monoclonal antibody. The blocking solution was removed from each well and rinsed with 0.05% Tween 20-containing PBS (PBST, Sigma Aldrich), after which 25 µL of antibody reaction solution was added to each well and the mixture plate was shaken for 1 hour or longer. After then removing the antibody reaction solution and washing with PBST, 150 µL of 4 X Read Buffer (Meso Scale Diagnostics) diluted to 1/2 with water for injection (Otsuka Pharmaceutical Factory, Inc.) was added and a Sector^{™} Imager S600 (Meso Scale Diagnostics) was used to measure the luminescence from each well. A calibration curve was generated from the measured values for the Fab-hGLB 1 calibration curve reference sample, and the measured values for each sample were fitted to the curve to calculate the amount of Fab-hGLB 1 per 1 mL of blood plasma (the Fab-hGLB 1 concentration in the blood plasma). Incidentally, the Fab-hGLB 1 in the calibration curve reference sample was obtained by expressing the fusion protein (Fab-hGLB 1) comprising the conjugate having human GLB 1 bonded to the C-terminus of the Fab region of the anti-hTfR antibody heavy chain including the amino acid sequence set forth as SEQ ID NO: 48, and the anti-hTfR antibody light chain including the amino acid sequence set forth as SEQ ID NO: 15, by a common method in CHO cells.

### [Example 23] Quantitation of Fab-hGLB1 in brain and spinal cord tissue

Measurement of the Fab-hGLB1 concentration in brain and spinal cord tissue was carried out by the method described in Example 22, homogenizing the brain and spinal cord obtained in Example 20 with RIPA Buffer (Wako Pure Chemical Industries, Ltd.) containing 0.025% Protease Inhibitor Cocktail (Sigma-Aldrich), centrifuging the obtained homogenate and using the collected supernatant. The Fab-hGLB1 concentration was calculated as the amount of Fab-hGLB 1 per 1 g of tissue (µg/mg tissue). Tables 15 and 16 show the results of measuring the Fab-hGLB1 concentrations in the brain and spinal cord tissue. In the Fab-hGLB1-rAAV administered group, Fab-hGLB1 was detected in the brain and spinal cord tissue at all dosages. In the Fab-hGLB1-rAAV non-administered group, however, Fab-hGLB1 was not detected in brain and spinal cord tissue of the GLB1 KO mice. The Fab-hGLB1 concentration also increased in a dose-dependent manner. These data indicate that intravenous injection of Fab-hGLB1-rAAV to mice allows Fab-GLB1 to be distributed throughout the central nerve tissue.

### [Table 15]

**(Table 15) Measurement results for Fab-hGLB 1 in brain tissue (µg/mg tissue**

| Administered group | | Mean | S.D. |
|---|---|---|---|
| Fab-hGLB1-rAAV (vg/kg) | 1.0 x 10¹³ | 0.312 | 0.186 |
| | 1.0 x 10¹⁴ | 0.401 | 0.034 |

### [Table 16]

**(Table 16) Measurement results for Fab-hGLB 1 in spinal cord tissue (µg/mg tissue)**

| Administered group | | Mean | S.D. |
|---|---|---|---|
| Fab-hGLB1-rAAV (vg/kg) | 1.0 x 10¹³ | 0.247 | 0.143 |
| | 1.0 x 10¹⁴ | 0.364 | 0.057 |

### [Example 24] Quantitation of lyso-GM1 in CSF, brain and spinal cord tissue

Quantitation of *lyso-GM1* in CSF, blood plasma and different tissues was carried out by the method described in WO2021/039644. Incidentally, *lyso*-GM1 is a substrate degraded by GLB1, and it accumulates in the tissue of GM1-gangliosidosis patients. Therefore, quantitation of *lyso-GM1* in tissue allows evaluation of the drug effect of a medicine administered to a GM1-gangliosidosis animal model. The Reduction ratio (%), representing the reduction of *lyso-GM1* in each tissue, was used as the indicator of drug effect. The Reduction ratio (%) was defined as: {([KO] - [WT]) - ([Test article] - [WT])} × 100 /([KO] - [WT]). [WT] represents the mean lyso-GM1 concentration for the normal control group, [KO] represents the mean lyso-GM1 concentration for the disease control group, and [Test article] represents the mean lyso-GM1 concentration for each test substance administered group.

The measurement results for lyso-GM1 concentration and Reduction ratio (%) in the CSF, brain and spinal cord are shown in Tables 17, 18 and 19. The lyso-GM1 concentration in the CSF was calculated as the lyso-GM1 concentration per 1 mL of CSF (µg/mL CSF). The lyso-GM1 concentrations in the brain and spinal cord were calculated as the lyso-GM1 concentrations per 1 mg of each tissue (µg/mg tissue). With the Fab-hGLB1-rAAV administered groups, the lyso-GM1 concentrations in the CSF, brain and spinal cord were lower in every administered group compared to the disease control group. This result indicates that with intravenous injection of Fab-hGLB1-rAAV to mice, Fab-hGLB1 expressed in the body crossed the BBB and reached central nervous system tissue, exhibiting the activity of hGLB 1 in the central nervous system tissue and decomposing lyso-GM1 that had accumulated in the central nervous system.

### [Table 17]

**(Table 17) Measurement results for lyso-GM1 in CSF (µg/mL CSF)**

| Administered group | | Mean (µg/mL) | S.D. | Reduction ratio (%) |
|---|---|---|---|---|
| Normal control group | | 0.00 | 0.00 | - |
| Disease control group | | 1.36 | 0.13 | - |
| Fab-hGLB1-rAAV administered group (vg/kg) | 1.0 x 10¹³ | 0.587 | 0.461 | 56.7 |
| | 1.0 x 10¹⁴ | 0.643 | 0.178 | 52.7 |

### [Table 18]

**(Table 18) Measurement results for lyso-GM1 in brain tissue (µg/mg tissue)**

| Administered group | | Mean (µg/mL) | S.D. | Reduction ratio (%) |
|---|---|---|---|---|
| Normal control group | | 0.142 | 0.027 | - |
| Disease control group | | 67.8 | 9.7 | - |
| Fab-hGLB1-rAAV administered group (vg/kg) | 1.0 x 10¹³ | 25.0 | 19.3 | 63.2 |
| | 1.0 x 10¹⁴ | 16.3 | 3.5 | 76.1 |

### [Table 19]

**(Table 19) Measurement results for lyso-GM1 in spinal cord tissue (µg/mg tissue**

| Administered group | | Mean (µg/mL) | S.D. | Reduction ratio (%) |
|---|---|---|---|---|
| Normal control group | | 0.0 | 0.00 | - |
| Disease control group | | 5.16 | 0.65 | - |
| Fab-hGLB1-rAAV administered group (vg/kg) | 1.0 x 10¹³ | 3.29 | 2.17 | 36.3 |
| | 1.0 x 10¹⁴ | 2.43 | 0.37 | 58.5 |

While not shown in the data, the lyso-GM1 concentrations in the blood plasma and in the liver, heart, kidneys and spleen were also similarly lowered in all of the administered groups compared to the normal control group.

The results shown above indicate that intravenous injection of Fab-hGLB 1-rAAV can cause Fab-hGLB1 to be expressed over long periods in the body, and that the expressed Fab-hGLB1 exhibits hGLB1 activity in the central nervous system and other tissues of pathological model mice, allowing decomposition of abnormally accumulated hGLB1 substrate, so that Fab-hGLB 1-rAAV is effective as a therapeutic agent for GM1-gangliosidosis, and particularly as a therapeutic agent for central nerve disorder associated with GM1-gangliosidosis.

### Industrial Applicability

According to the invention it is possible to provide a medicine which is a nucleic acid molecule in a form of a plasmid, in a form encapsulated in a recombinant virus virion, or in a form encapsulated in liposomes or lipid nanoparticles, which is capable of expressing a fusion protein of a ligand and a protein having physiological activity in cells, tissue or the body.

### Reference Signs List

1 First AAV-ITR functional equivalent
2 Mouse α-fetoprotein enhancer/mouse albumin promoter
3 Chicken β actin/MVM chimeric intron
4 Gene encoding conjugate having hI2S bonded to C-terminal end of Fab region of anti-hTfR antibody heavy chain
5 Internal ribosome entry site from 5' untranslated region of murine encephalomyocarditis virus
6 Gene encoding anti-hTfR antibody light chain
7 Bovine growth hormone poly-A signal
8 Second AAV-ITR functional equivalent
9 Ampicillin resistance gene
10 Replication origin (ColE1 ori)
11 p5 promoter
12 AAV2 Rep Region
13 AAV8 Cap region
14 p5 promoter functioning as enhancer

### [Sequence Listing Free Text]

SEQ ID NO: 1: Example 1 of linker amino acid sequence
SEQ ID NO: 2: Example 2 of linker amino acid sequence
SEQ ID NO: 3: Example 3 of linker amino acid sequence
SEQ ID NO: 4: Nucleotide sequence of first inverted terminal repeat (first ITR) in AAV serotype 2, wild type
SEQ ID NO: 5: Nucleotide sequence of first inverted terminal repeat (second ITR) in AAV serotype 2, wild type
SEQ ID NO: 6: Preferred example of nucleotide sequence of first inverted terminal repeat (first ITR), synthetic sequence
SEQ ID NO: 7: Preferred example of nucleotide sequence of second inverted terminal repeat (second ITR), synthetic sequence
SEQ ID NO: 8: Nucleotide sequence of mouse α-fetoprotein enhancer/mouse albumin promoter, synthetic sequence
SEQ ID NO: 9: Nucleotide sequence of chicken β actin/MVM chimeric intron, synthetic sequence
SEQ ID NO: 10: Example of nucleotide sequence of internal ribosome entry site from 5' untranslated region of murine encephalomyocarditis virus
SEQ ID NO: 11: Amino acid sequence of human transferrin receptor
SEQ ID NO: 12: Example of amino acid sequence of heavy chain Fab region of anti-hTfR antibody
SEQ ID NO: 13: Example 4 of linker amino acid sequence
SEQ ID NO: 14: Amino acid sequence of human I2S
SEQ ID NO: 15: Example of amino acid sequence of anti-hTfR antibody light chain
SEQ ID NO: 16: Example of amino acid sequence of anti-hTfR antibody heavy chain
SEQ ID NO: 17: Nucleotide sequence of bovine growth hormone poly-A signal
SEQ ID NO: 18: Nucleotide sequence of DNA fragment synthesized for creation of pAAV-mMAP-Fab-hI2S vector, synthetic sequence
SEQ ID NO: 19: Nucleotide sequence of DNA fragment synthesized for creation of pR2(mod)C6 vector, synthetic sequence
SEQ ID NO: 20: Nucleotide sequence of DNA fragment synthesized for creation of pR2(mod)C8 vector, synthetic sequence
SEQ ID NO: 21: Nucleotide sequence of AAV2 Rep region including p5 promoter, synthetic sequence
SEQ ID NO: 22: Nucleotide sequence of AAV8 Cap region
SEQ ID NO: 23: Nucleotide sequence of p5 promoter
SEQ ID NO: 24: Nucleotide sequence of AAV2 Rep region, synthetic sequence
SEQ ID NO: 25: Nucleotide sequence of primer SI-1, synthetic sequence
SEQ ID NO: 26: Nucleotide sequence of primer SI-2, synthetic sequence
SEQ ID NO: 27: Nucleotide sequence of probe SI-1, synthetic sequence
SEQ ID NO: 28: Nucleotide sequence of primer SI-3, synthetic sequence
SEQ ID NO: 29: Nucleotide sequence of primer SI-4, synthetic sequence
SEQ ID NO: 30: Nucleotide sequence of probe SI-2, synthetic sequence
SEQ ID NO: 31: Example of amino acid sequence 1 of anti-hTfR antibody light chain CDR1
SEQ ID NO: 32: Example of amino acid sequence 2 of anti-hTfR antibody light chain CDR1
SEQ ID NO: 33: Example of amino acid sequence 1 of anti-hTfR antibody light chain CDR2
SEQ ID NO: 34: Example of amino acid sequence 2 of anti-hTfR antibody light chain CDR2
SEQ ID NO: 35: Example of amino acid sequence 1 of anti-hTfR antibody light chain CDR3
SEQ ID NO: 36: Example of amino acid sequence 1 of anti-hTfR antibody heavy chain CDR1
SEQ ID NO: 37: Example of amino acid sequence 2 of anti-hTfR antibody heavy chain CDR1
SEQ ID NO: 38: Example of amino acid sequence 1 of anti-hTfR antibody heavy chain CDR2
SEQ ID NO: 39: Example of amino acid sequence 2 of anti-hTfR antibody heavy chain CDR2
SEQ ID NO: 40: Example of amino acid sequence 1 of anti-hTfR antibody heavy chain CDR3
SEQ ID NO: 41: Example of amino acid sequence 2 of anti-hTfR antibody heavy chain CDR3
SEQ ID NO: 42: Amino acid sequence of conjugate of anti-hTfR antibody heavy chain Fab region and human I2S
SEQ ID NO: 43: Preferred example of nucleotide sequence of first long terminal repeat (first LTR), synthetic sequence
SEQ ID NO: 44: Preferred example of nucleotide sequence of second long terminal repeat (second LTR), synthetic sequence
SEQ ID NO: 45: Nucleotide sequence of leader of Sendai virus genome
SEQ ID NO: 46: Nucleotide sequence of trailer of Sendai virus genome
SEQ ID NO: 47: Nucleotide sequence of DNA fragment synthesized for creation of pAAV-mMAP-Fab-hGLB1 vector, synthetic sequence
SEQ ID NO: 48: Example of amino acid sequence for conjugate of anti-hTfR antibody heavy chain Fab region and human β-galactosidase
SEQ ID NO: 49: Nucleotide sequence encoding porcine teschovirus 2A peptide
SEQ ID NO: 50: Amino acid sequence of human β-galactosidase [Sequence Listing]

## Claims

1. A nucleic acid molecule comprising any one of the following nucleotide sequences (1) to (6):
(1) a nucleotide sequence comprising a first inverted terminal repeat (ITR) or a functional equivalent thereof, a nucleotide sequence encoding a fusion protein of a ligand and a protein having physiological activity, and a nucleotide sequence comprising a second inverted terminal repeat (ITR) or a functional equivalent thereof, in this order toward the downstream end;
(2) a nucleotide sequence comprising a first inverted terminal repeat (ITR) or a functional equivalent thereof, a nucleotide sequence comprising a regulatory element of gene expression, a nucleotide sequence encoding a fusion protein of a ligand and a protein having physiological activity, and a nucleotide sequence comprising a second inverted terminal repeat (ITR) or a functional equivalent thereof, in this order toward the downstream end;
(3) a nucleotide sequence comprising a first long terminal repeat (LTR) or a functional equivalent thereof, a nucleotide sequence encoding a fusion protein of a ligand and a protein having physiological activity, and a nucleotide sequence comprising a second long terminal repeat (LTR) or a functional equivalent thereof, in this order toward the downstream end;
(4) a nucleotide sequence comprising a first long terminal repeat (LTR) or a functional equivalent thereof, a nucleotide sequence comprising a regulatory element of gene expression, a nucleotide sequence encoding a fusion protein of a ligand and a protein having physiological activity, and a nucleotide sequence comprising a second long terminal repeat (LTR) or a functional equivalent thereof, in this order toward the downstream end;
(5) a nucleotide sequence comprising a leader or a functional equivalent thereof, a nucleotide sequence encoding a fusion protein of a ligand and a protein having physiological activity, and a nucleotide sequence comprising a trailer or a functional equivalent thereof, in this order toward the downstream end; or
(6) a nucleotide sequence comprising a leader or a functional equivalent thereof, a nucleotide sequence comprising a regulatory element of gene expression, a nucleotide sequence encoding a fusion protein of a ligand and a protein having physiological activity, and a nucleotide sequence comprising a trailer or a functional equivalent thereof, in this order toward the downstream end.

2. The nucleic acid molecule according to claim 1, wherein the ligand is an antibody.

3. The nucleic acid molecule according to claim 2, selected from the following (1) to (4);
(1) the nucleotide sequence encoding the fusion protein of the antibody and the protein having physiological activity comprises a nucleotide sequence encoding a conjugate in which the protein having physiological activity is bonded to the C-terminus of the heavy chain of the antibody, and a nucleotide sequence encoding the light chain of the antibody;
(2) the nucleotide sequence encoding the fusion protein of the antibody and the protein having physiological activity comprises a nucleotide sequence encoding a conjugate in which the protein having physiological activity is bonded to the N-terminus of the heavy chain of the antibody, and a nucleotide sequence encoding the light chain of the antibody;
(3) the nucleotide sequence encoding the fusion protein of the antibody and the protein having physiological activity comprises a nucleotide sequence encoding a conjugate in which the protein having physiological activity is bonded to the C-terminus of the light chain of the antibody, and a nucleotide sequence encoding the heavy chain of the antibody; or
(4) the nucleotide sequence encoding the fusion protein of the antibody and the protein having physiological activity comprises a nucleotide sequence encoding a conjugate in which the protein having physiological activity is bonded to the N-terminus of the light chain of the antibody, and a nucleotide sequence encoding the heavy chain of the antibody.

4. The nucleic acid molecule according to claim 2, selected from the following (1) to (4);
(1) the nucleotide sequence encoding the fusion protein of the antibody and the protein having physiological activity comprises a nucleotide sequence encoding a conjugate in which the protein having physiological activity is bonded to the C-terminus of the heavy chain of the antibody via a linker, and a nucleotide sequence encoding the light chain of the antibody;
(2) the nucleotide sequence encoding the fusion protein of the antibody and the protein having physiological activity comprises a nucleotide sequence encoding a conjugate in which the protein having physiological activity is bonded to the N-terminus of the heavy chain of the antibody via a linker, and a nucleotide sequence encoding the light chain of the antibody;
(3) the nucleotide sequence encoding the fusion protein of the antibody and the protein having physiological activity comprises a nucleotide sequence encoding a conjugate in which the protein having physiological activity is bonded to the C-terminus of the light chain of the antibody via a linker, and a nucleotide sequence encoding the heavy chain of the antibody; or
(4) the nucleotide sequence encoding the fusion protein of the antibody and the protein having physiological activity comprises a nucleotide sequence encoding a conjugate in which the protein having physiological activity is bonded to the N-terminus of the light chain of the antibody via a linker, and a nucleotide sequence encoding the heavy chain of the antibody.

5. The nucleic acid molecule according to claim 4, wherein the linker is a peptide consisting of 1 to 50 amino acid residues.

6. The nucleic acid molecule according to claim 5, wherein the linker is a peptide comprising an amino acid sequence selected from the group consisting of a single glycine, a single serine, the amino acid sequence Gly-Ser, the amino acid sequence Gly-Gly-Ser, the amino acid sequence set forth as SEQ ID NO: 1, the amino acid sequence set forth as SEQ ID NO: 2, the amino acid sequence set forth as SEQ ID NO: 3, and an amino acid sequence consisting of 2 to 10 of any of aforementioned amino acid sequences that are consecutively linked.

7. The nucleic acid molecule according to claim 2, selected from the following (1) to (4);
(1) the nucleotide sequence encoding the fusion protein of the antibody and the protein having physiological activity comprises a nucleotide sequence encoding a conjugate in which the heavy chain of the antibody is bonded to the C-terminus of the light chain of the antibody via a second linker and the protein having physiological activity is bonded to the C-terminus of the heavy chain of the antibody directly or via a linker;
(2) the nucleotide sequence encoding the fusion protein of the antibody and the protein having physiological activity comprises a nucleotide sequence encoding a conjugate in which the light chain of the antibody is bonded to the C-terminus of the heavy chain of the antibody via a second linker and the protein having physiological activity is bonded to the C-terminus of the light chain of the antibody directly or via a linker;
(3) the nucleotide sequence encoding the fusion protein of the antibody and the protein having physiological activity comprises a nucleotide sequence encoding a conjugate in which the heavy chain of the antibody is bonded to the C-terminus of the protein having physiological activity directly or via a linker and the light chain of the antibody is bonded to the C-terminus of the heavy chain of the antibody via a second linker; or
(4) the nucleotide sequence encoding the fusion protein of the antibody and the protein having physiological activity comprises a nucleotide sequence encoding a conjugate in which the light chain of the antibody is bonded to the C-terminus of the protein having physiological activity directly or via a linker and the heavy chain of the antibody is bonded to the C-terminus of the light chain of the antibody via a second linker.

8. The nucleic acid molecule according to claim 7, wherein the linker is a peptide consisting of 1 to 50 amino acid residues.

9. The nucleic acid molecule according to claim 8, wherein the linker is a peptide comprising an amino acid sequence selected from the group consisting of a single glycine, a single serine, the amino acid sequence Gly-Ser, the amino acid sequence Gly-Gly-Ser, the amino acid sequence set forth as SEQ ID NO: 1, the amino acid sequence set forth as SEQ ID NO: 2, the amino acid sequence set forth as SEQ ID NO: 3, and an amino acid sequence consisting of 2 to 10 of any of aforementioned amino acid sequences that are consecutively linked.

10. The nucleic acid molecule according to claim 9, wherein the second linker consists of 8 to 50 amino acid residues.

11. The nucleic acid molecule according to claim 8, wherein the second linker is selected from the group consisting of the amino acid sequence Gly-Ser, the amino acid sequence Gly-Gly-Ser, the amino acid sequence Gly-Gly-Gly, the amino acid sequence set forth as SEQ ID NO: 1, the amino acid sequence set forth as SEQ ID NO: 2, the amino acid sequence set forth as SEQ ID NO: 3, an amino acid sequence consisting of three consecutively linked sequences that are each the sequence set forth as SEQ ID NO: 1, and an amino acid sequence consisting of 2 to 10 of any of aforementioned amino acid sequences that are consecutively linked.

12. The nucleic acid molecule according to claim 3, selected from the following (1) to (4);
(1) the nucleotide sequence encoding the fusion protein of the antibody and the protein having physiological activity comprises a nucleotide sequence encoding a conjugate in which the protein having physiological activity is bonded to the C-terminus of the heavy chain of the antibody, a nucleotide sequence encoding an internal ribosome entry site, and a nucleotide sequence encoding the light chain of the antibody, in this order toward the downstream end;
(2) the nucleotide sequence encoding the fusion protein of the antibody and the protein having physiological activity comprises a nucleotide sequence encoding a conjugate in which the protein having physiological activity is bonded to the N-terminus of the heavy chain of the antibody, a nucleotide sequence encoding an internal ribosome entry site, and a nucleotide sequence encoding the light chain of the antibody, in this order toward the downstream end;
(3) the nucleotide sequence encoding the fusion protein of the antibody and the protein having physiological activity comprises a nucleotide sequence encoding a conjugate in which the protein having physiological activity is bonded to the C-terminus of the light chain of the antibody, a nucleotide sequence encoding an internal ribosome entry site, and a nucleotide sequence encoding the heavy chain of the antibody, in this order toward the downstream end; or
(4) the nucleotide sequence encoding the fusion protein of the antibody and the protein having physiological activity comprises a nucleotide sequence encoding a conjugate in which the protein having physiological activity is bonded to the N-terminus of the light chain of the antibody, a nucleotide sequence encoding an internal ribosome entry site, and a nucleotide sequence encoding the heavy chain of the antibody, in this order toward the downstream end.

13. The nucleic acid molecule according to any one of claims 4 to 6, selected from the following (1) to (4);
(1) the nucleotide sequence encoding the fusion protein of the antibody and the protein having physiological activity comprises a nucleotide sequence encoding a conjugate in which the protein having physiological activity is bonded to the C-terminus of the heavy chain of the antibody via a linker, a nucleotide sequence encoding an internal ribosome entry site, and a nucleotide sequence encoding the light chain of the antibody, in this order toward the downstream end;
(2) the nucleotide sequence encoding the fusion protein of the antibody and the protein having physiological activity comprises a nucleotide sequence encoding a conjugate in which the protein having physiological activity is bonded to the N-terminus of the heavy chain of the antibody via a linker, a nucleotide sequence encoding an internal ribosome entry site, and a nucleotide sequence encoding the light chain of the antibody, in this order toward the downstream end;
(3) the nucleotide sequence encoding the fusion protein of the antibody and the protein having physiological activity comprises a nucleotide sequence encoding a conjugate in which the protein having physiological activity is bonded to the C-terminus of the light chain of the antibody via a linker, a nucleotide sequence encoding an internal ribosome entry site, and a nucleotide sequence encoding the heavy chain of the antibody, in this order toward the downstream end; or
(4) the nucleotide sequence encoding the fusion protein of the antibody and the protein having physiological activity comprises a nucleotide sequence encoding a conjugate in which the protein having physiological activity is bonded to the N-terminus of the light chain of the antibody via a linker, a nucleotide sequence encoding an internal ribosome entry site, and a nucleotide sequence encoding the heavy chain of the antibody, in this order toward the downstream end.

14. The nucleic acid molecule according to claim 12 or 13, wherein the internal ribosome entry site is derived from the 5' untranslated region of a virus or gene selected from the group consisting of a virus belonging to the family Picornaviridae, foot-and-mouth disease virus, hepatitis A virus, hepatitis C virus, coronavirus, bovine enterovirus, Cyler's murine encephalomyelitis virus, Coxsackievirus group B, the human immunoglobulin heavy chain binding protein gene, the Drosophila Antennapedia gene and the Drosophila Ultrabithorax gene.

15. The nucleic acid molecule according to claim 12 or 13, wherein the internal ribosome entry site is derived from the 5' untranslated region of a virus belonging to the family Picornaviridae.

16. The nucleic acid molecule according to any one of claims 2 to 14, wherein the antibody is an antigen-binding fragment.

17. The nucleic acid molecule according to any one of claims 2 to 14, wherein the antibody is Fab.

18. The nucleic acid molecule according to claim 2, wherein the antibody is a single-domain antibody.

19. The nucleic acid molecule according to any one of claims 2 to 18, wherein the antibody has specific affinity for a protein present on the surfaces of vascular endothelial cells.

20. The nucleic acid molecule according to claim 19, wherein the vascular endothelial cells are cerebrovascular endothelial cells.

21. The nucleic acid molecule according to claim 19 or 20, wherein the protein present on the surfaces of the vascular endothelial cells is selected from the group consisting of transferrin receptor, insulin receptor, leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor.

22. The nucleic acid molecule according to any one of claims 1 to 21, wherein the regulatory element of gene expression is selected from the group consisting of cytomegalovirus-derived promoter, SV40 early promoter, human elongation factor-1α (EF-1α) promoter, human ubiquitin C promoter, retroviral Rous sarcoma virus-LTR promoter, dihydrofolate reductase promoter, β-actin promoter, phosphoglycerate kinase (PGK) promoter, mouse albumin promoter, human albumin promoter, human α-1 antitrypsin promoter and mouse α-fetoprotein enhancer/mouse albumin promoter.

23. The nucleic acid molecule according to claim 1, wherein the ligand has specific affinity for a protein present on the surfaces of vascular endothelial cells.

24. The nucleic acid molecule according to claim 23, wherein the vascular endothelial cells are cerebrovascular endothelial cells.

25. The nucleic acid molecule according to claim 23 or 24, wherein the protein present on the surfaces of the vascular endothelial cells is selected from the group consisting of transferrin receptor, insulin receptor, leptin receptor, insulin-like growth factor I receptor, insulin-like growth factor II receptor, lipoprotein receptor, glucose transporter 1, organic anion transporter, monocarboxylic acid transporter, low density lipoprotein receptor-related protein 1, low density lipoprotein receptor-related protein 8, and membrane-bound precursor of heparin-binding epidermal growth factor-like growth factor.

26. The nucleic acid molecule according to claim 23 or 24, wherein the ligand is selected from the group consisting of transferrin, insulin, leptin, insulin-like growth factor I, insulin-like growth factor II, lipoprotein, and low density lipoprotein.

27. The nucleic acid molecule according to any one of claims 1 to 26, wherein the protein having physiological activity is selected from the group consisting of growth hormone, a lysosomal enzyme, somatomedin, insulin, glucagon, a cytokine, a lymphokine, a blood coagulation factor, an antibody, afusion protein of an antibody and other protein, granulocyte-macrophage colony-stimulating factor (GM-CSF), granulocyte-colony stimulating factor (G-CSF), macrophage colony-stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, nerve growth factor (NGF), ciliary neurotrophic factor (CNTF), glial cell-line neurotrophic factor (GDNF), neurotrophin 3, neurotrophin 4/5, neurotrophin 6, neuregulin 1, activin, basic fibroblast growth factor (bFGF), fibroblast growth factor 2 (FGF2), epidermal growth factor (EGF), vascular endothelial growth factor (VEGF), interferon-α, interferon-β, interferon-γ, interleukin-6, PD-1, PD-1 ligand, tumor necrosis factor-α receptor (TNF-α receptor), an enzyme having beta amyloid degrading activity, etanercept, pegvisomant, metreleptin, abatacept, asfotase, GLP-1 receptor agonist, and an antibody medicine.

28. The nucleic acid molecule according to any one of claims 1 to 26, wherein the protein having physiological activity is selected from the group consisting of α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, palmitoyl protein thioesterase-1, tripeptidyl peptidase-1, hyaluronidase-1, CLN1 and CLN2.

29. The nucleic acid molecule according to any one of claims 1 to 28, wherein the first inverted terminal repeat and the second inverted terminal repeat are those derived from an adeno-associated virus, or those derived from an adenovirus, or a variant thereof.

30. The nucleic acid molecule according to any one of claims 1 to 28, wherein the first inverted terminal repeat comprises the nucleotide sequence set forth as SEQ ID NO: 4, and the second inverted terminal repeat comprises the nucleotide sequence set forth as SEQ ID NO: 5.

31. The nucleic acid molecule according to any one of claims 1 to 28, wherein the first inverted terminal repeat is selected from the following (1) to (3) and the second inverted terminal repeat is selected from the following (4) to (6):
(1) a nucleotide sequence having identity not lower than 80% to the nucleotide sequence set forth as SEQ ID NO: 4,
(2) a nucleotide sequence having identity not lower than 90% to the nucleotide sequence set forth as SEQ ID NO: 4,
(3) the nucleotide sequence set forth as SEQ ID NO: 4, but modified by 1 to 20 nucleotide substitutions, deletions or additions, and
(4) a nucleotide sequence having identity not lower than 80% to the nucleotide sequence set forth as SEQ ID NO: 5,
(5) a nucleotide sequence having identity not lower than 90% to the nucleotide sequence set forth as SEQ ID NO: 5,
(6) the nucleotide sequence set forth as SEQ ID NO: 5, but modified by 1 to 20 nucleotide substitutions, deletions or additions.

32. The nucleic acid molecule according to any one of claims 1 to 28, wherein the functional equivalent of the first inverted terminal repeat comprises the nucleotide sequence set forth as SEQ ID NO: 6, and the functional equivalent of the second inverted terminal repeat comprises the nucleotide sequence set forth as SEQ ID NO: 7.

33. The nucleic acid molecule according to any one of claims 1 to 28, wherein the functional equivalent of the first inverted terminal repeat is selected from the following (1) to (3) and the functional equivalent of the second inverted terminal repeat is selected from the following (4) to (6):
(1) a nucleotide sequence having identity not lower than 80% to the nucleotide sequence set forth as SEQ ID NO: 6,
(2) a nucleotide sequence having identity not lower than 90% to the nucleotide sequence set forth as SEQ ID NO: 6,
(3) the nucleotide sequence set forth as SEQ ID NO: 6, but modified by 1 to 20 nucleotide substitutions, deletions or additions, and
(4) a nucleotide sequence having identity not lower than 80% to the nucleotide sequence set forth as SEQ ID NO: 7,
(5) a nucleotide sequence having identity not lower than 90% to the nucleotide sequence set forth as SEQ ID NO: 7,
(6) the nucleotide sequence set forth as SEQ ID NO: 7, but modified by 1 to 20 nucleotide substitutions, deletions or additions.

34. The nucleic acid molecule according to any one of claims 1 to 28, wherein the first long terminal repeat and the second long terminal repeat are those derived from a lentivirus or retrovirus, or a variant thereof.

35. The nucleic acid molecule according to any one of claims 1 to 28, wherein the first long terminal repeat comprises the nucleotide sequence set forth as SEQ ID NO: 43, and the second long terminal repeat comprises the nucleotide sequence set forth as SEQ ID NO: 44.

36. The nucleic acid molecule according to any one of claims 1 to 28, wherein the first long terminal repeat is selected from the following (1) to (3) and the second long terminal repeat is selected from the following (4) to (6):
(1) a nucleotide sequence having identity not lower than 80% to the nucleotide sequence set forth as SEQ ID NO: 43,
(2) a nucleotide sequence having identity not lower than 90% to the nucleotide sequence set forth as SEQ ID NO: 43,
(3) the nucleotide sequence set forth as SEQ ID NO: 43, but modified by 1 to 20 nucleotide substitutions, deletions or additions, and
(4) a nucleotide sequence having identity not lower than 80% to the nucleotide sequence set forth as SEQ ID NO: 44,
(5) a nucleotide sequence having identity not lower than 90% to the nucleotide sequence set forth as SEQ ID NO: 44,
(6) the nucleotide sequence set forth as SEQ ID NO: 44, but modified by 1 to 20 nucleotide substitutions, deletions or additions.

37. The nucleic acid molecule according to any one of claims 1 to 28, wherein the leader and the trailer are ethose derived from Sendai virus, or a variant thereof.

38. The nucleic acid molecule according to any one of claims 1 to 28, wherein the leader comprises the nucleotide sequence set forth as SEQ ID NO: 45, and the trailer comprises the nucleotide sequence set forth as SEQ ID NO: 46.

39. The nucleic acid molecule according to any one of claims 1 to 28, wherein the leader is selected from the following (1) to (3) and the trailer is selected from the following (4) to (6):
(1) a nucleotide sequence having identity not lower than 80% to the nucleotide sequence set forth as SEQ ID NO: 45,
(2) a nucleotide sequence having identity not lower than 90% to the nucleotide sequence set forth as SEQ ID NO: 45,
(3) the nucleotide sequence set forth as SEQ ID NO: 45, but modified by 1 to 20 nucleotide substitutions, deletions or additions, and
(4) a nucleotide sequence having identity not lower than 80% to the nucleotide sequence set forth as SEQ ID NO: 46,
(5) a nucleotide sequence having identity not lower than 90% to the nucleotide sequence set forth as SEQ ID NO: 47,
(6) the nucleotide sequence set forth as SEQ ID NO: 47, but modified by 1 to 20 nucleotide substitutions, deletions or additions.

40. A cell, tissue or animal, into which the nucleic acid molecule according to any one of claims 1 to 39 has been introduced.

41. A stem cell, into which the nucleic acid molecule according to any one of claims 1 to 39 has been introduced.

42. The stem cell according to claim 41, which is selected from the group consisting of mesenchymal stem cells, dental pulp stem cells, hematopoietic stem cells, embryonic stem cells, endothelial stem cells, mammary stem cells, intestinal stem cells, liver stem cells, pancreatic stem cells, neural stem cells and iPS cells.

43. A plasmid comprising the nucleic acid molecule according to any one of claims 1 to 39.

44. A virion comprising the nucleic acid molecule according to any one of claims 1 to 39.
